# EUROPEAN PATENT SPECIFICATION

(11) **EP 0 749 437 B1**
(45) Date of publication and mention of the grant of the patent: **05.12.2001**
(21) Application number: 94931274.8
(22) Date of filing: 13.09.1994
(51) Int. Cl.: C07J 41/00, C07J 51/00, C07J 43/00, A61K 31/56, C07J 9/00, C07J 17/00, C07J 31/00

(54) **PHARMACEUTICAL USES OF STEROID DERIVATIVES**
VERWENDUNG VON STEROIDDERIVATEN ALS ARZNEIMITTEL
UTILISATIONS PHARMACEUTIQUES DE DERIVES DE STEROIDES

(30) Priority: 10.03.1994 WO PCT/US94/02397
(43) Date of publication of application: 27.12.1996
(73) Proprietor: MAGAININ PHARMACEUTICALS INC., Plymouth Meeting PA 19462 (US)
(72) Inventor: FRYE, Leah, L., Ravena, NY 12143 (US); ZASLOFF, Michael, A., Merion Station, PA 19066 (US); KINNEY, William, A., Churchville, PA 18966 (US); MORIARTY, Robert, Oak Park, IL 60302 (US); COLLINS, Delwood, C., Lexington, KY 40536-0284 (US)
(74) Representative: Kolb, Helga, Dr. Dipl.-Chem.
(86) International application number: US9410265
(87) International publication number: WO9524415

(56) References cited:
- FR-A- 2 361 899
- US-A- 3 370 070
- US-A- 5 004 737
- US-A- 5 192 756
- STEROIDS, vol.58, no.8, August 1993, STONEHAM, USA pages 370 - 378 S. L. WEHRLI ET AL 'Structure of the novel steroidal antibiotic squalamine determined by two-dimensional NMR spectroscopy' cited in the application

## Description

Squalamine, 3β- (N- [3-aminopropyl] -1,4-butanediamine)-7α,24ζ-dihydroxy-5α-cholestane 24-sulfate, is an aminosterol that has been isolated from the dogfish shark, *Squalus acanthias.* See K. Moore, S. Wehrli, H. Roder, M. Rogers, J. Forrest, D. McCrimmon, and M. Zasloff, *Proc. Nat. Acad. Sci.* (USA), Vol. 90, February 1993, 1354-1358. See also U.S. Patent No. 5,192,756 to Zasloff et al.

This water-soluble steroid exhibits potent bactericidal activity against both gram-positive and gram-negative bacteria. In addition, squalamine is antifungal and exhibits lytic activity against protozoa. The molecule was initially recovered as a natural product through extraction of several tissues of the dogfish shark, including the stomach, liver, gallbladder and spleen. Its structure was determined by fast atom bombardment mass spectroscopy and NMR spectroscopy (S. Wehrli et al., *Steroids* 58, 1993, 370-378). The chemical structure of squalamine is that of a cationic steroid characterized by a condensation of an anionic bile salt intermediate with spermidine. Squalamine represented the first example of a steroid to which spermidine is covalently coupled and a new class of antibiotics (K. Moore, S. Wehrli, H. Roder, M. Rogers, J. Forrest, D. McCrimmon, and M. Zasloff, *Proc Nat. Acad. Sci.* (USA), Vol. 90, February 1993, 1354-1358).

### Summary of the Invention

The invention relates to the use of compositions containing squalamine or one of the compounds of the Formula I : 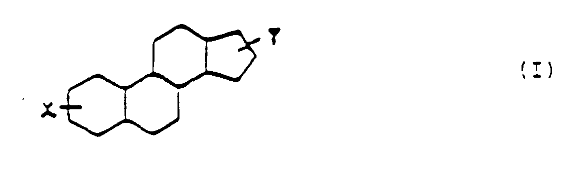 wherein X and Y are each independently selected from a cationic hydrophilic side chain and an anionic hydrophilic side chain; and the steroid ring nucleus is saturated, unsaturated or partially saturated, and is substituted with at least one group selected from -OH, -NH₂, -SH, -F, alkoxy and alkyl, for the production of a pharmaceutical for inhibiting angiogenesis, cytotoxic agents or fibroblast growth inhibiting agents.

The invention therefore relates to the therapeutical use of such compositions to treat cancer.

Additionally, the invention relates to the use of compositions containing squalamine or one of the compounds of the Formula I as Na⁺/K⁺-ATPase inhibitors. The invention therefore relates to the therapeutical use of such compositions as diuretics, antihypertensive agents, gastric-acid antisecretory agents, muscle relaxants and the like.

Preferred compounds are of the Formula II: 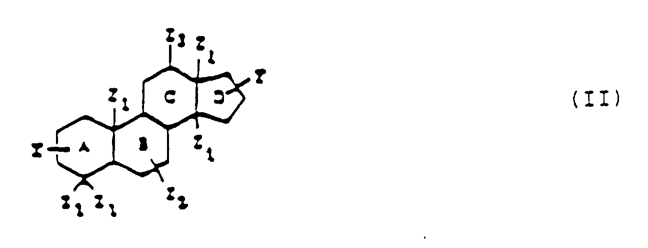 wherein each Z₁ is independently selected from H and C₁-C₄ alkyl; and Z₂ and Z₃ are each independently selected from H, OH, NH₂ and SH.

### Detailed Description of Preferred Embodiments

The present invention is directed to the use of steroid compounds of the Formula I: 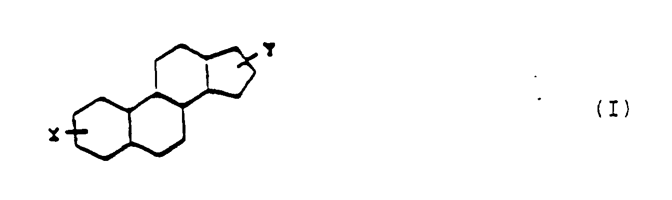 wherein X is a cationic hydrophilic side chain having at least two positively charged amino groups; Y is an anionic hydrophilic side chain; and the steroid ring nucleus 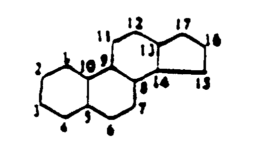 is saturated, partially saturated or unsaturated, and is substituted with at least one group selected from -OH, -NH₂, -SH, alkoxy and alkyl, preferably -OH for the preparation of medicaments.

Compounds of the Formula I preferably have a net positive charge. Preferably X is a cationic side chain and Y is an anionic side chain. On the steroid ring nucleus, X is preferably at position 3, 15, 16 or 17 and at a position different from that of Y, more preferably at position 3. Y is preferably at position 3, 15, 16 or 17, preferably at position 17. Each side chain, as an independent compound, is hydrophilic and preferably water-soluble.

The anionic side chain, Y, is a hydrocarbon chain substituted with a negatively charged group, preferably sulfate, sulfite, carboxylate or phosphate, more preferably sulfate. The hydrocarbon chain may be, for example, aliphatic, cycloaliphatic or aromatic, and may be saturated or unsaturated. The anionic side chain is hydrophilic and therefore the hydrocarbon generally has no more than twelve carbon atoms.

The anionic side chain may also be a hydrocarbon chain having at least one carboxyl group or carboxylate group, e.g., -R₁-COOM wherein R₁ is a C₁-C₁₂ hydrocarbon, such as a C₁-C₁₂ alkyl, and M is hydrogen or an appropriate ionizable cation, e.g., sodium or potassium. The hydrocarbon chain may further have a negatively charged group, such as sulfate, sulfite or phosphate.

The anionic side chain may also be a substituted amide wherein the substituent group of the amide is a hydrocarbon chain substituted with at least one negatively charged group such as phosphate, sulfate, sulfite, carboxylate, sulfonate or phosphonate, e.g.: 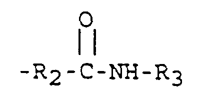 wherein R₂ is a C₁-C₁₂ hydrocarbon group and R₃ is a hydrocarbon group substituted with a sulfate, sulfite, phosphate, carboxylate, sulfonate or phosphonate group. R₂ and R₃ may be the same or different hydrocarbon groups. The hydrocarbon groups are preferably aliphatic and contain no more than about nineteen carbon atoms. Thus, for example, R₃ may be -(CH₂)₂-SO₃H.

The anionic side chain Y may also contain an oxygen, nitrogen or sulfur atom linking Y to the C-17 position of the steroid ring nucleus.

Particularly preferred anionic side chains include the following: 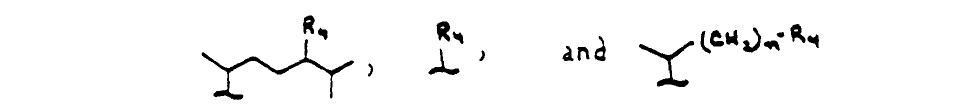 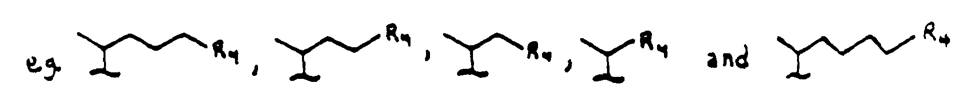 where R₄ is OSO₃⁻, OPO₃⁻, CO₂⁻ or OSO₂⁻, and n is an integer of from 1 to 18.

The cationic side chain, X, is a hydrophilic amine which has at least one amino group that is not directly linked to the steroid ring nucleus. The amine may be, for example, an aliphatic amine, cycloaliphatic amine, heterocyclic amine or aromatic amine, provided that there is at least one positively charged amine group in the side chain. The cationic side chain, which includes at least one positively charged amine group, may be linked to the steroid ring nucleus through one of the following groups: -NR₅-, where R₅ is a hydrocarbon, more particularly an alkyl, or hydrogen; -S-; or -O-.

Thus, the cationic side chain may be depicted by the formula R₆-Z, wherein Z is -NR₅ as defined above, -S or -O, and R₆ is an organic amine. The organic amine may be a hydrocarbon amine wherein the amine group is in the hydrocarbon chain (such as in a polyamine or heterocyclic amine) or where the amine group is a substituent group on the hydrocarbon side chain. Thus, for example, the cationic side chain may be an aliphatic polyamine containing two or more amine groups, such as spermidine or ethylene diamine; or a heterocyclic amine such as pyridine or pyrrolidine; or an organic amine, such as an alkylamine. The organic amine may also be a peptide or an amino acid containing at least one positively charged amino group. Thus, for example, the peptide may include arginine, lysine or histidine, or R₆ may be an amino acid such as arginine, lysine, etc. The organic amine may also be an amine substituted sugar such as glucosamine and galactosamine.

As hereinabove indicated, the cationic side chain is hydrophilic and therefore the number of carbon atoms in the hydrocarbon moieties is such as to retain hydrophilicity. In general, the hydrocarbon moieties contain no more than 12 carbon atoms.

Particularly preferred cationic side chains include: 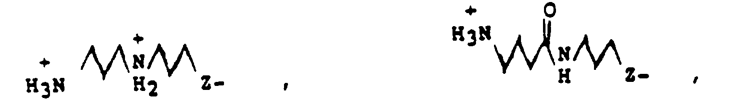 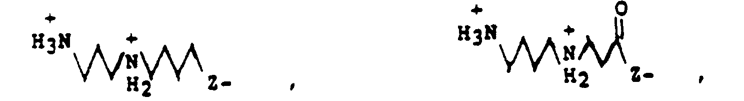 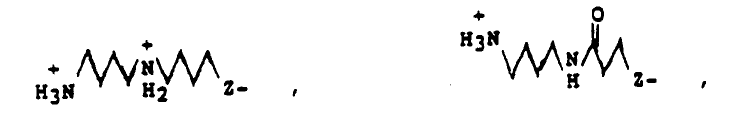 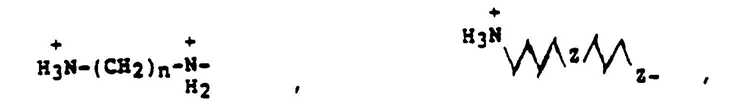 and 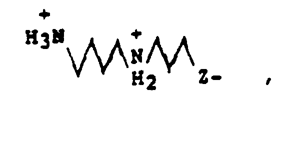 wherein Z is O, S or NR₅, where R₅ is hydrogen or alkyl; and n is an integer of from 2 to 12.

The steroid ring nucleus may be saturated, partially saturated or unsaturated, and is preferably saturated. The steroid ring also includes at least one hydrophilic substituent group at a position on the steroid ring nucleus different from both the X and Y side chains. Such hydrophilic substituent group is generally -OH, -NH₂, alkoxy or -SH, preferably -OH.

Preferred compounds are those of the Formula II: 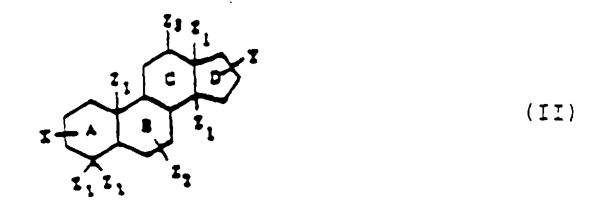 wherein each Z₁ is independently selected from H and C₁-C₄ alkyl; and Z₂ and Z₃ are each independently selected from H, OH, NH₂ and SH. Preferably, the Z₂ and Z₃ hydrophilic substituent groups are located at position 6, 7, 11 and/or 12 of the steroid ring nucleus.

The alkyl group of Z₁ is preferably substituted at position 10 and/or 13, more preferably at both positions, of the steroid ring nucleus. When the steroid ring nucleus includes an alkyl substituent group(s), it is preferably located in a plane opposite to the plane of the hydrophilic substituent group(s). Thus, if the hydrophilic group(s) are alpha group(s), the alkyl group(s) are beta group(s) and vice-versa.

Preferred compounds are of the following formulae, wherein X, Y, Z₁, Z₂ and Z₃ are as defined above:

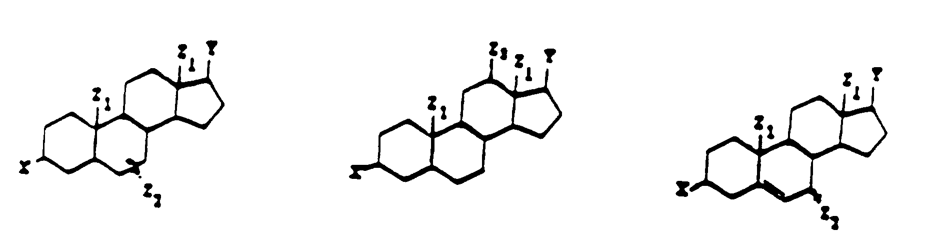

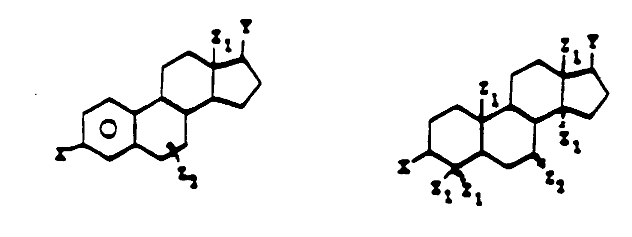

Where reference is made herein to "hydrocarbon" or "alkyl" groups, it will be understood that these groups may be branched or straight.chain.

The compounds for use in the invention include stereoisomers of compounds of the general formulae and prodrugs thereof, as well as pharmaceutically acceptable salts of such compounds. The compounds of the invention include those of the above general Formulae I or II and their salts, as optically pure stereoisomers or as a mixture of stereoisomers.

Preferred compounds of the invention include the following: 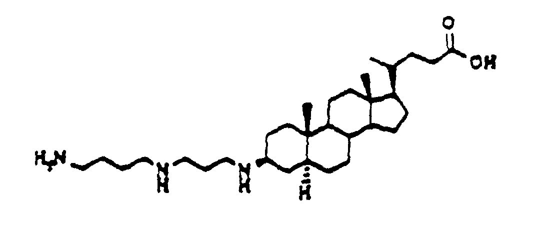 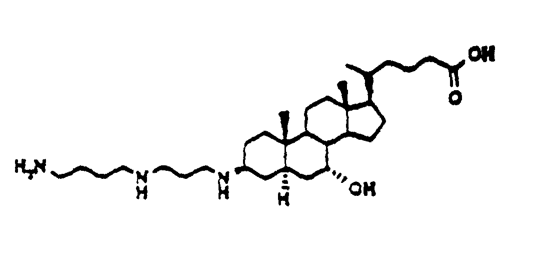 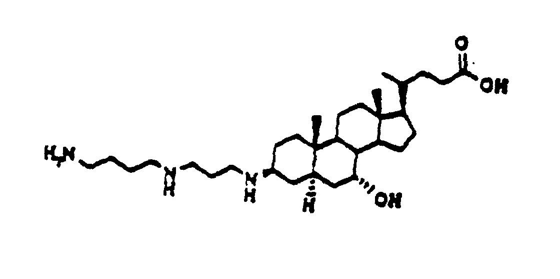 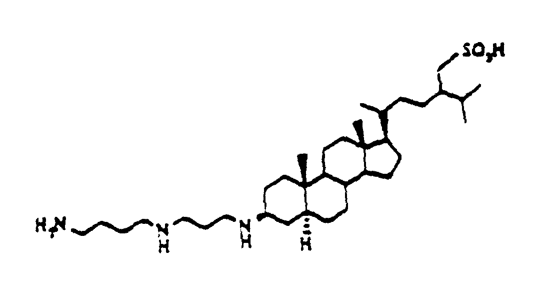 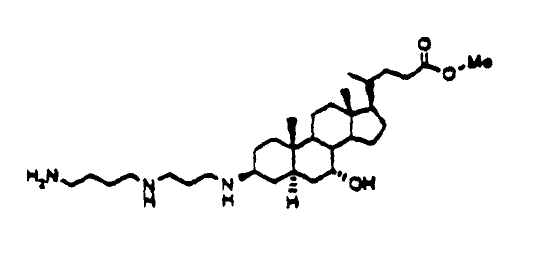 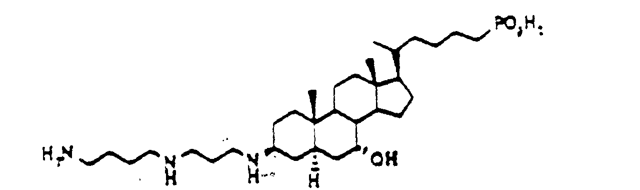 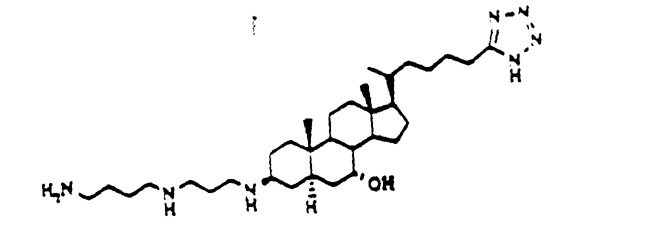 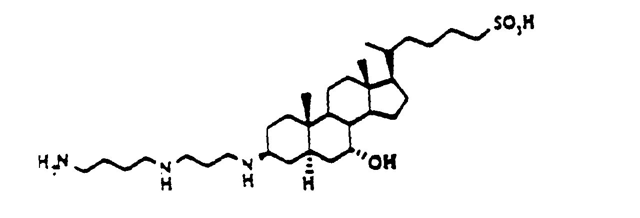 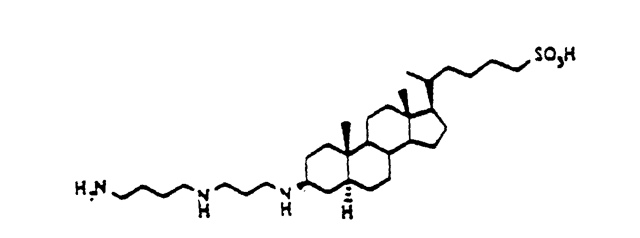 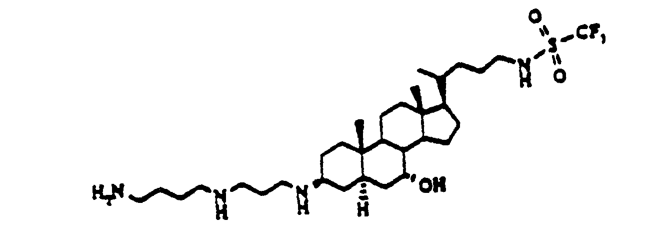 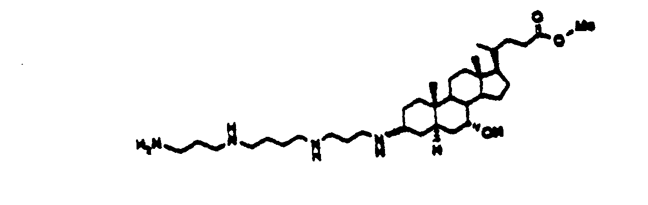 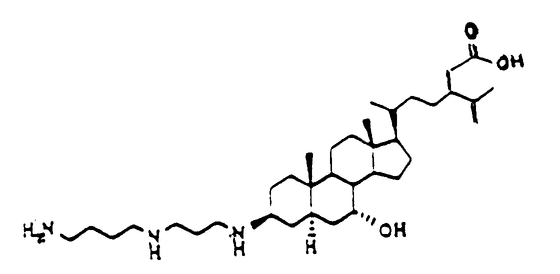 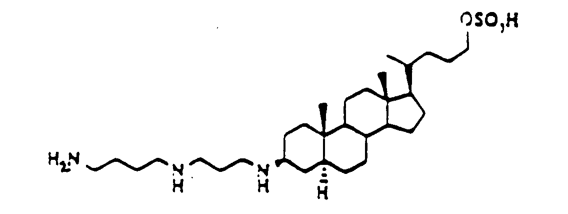 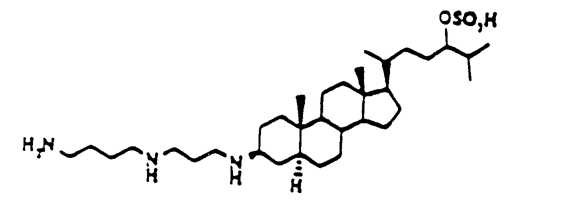 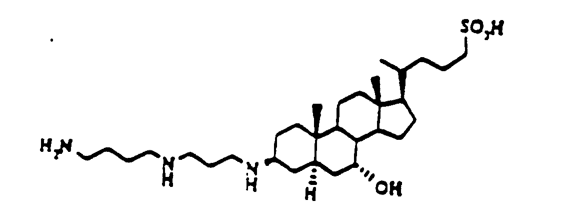 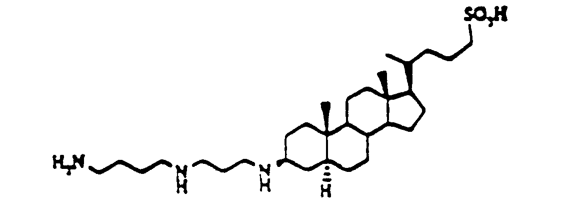 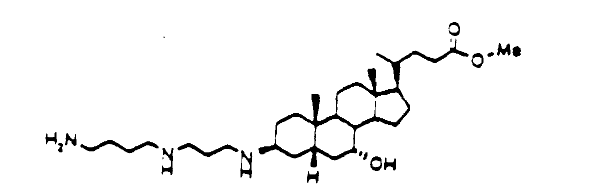 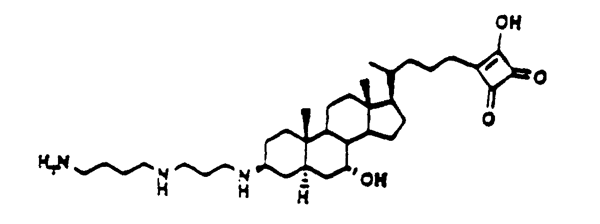 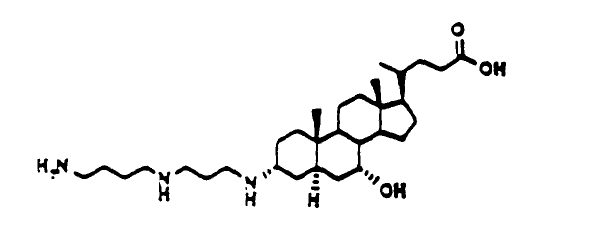 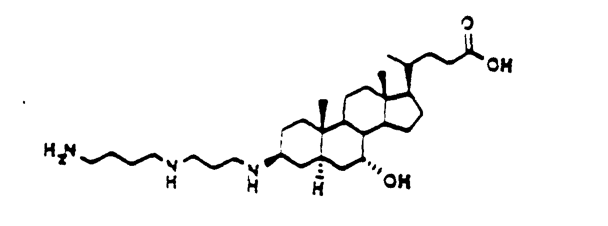 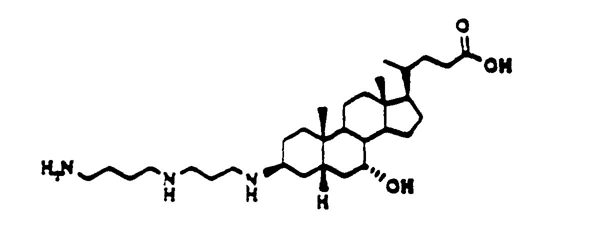 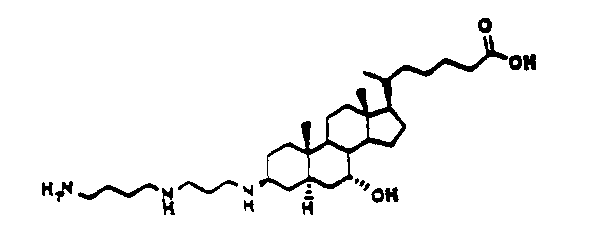 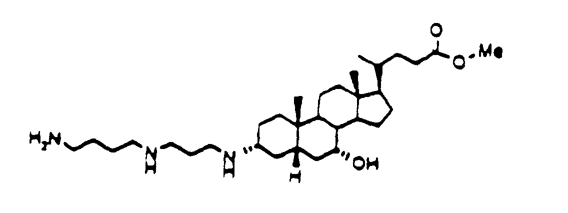 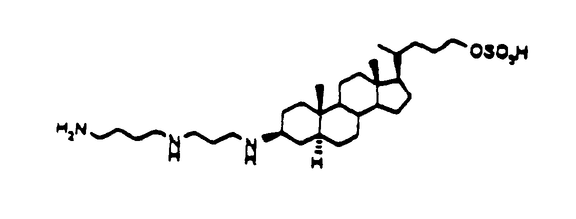 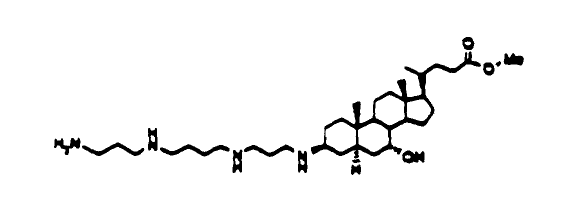 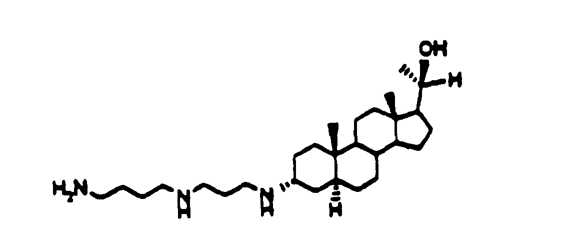 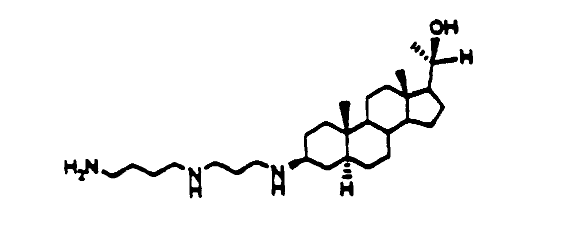 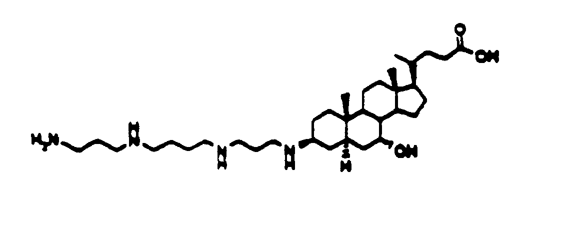 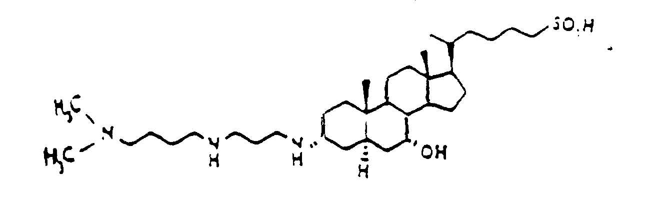 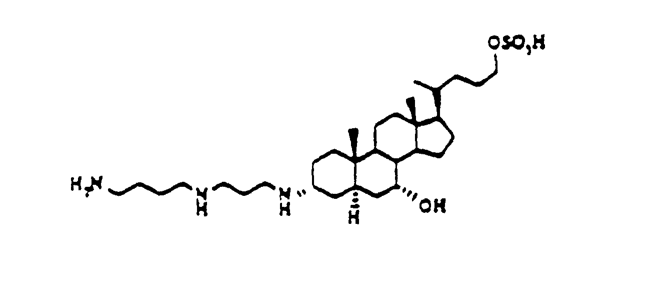 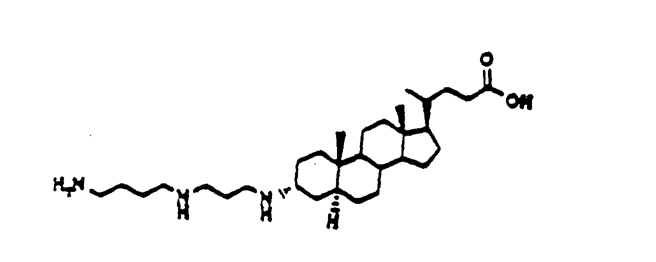 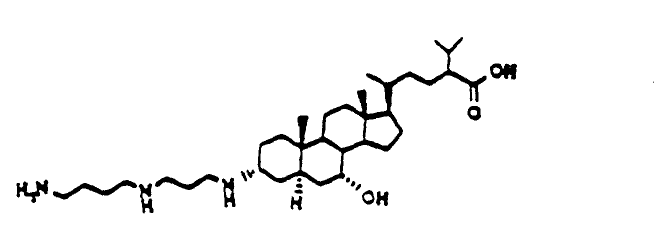 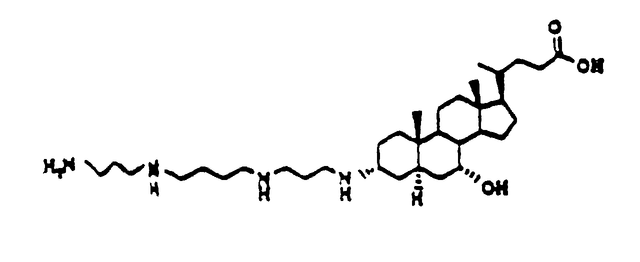 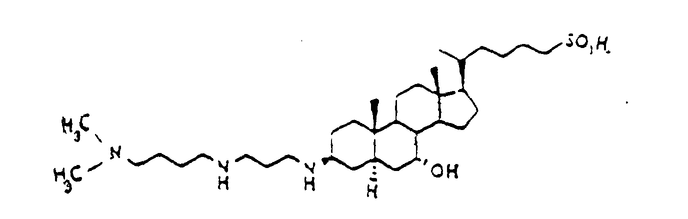 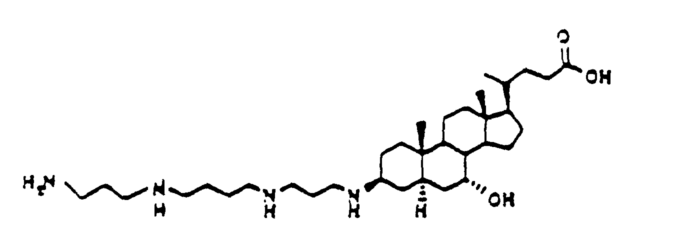 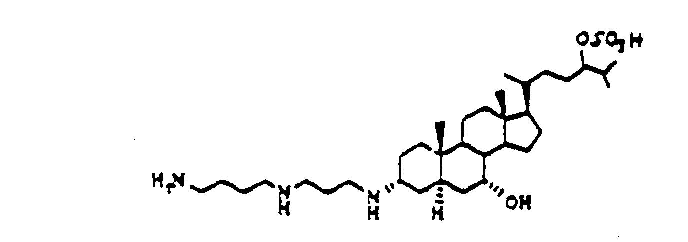 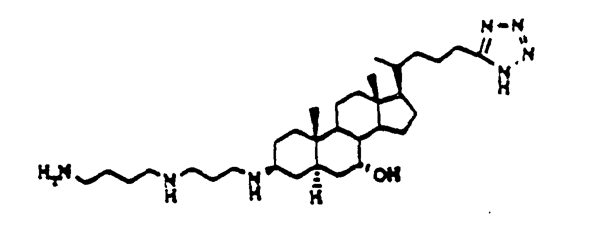 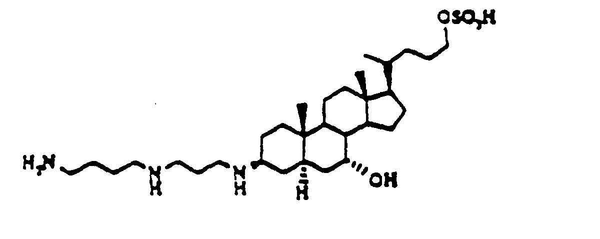 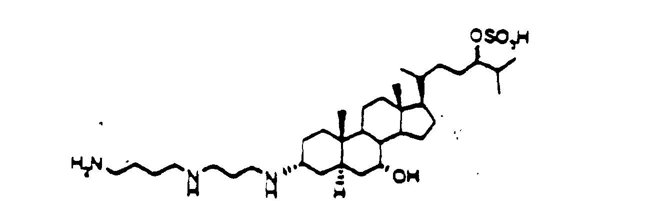 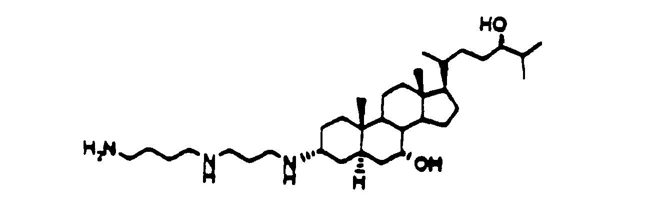 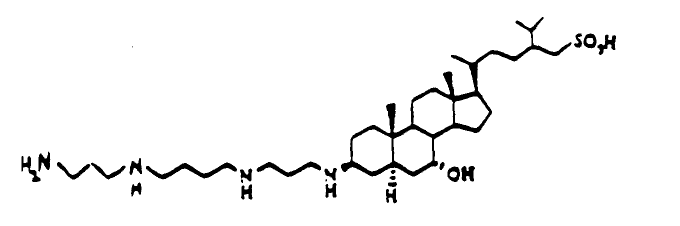 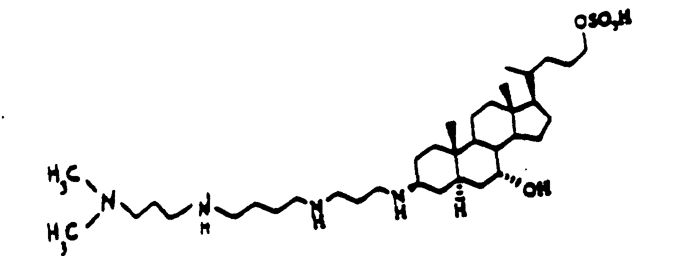 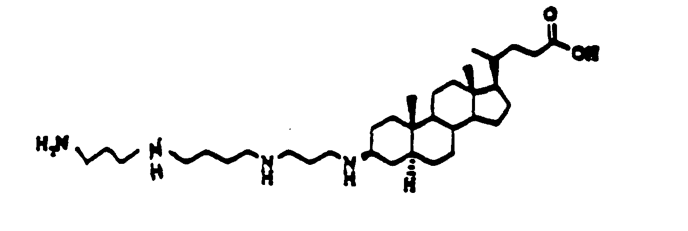 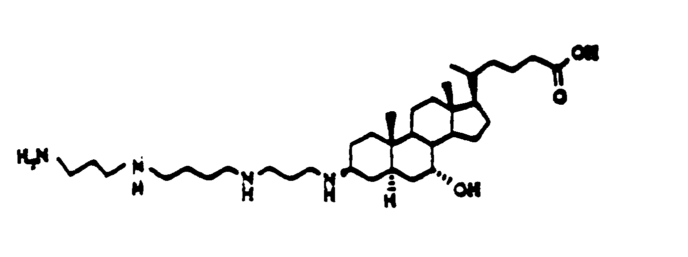 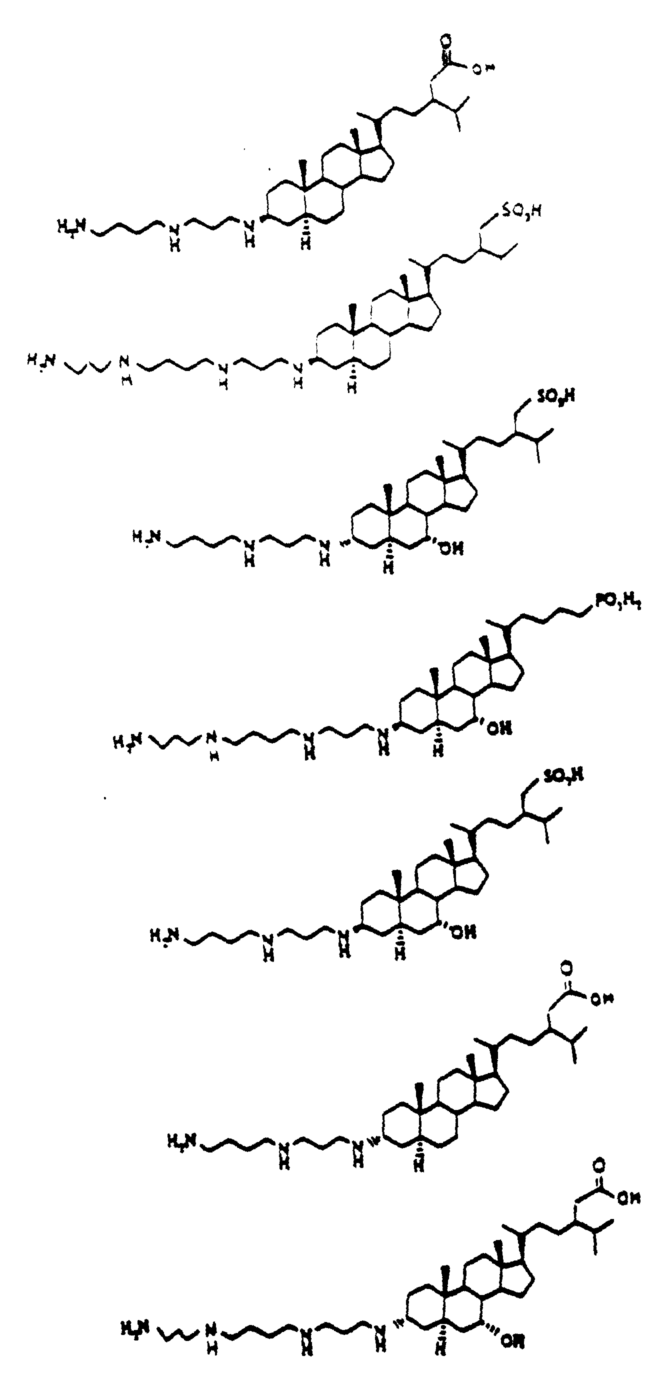 Preferred compounds of the invention are also set forth in the examples below.

### Syntheses

Compounds of the invention may be synthesized as described in the examples below.

### Example A

A suitably protected flat ring nucleus is constructed. In many cases, this flat ring system already has an attached side chain which will become the anionic side chain. Flat ring systems with the anionic side chain attached are synthesized as described in Examples A(1)-(8). Flat ring systems which have at least part of the anionic side chain added are synthesized as described in Examples A(9)-(12). Preparation of the cationic side chain is described in Example A(13). Attachment of the cationic side chain through the C-3 amine, alcohol or thiol is exemplified in Example A(14). Introduction of an anionic group into an anionic side chain is then exemplified. Sulfation is illustrated by Example A(15). Introduction of phosphate is illustrated by Example A(16). Preparation of carboxylates is illustrated by Example A(17).

### Example A(1)

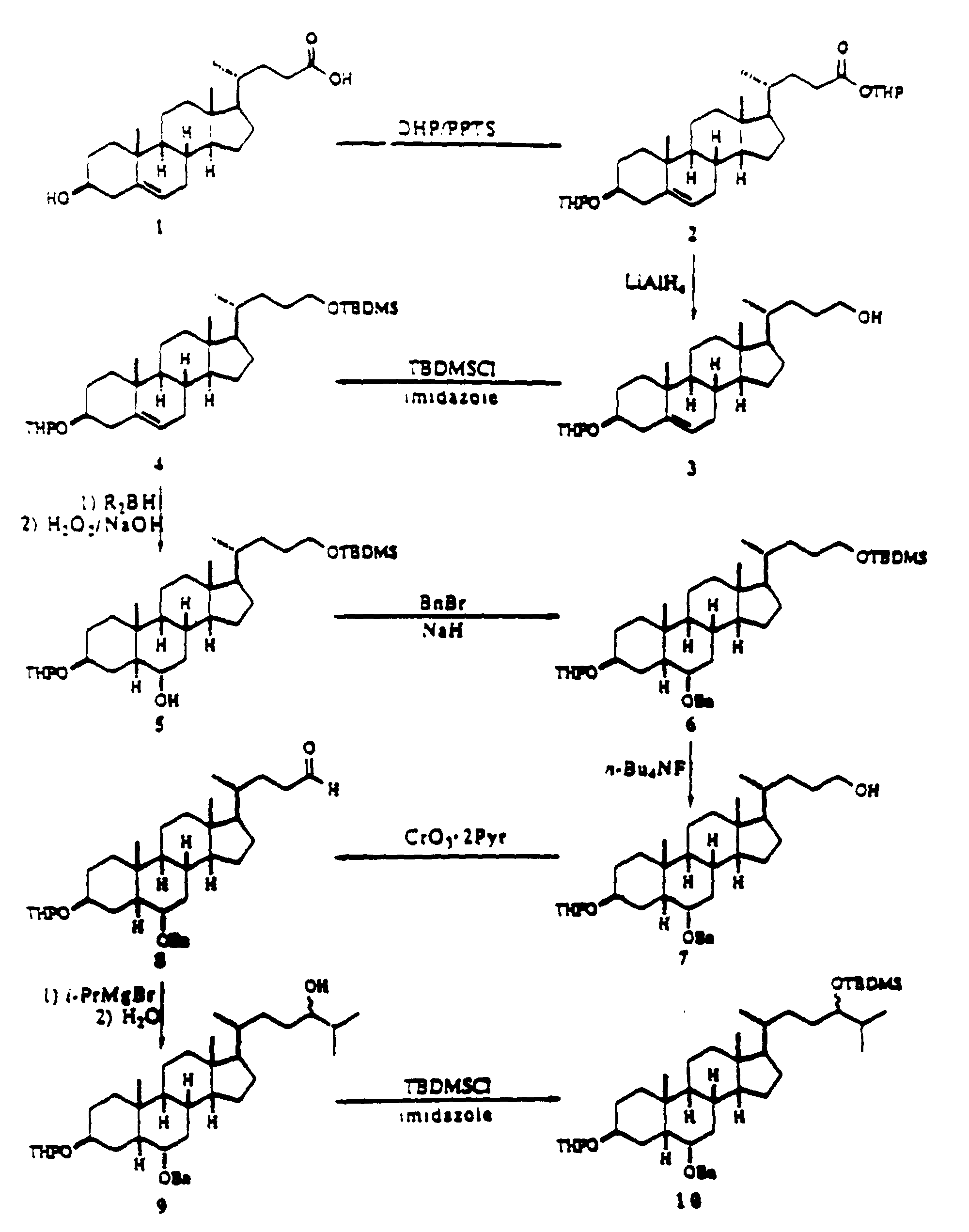

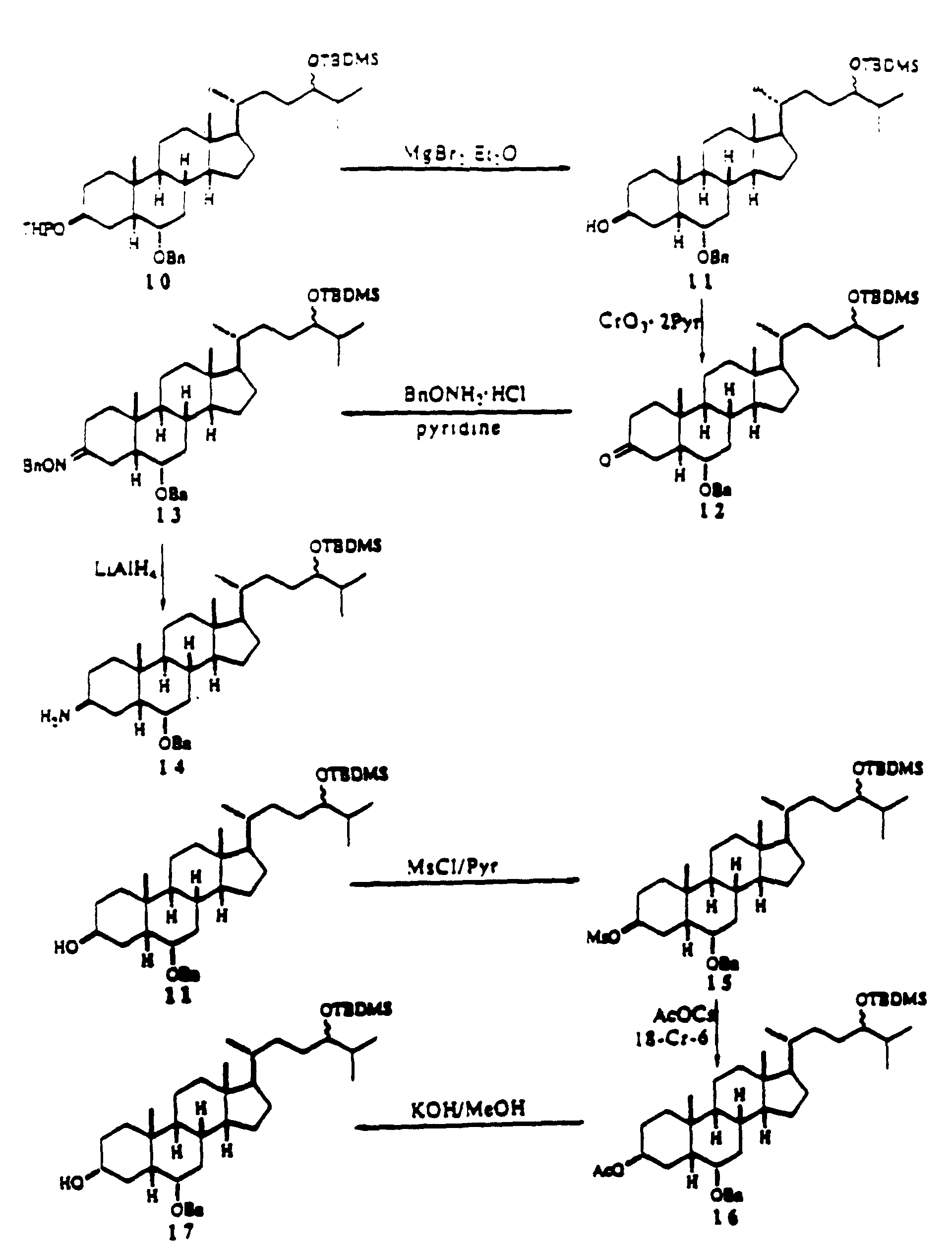

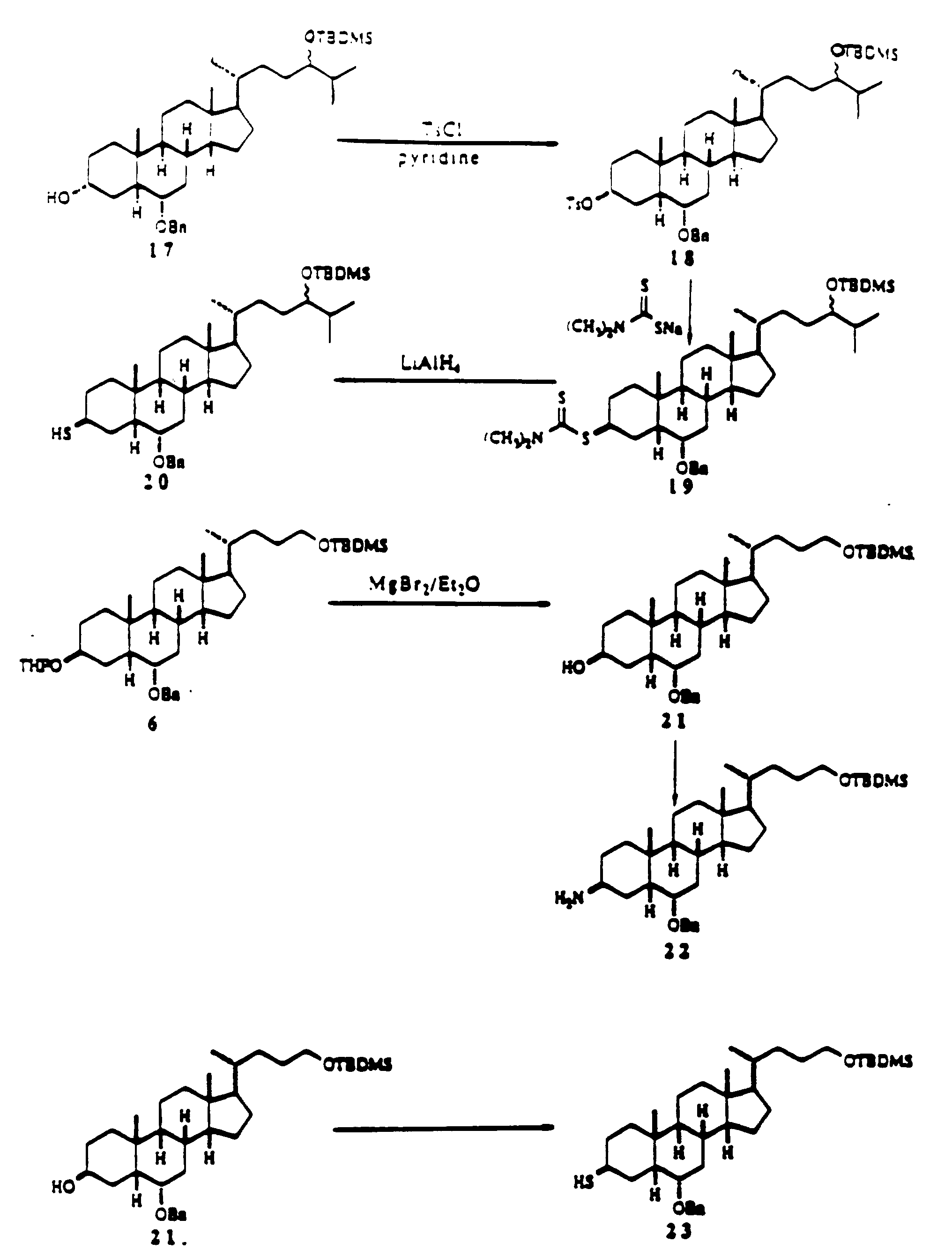

Cholenic acid **1** is treated with dihydropyran (DHP) and a catalytic amount of pyridinium p-toluenesulfonate (PPTS) (M. Miyashita, A. Yoshikoshi, P.A. Grieco, *J. Org. Chem.* 42, 1977, 3772) to give compound **2.** Reduction of compound **2** with lithium aluminum hydride or similar reducing agent gives 24-alcohol **3.** The 24-alcohol is then protected as the t-butyldimethylsilyl (TBDMS) ether by treatment with TBDMS chloride and imidazole (E.J. Corey, A. Venkateswarlu, *J. Am. Chem. Soc.* 94, 1972, 6190). Hydroboration-oxidation of compound **4** results in formation of 6α-alcohol **5** with the desired trans A-B ring junction (S. Wolfe, M. Nussim, Y. Mazur, F. Sondheimer, *J. Org. Chem.* 24, 1959, 1034; K. Burgess, J. Cassidy, M. J. Ohlmeyer, *J. Org. Chem.* 56, 1991, 1020). The 6α-alcohol is then protected as the benzyl ether by treatment with benzyl bromide (BnBr) and sodium hydride (J.D. White, G.N. Reddy, G.O. Spessard, *J. Am. Chem. Soc.* 110, 1988, 1624) to give compound **6.** The TBDMS protecting group is then removed by treatment with tetra-n-butylammonium fluoride to give compound **7** (E.J. Corey, A. Venkateswarlu, *J. Am. Chem.* Soc. 94, 1972, 6190). Oxidation of the resultant 24-alcohol with Collin's reagent (CrO₃ 2Pyr) gives aldehyde **8.** Treatment of this aldehyde with isopropylmagnesium bromide followed by quenching with water gives alcohol **9**. The resultant 24-alcohol is then protected as the TBDMS ether to give compound **10** as described above for the protection of compound **3**. The 3β-tetrahydropyranyl (THP) protecting group is then selectively removed by treatment with magnesium bromide in ether to give 3β-alcohol **11** (S. Kim, J. H. Park, *Tetrahedron Lett.* 28, 1987, 439). Oxidation with Collin's reagent followed by treatment with benzyloxyamine hydrochloride and pyridine gives oxime **13.** Reduction with lithium aluminum hydride gives 3β-amine **14.**

The 3β-alcohol of compound **11** is inverted by the method described by Adam et al. (P. Adam, J.-C. Schmid, P. Albrecht, *Tetrahedron Lett.* 32, 1991, 2955) to give 3α-alcohol **17**. This is accomplished by treatment with mesyl chloride in pyridine to give compound **15,** displacement with cesium acetate to give compound **16,** and hydrolysis with methanolic potassium hydroxide. Treatment of 3α-alcohol **17** with p-toluenesulfonyl chloride in pyridine gives tosylate **18.** Displacement of the tosylate is mediated by treatment with sodium N,N-dimethyldithiocarbamate to give compound **19,** which is reduced with lithium aluminum hydride to give compound **20** (J.L. Wardell, "The Chemistry of the Thiol Group," S. Patai (ed.), John Wiley and Sons, New York, 1974, 519).

Selective deprotection of the 3β-THP of compound **6** with magnesium bromide gives compound **21** (S. Kim, J.H. Park, *Tetrahedron Lett.* 28, 1987, 439). The resultant 3β-alcohol is converted to 3β-amine **22** in a manner analogous to the conversion of compound **11** to compound **14.**

Compound **21** is converted to the corresponding 3β-thiol **23** in a manner analogous to the conversion of compound **11** to compound **20.**

### Example A(2)

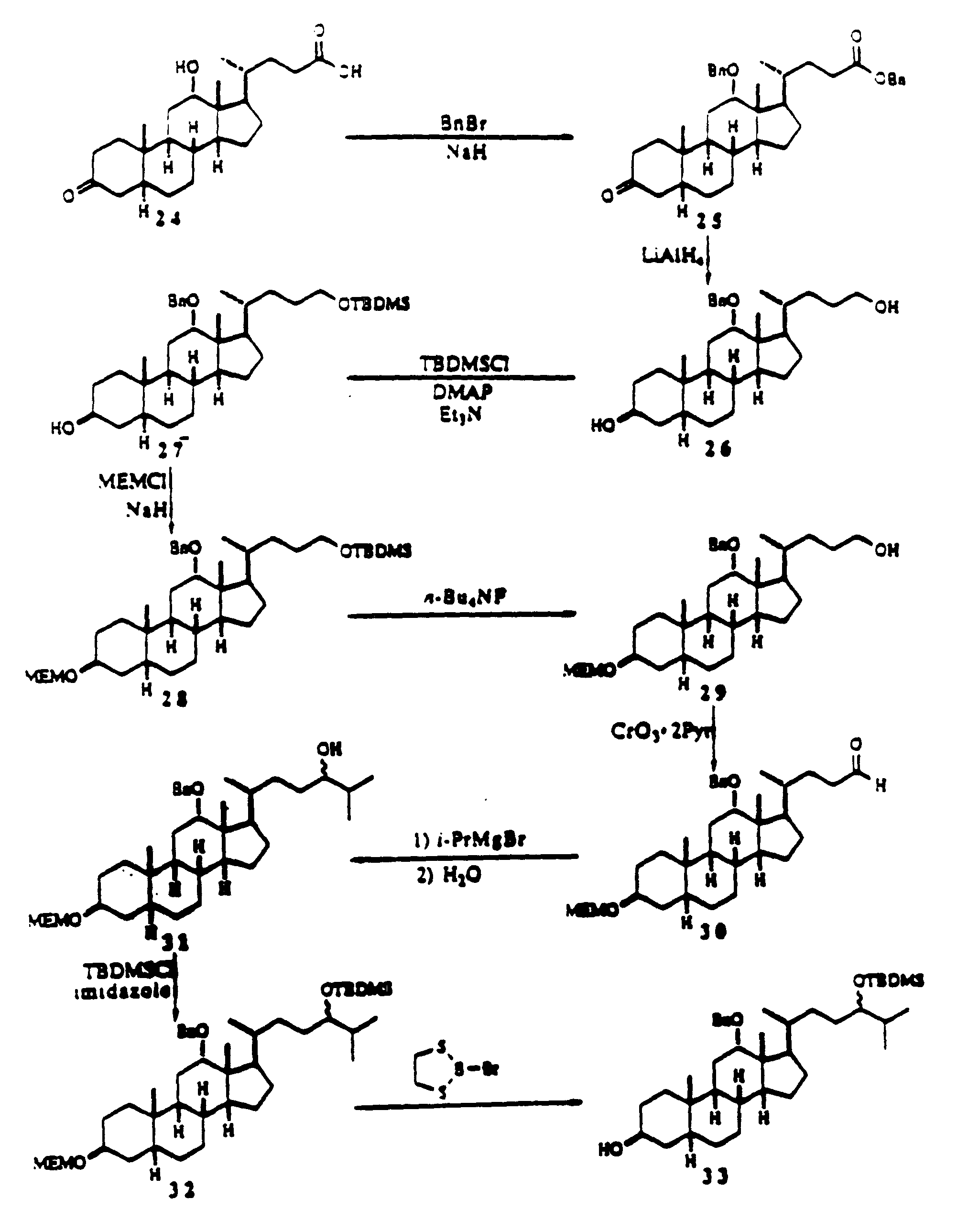

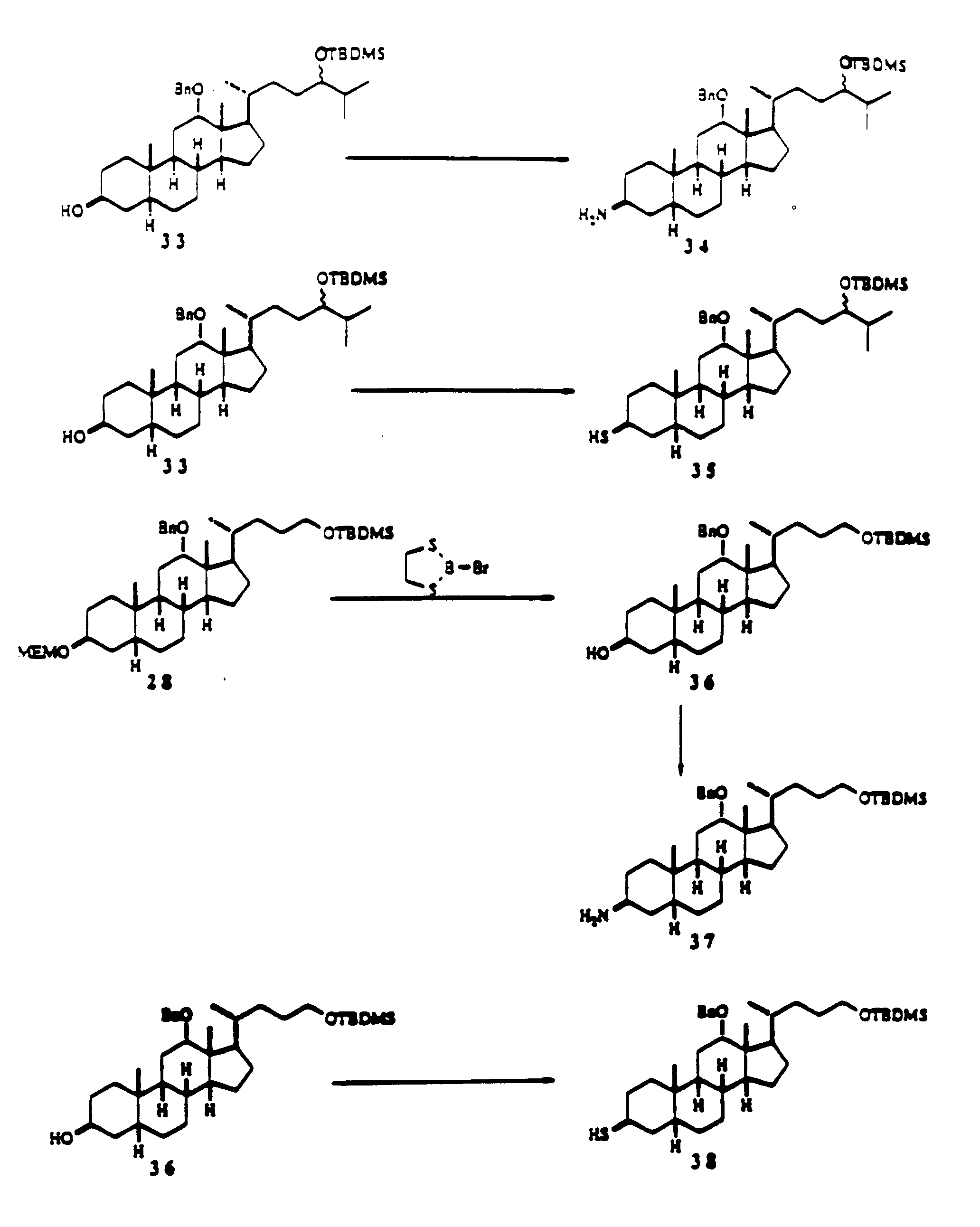

Treatment of known cholenic acid derivative **24** (M.N. Iqbal, W.H. Elliot, *Steroids* 53, 1989, 413) with benzyl bromide and sodium hydride gives compound **25** (J.D. White, G.N. Reddy, G.O. Spessard, *J. Am. Chem. Soc.* 110, 1988, 1624). Reduction with lithium aluminum hydride gives 3β,24-diol **26.** Selective protection of the primary alcohol with TBDMS chloride, dimethylaminopyridine (DMAP), and triethylamine gives compound **27** (S.K. Chaudhary, O. Hernandez, Tetrahedron Lett., 1979, 99). The secondary 3β-alcohol of compound **27** is then protected as the 2-methoxyethoxymethyl (MEM) ether by treatment with MEM chloride and NaH (E.J. Corey, J.-L. Gras, P. Ulrich, *Tetrahedron Lett.,* 1976, 809) to give compound **28.** Removal of the TBDMS ether with tetra-n-butylammonium fluoride (E.J. Corey, A. Venkateswarlu, *J. Am. Chem. Soc.* 94, 1972, 6190) (giving compound **29**), oxidation with Collin's reagent (giving compound **30**), and treatment with isopropylmagnesium bromide gives compound **31.** Protection of the 24-alcohol with TBDMS chloride yields compound **32** (E.J. Corey, A. Venkateswarlu, *J. Am. Chem. Soc.* 94, 1972, 6190). Selective removal of the MEM protecting group of the 3β-alcohol gives compound **33** (D.R. Williams, S. Sakdarat, *Tetrahedron Lett.* 24, 1983, 3965).

3β-Alcohol **33** is converted to the corresponding 3β-amine **34** in a manner analogous to the conversion of compound **11** to compound **14.** 3β-Alcohol **33** is converted to the corresponding 3β-thiol **35** in a manner analogous to the conversion of compound **11** to compound **20.**

The MEM protecting group of the 3β-alcohol of compound **28** is selectively removed to give compound **36** (D.R. Williams, S. Sakdarat, *Tetrahedron Lett.* 24, 1983, 3965). 3β-Alcohol **36** is converted to 3β-amine **37** in a manner analogous to the conversion of compound **11** to compound **14.** Compound **36** is converted the corresponding 3β-thiol, compound **38,** in a manner analogous to the conversion of compound **11** to compound **20.**

### Example A(3)

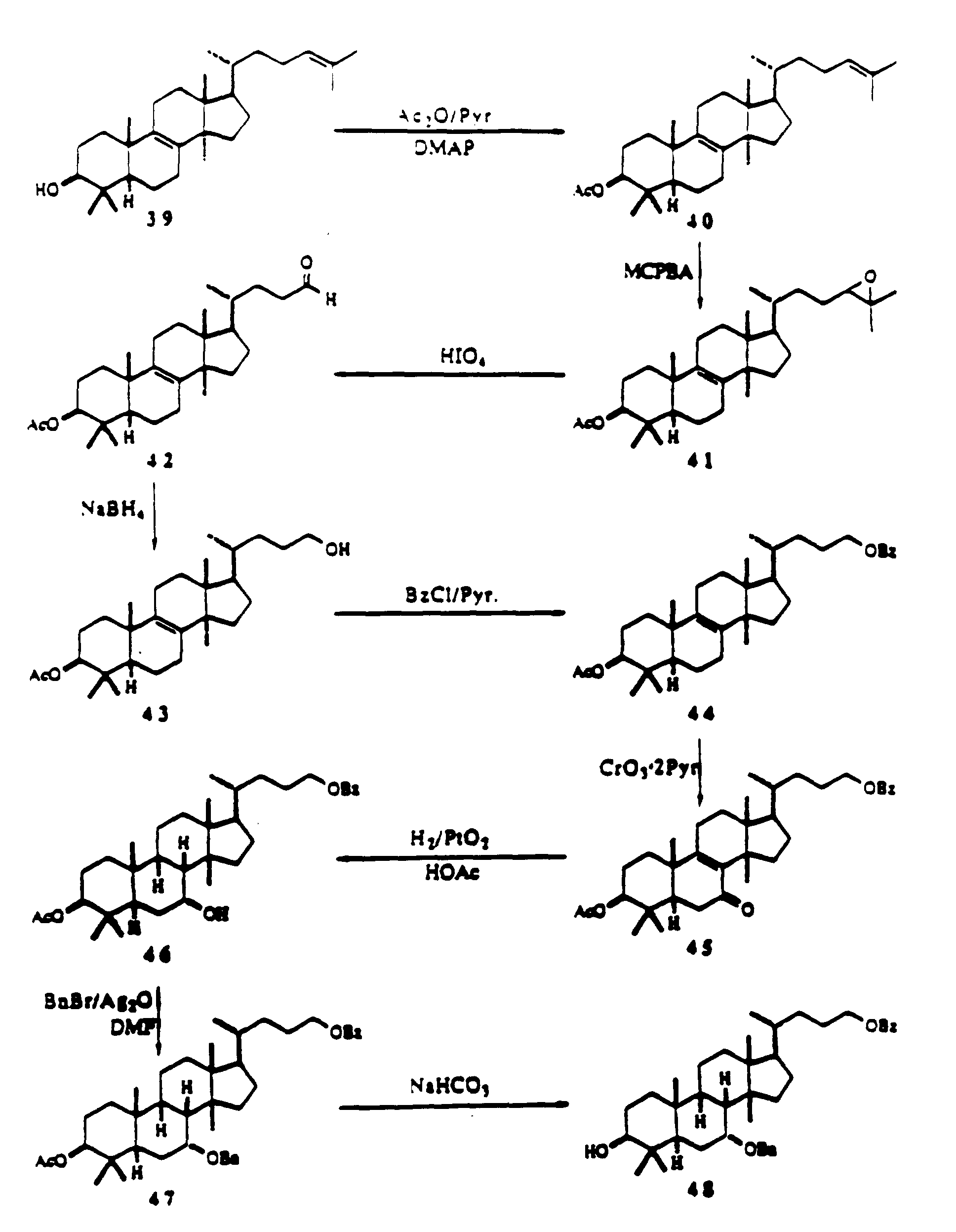

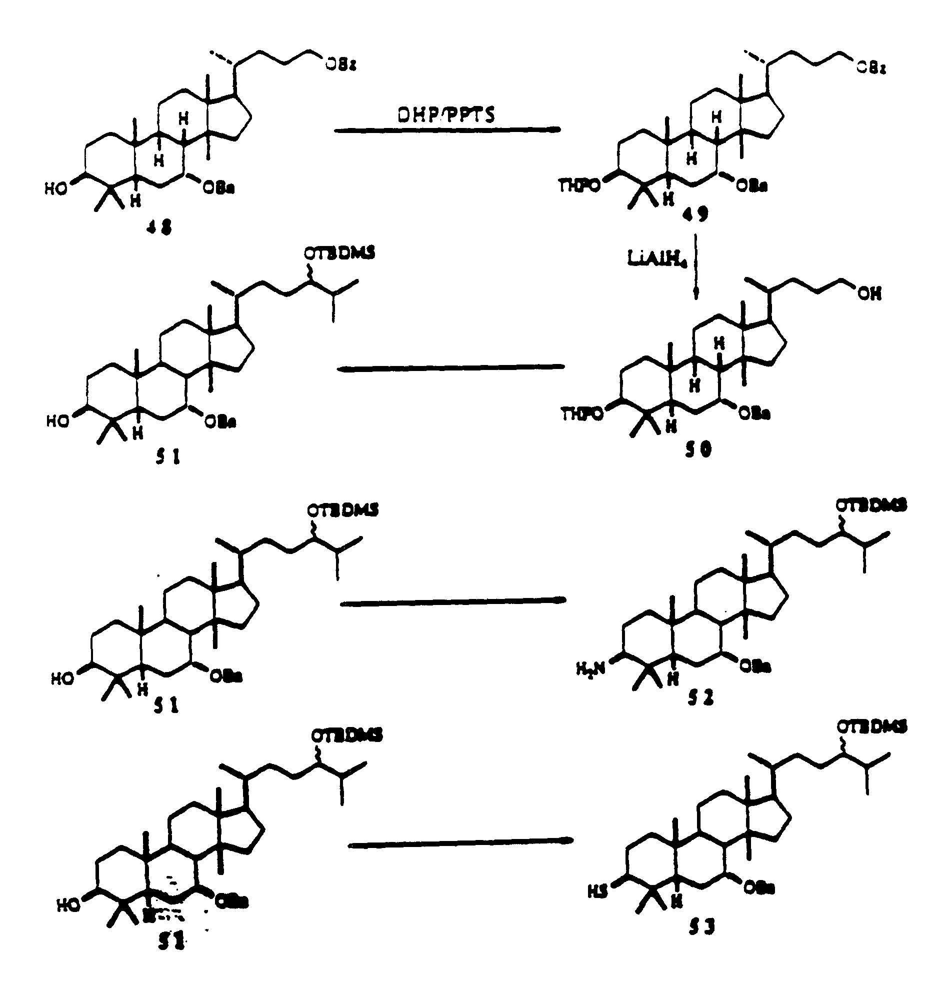

Lanosterol **39** is acetylated by treatment with acetic anhydride, pyridine, and a catalytic amount of DMAP to give compound **40.** The 24-double bond is selectively epoxidized by treatment with m-chloroperoxybenzoic acid (MCPBA) as described by Sato and Sonoda (Y. Sato, Y. Sonoda, *Chem. Pharm. Bull.* 29, 1987, 356). The epoxide **41** is oxidatively cleaved by treatment with periodic acid to give aldehyde **42** (J.P. Nagarkatti, K.R. Ashley, Tetrahedron Lett., 1973, 4599). Reduction of 24-aldehyde **42** with sodium borohydride or other appropriate reducing agent gives alcohol **43.** Treatment with benzoyl chloride and pyridine gives compound **44.** Treatment of compound **44** with Collin's reagent results in allylic oxidation product **45** (W.G. Salmond, M.A. Barta, J.L. Havens, *J. Org. Chem.* 43, 1978, 2057). Reduction of the double bond, epimerization, followed by reduction of the ketone is accomplished by treatment of compound **45** with hydrogen in acetic acid with a catalytic amount of Adam's catalyst (PtO₂) (Y. Sonoda, Y. Tanoue, M. Yamaguchi, Y. Sato, *Chem. Pharm. Bull.* 35, 1987, 394) to give compound **46.** Protection of the 7α-alcohol as the benzyl ether with benzyl bromide and silver oxide gives compound **47** (L. Van Hijfte, R.D. Little, *J. Org. Chem.* 50, 1985, 3940). The acetate at C-3 of compound **47** is then removed by treatment with sodium bicarbonate. The resultant alcohol **48** is then protected as the THP ether to give compound **49** (M. Miyashita, A. Yoshikoshi, P.A. Grieco, *J. Org. Chem.* 42, 1977, 3772). The benzoate protecting group of the 24-alcohol is removed with lithium aluminum hydride to give compound **50.** Compound **50** is then converted to compound **51** in a manner analogous to the conversion of compound **7** to compound **11.**

3β-Alcohol **51** is converted to 3β-amine **52** in a manner analogous to the conversion of compound **11** to compound **14.**

3β-Alcohol **51** is converted to 3β-thiol **53** in a manner analogous to the conversion of compound **11** to compound **20.**

### Example A(4)

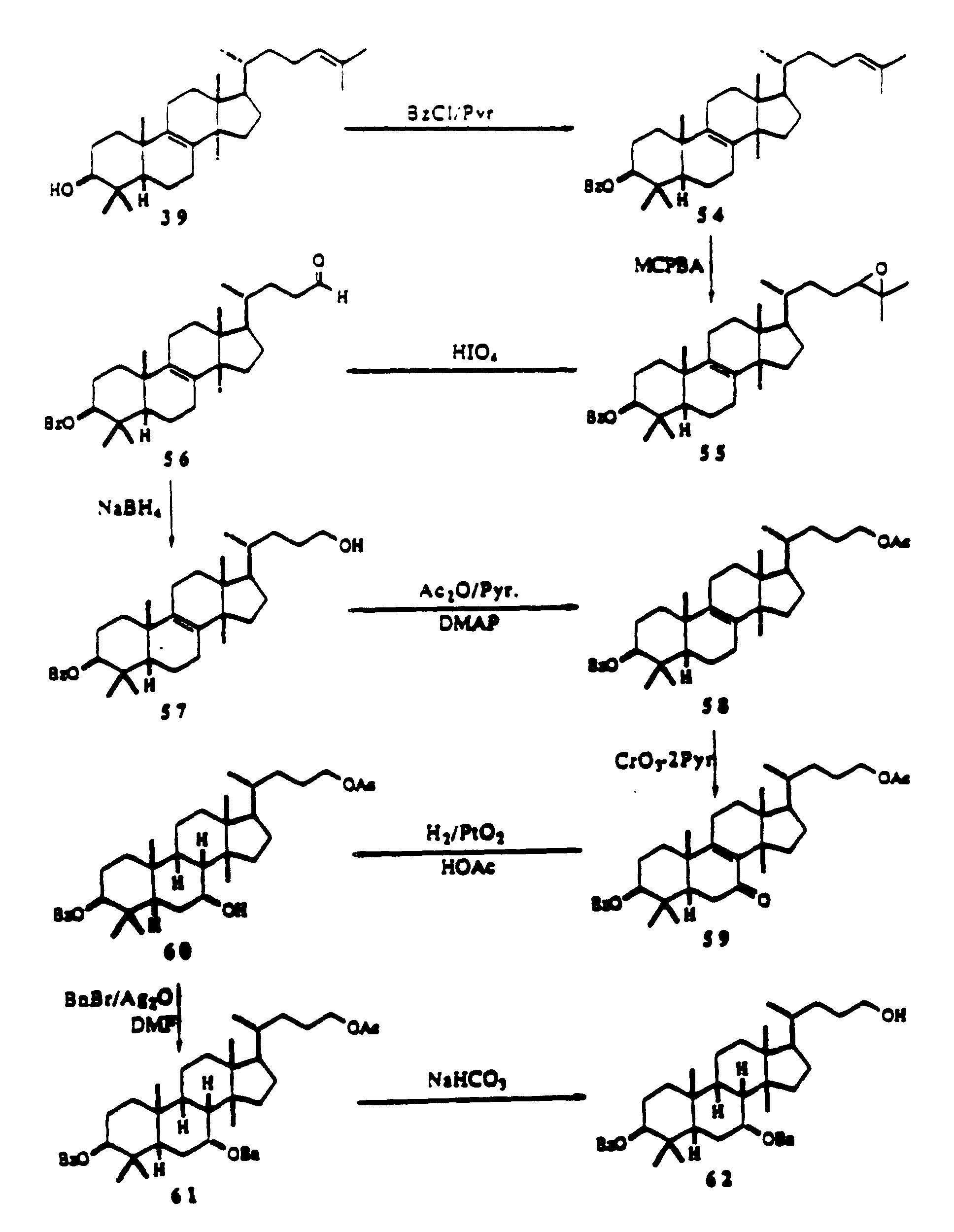

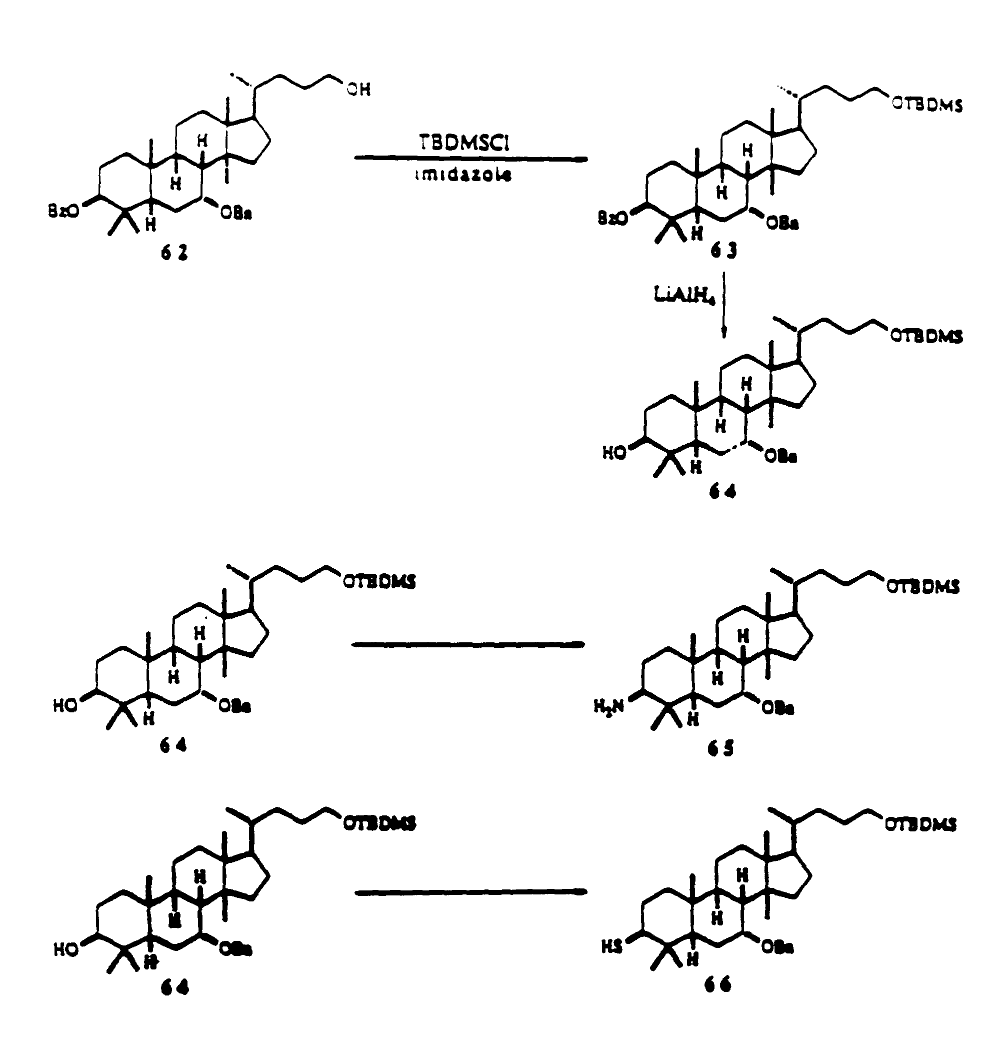

Lanosterol **39** is created with benzoyl chloride/pyridine to give compound **54,** MCPBA to give compound **55,** and periodic acid to give aldehyde **56** in a manner similar to the conversion of compound **39** to compound **42.** Reduction with sodium borohydride gives compound **57.** The resultant alcohol **57** is protected as the corresponding acetate **58.** Allylic oxidation (giving compound **59**) followed by reduction gives compound **60.** The resultant 7α-alcohol is protected as the benzyl ether by treatment with benzyl bromide and silver oxide in DMF (L. Van Hijfte, R.D. Little, *J. Org. Chem.* 50, 1985, 3940) to give compound **61.** The acetate is removed by treatment with sodium bicarbonate and the resultant alcohol **62** is protected as the TBDMS ether by treatment with TBDMS chloride and imidazole, giving compound **63.** 3β-Alcohol **64** is obtained by reductive removal of the benzoate by treatment with lithium aluminum hydride.

Compound **64** is converted to 3β-amino compound **65** in a manner analogous to the conversion of compound **11** to compound **14.**

Compound **64** is converted to the corresponding 3β-thiol, compound **66,** in a manner analogous to the conversion of compound **11** to compound **20.**

### Example A(5)

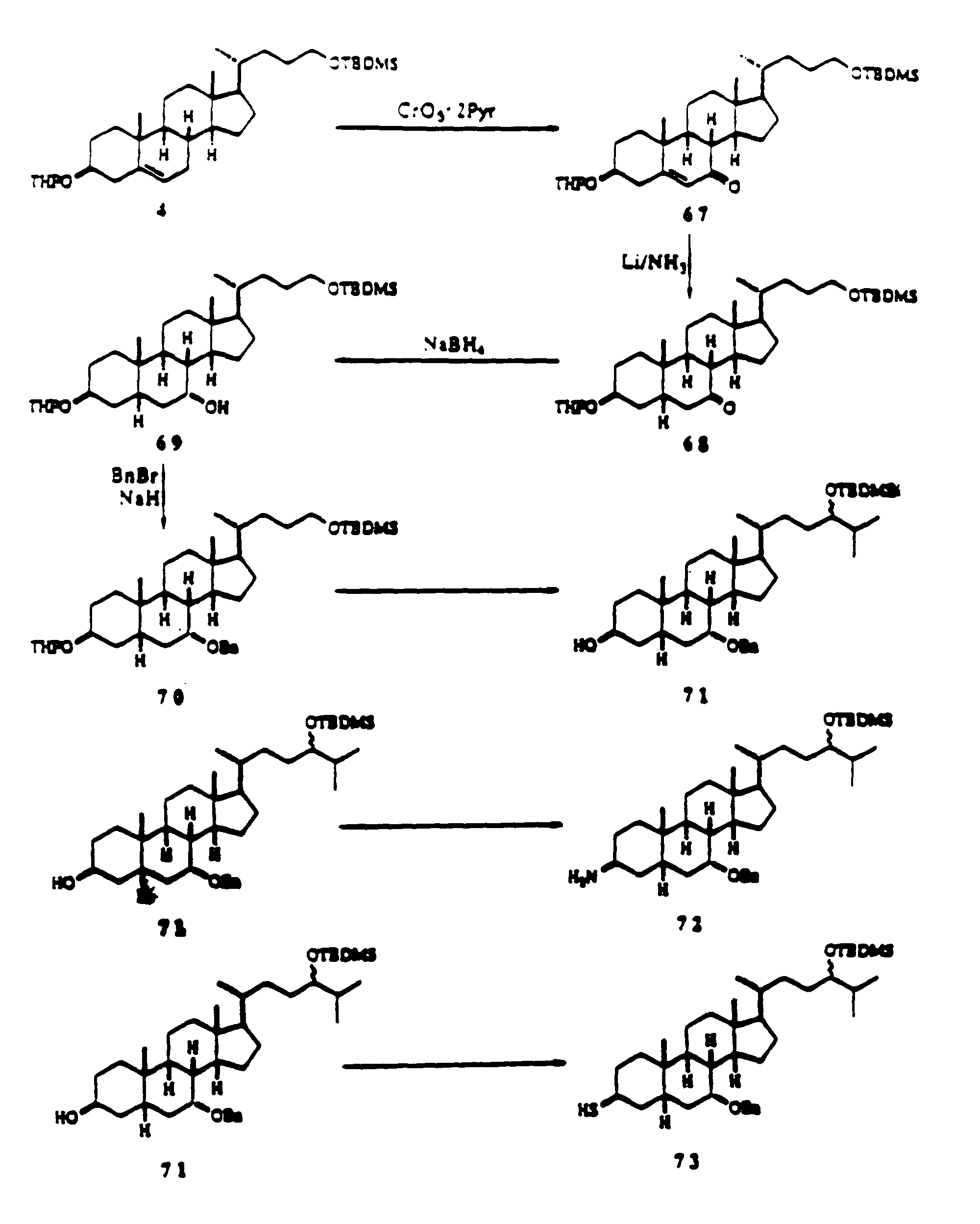

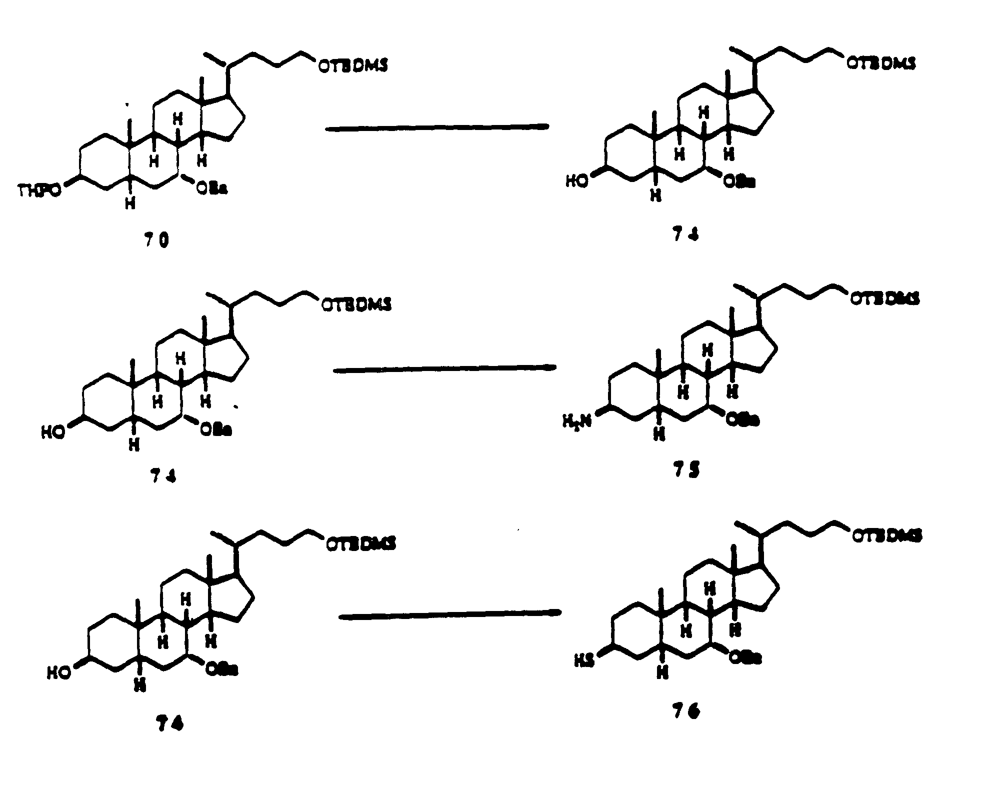

Allylic oxidation of compound **4** with Collin's reagent (W.G. Salmond, M.A. Barta, J.L. Havens, *J. Org. Chem.* 43, 1978, 2057) gives enone **67.** Reduction of the resultant enone with lithium in ammonia (H.L. Dryden, Jr., "Organic Reactions in Steroid Chemistry," vol. 1, J. Fried and J.A. Edwards (eds.), Van Norstrand Reinhold Co., New York, 1972, 27-31) results in formation of ketone **68.** Reduction of compound **68** with sodium borohydride followed by protection of the resultant alcohol **69** as the benzyl ether gives compound **70.** Compound **70** is converted to compound **71** in a manner analogous to the conversion of compound **6** to compound **11.** 3β-Alcohol **71** is transformed into the corresponding 3β-amine **72** in a manner analogous to the conversion of compound **11** to compound **14.** 3β-Thiol **73** is prepared from compound **71** in a manner analogous to the conversion of compound **11** to compound **20.**

Selective removal of the THP protecting group of compound **70** to give compound **74** is accomplished by treatment with magnesium bromide in ether (S. Kim, J.H. Park, *Tetrahedron Lett.* 28, 1987, 439). Compound **74** is converted to the corresponding 3β-amine **75** in a manner analogous to the conversion of compound **11** to compound **14.** Compound **74** is converted to compound **76** in a manner analogous to the conversion of compound **11** to compound **20.**

### Example A(6)

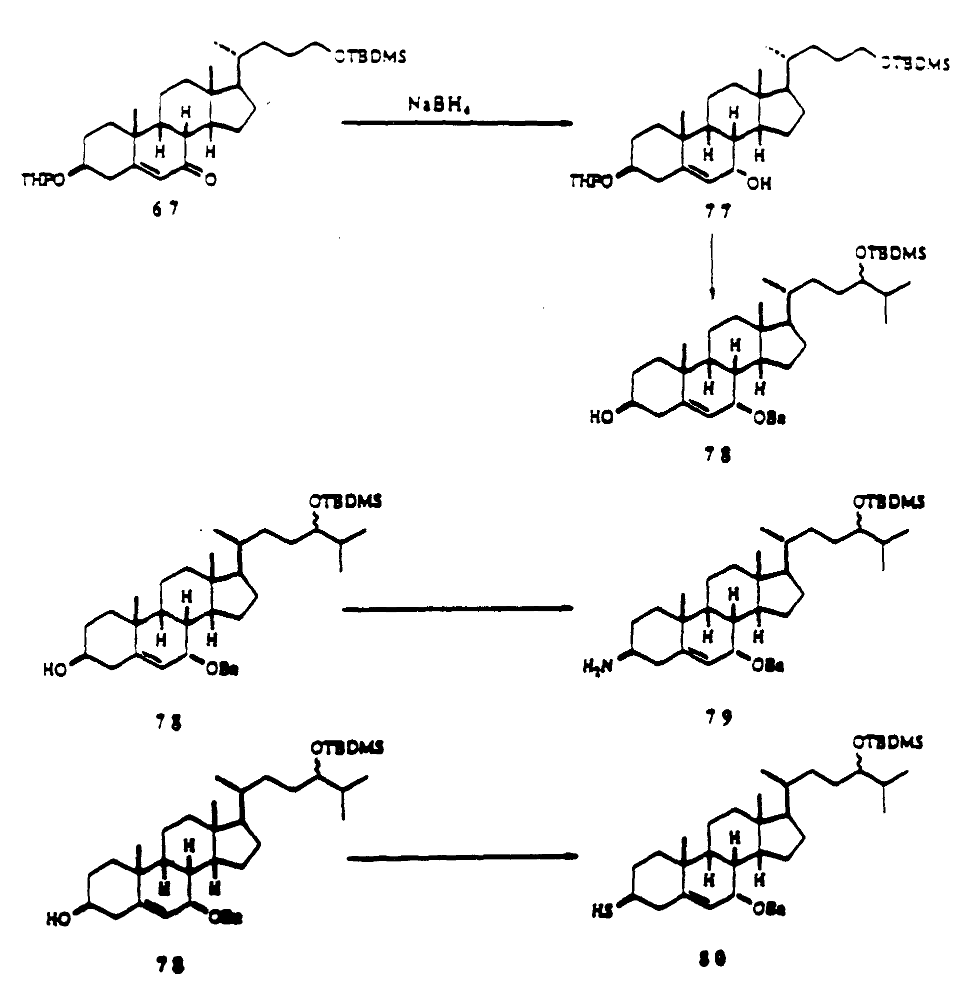

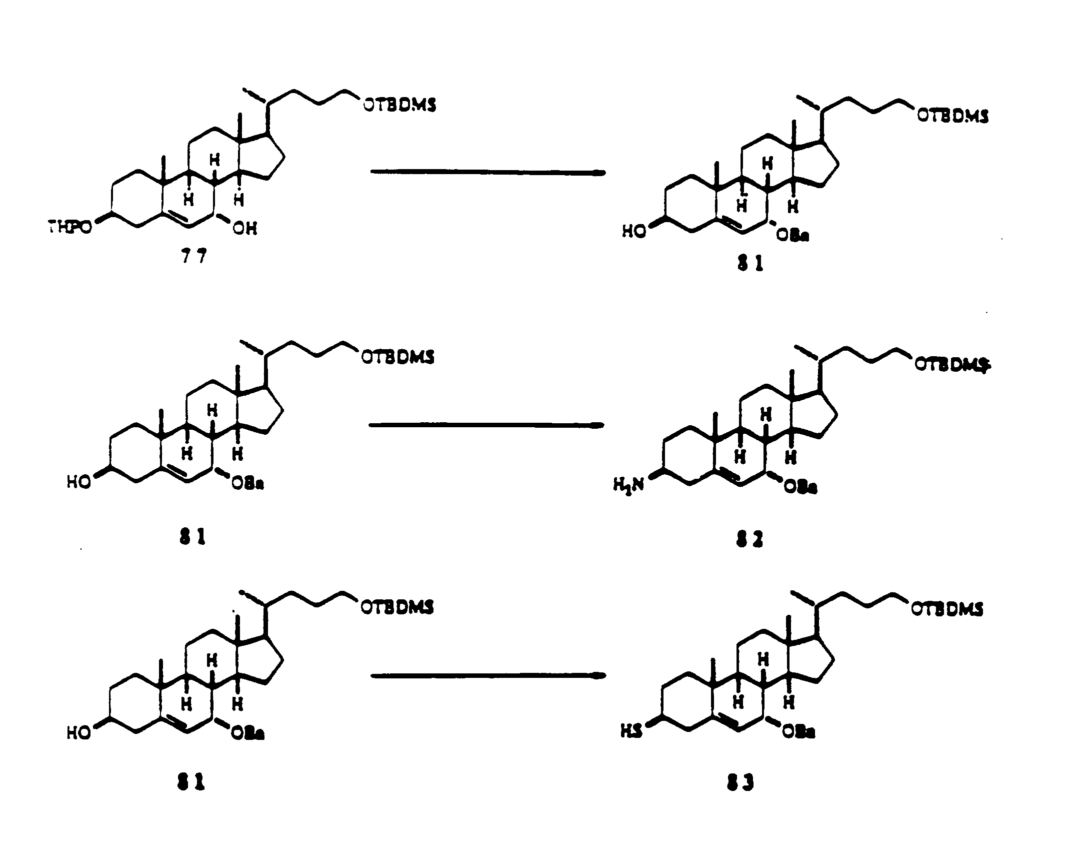

Enone **67** is reduced with sodium borohydride to give allylic alcohol **77.** Compound **77** is then converted to compound **78** in a manner analogous to the conversion of compound **5** to compound **11.** Compound **78** is converted to compound **79** in a manner analogous to the conversion of compound **11** to compound **14.** 3β-Alcohol **78** is converted to 3β-thiol **80** in a manner analogous to the conversion of compound **11** to compound **20.**

Protection of allylic alcohol **77** with benzyl bromide and sodium hydride followed by removal of the THP protecting group at C-3 by treatment with magnesium bromide (S. Kim, J.H. Park, *Tetrahedron Lett.* 28, 1987, 439) gives compound **81.** Conversion of 3β-alcohol **81** to the corresponding 3β-amine, compound **82,** is accomplished in a manner analogous to the conversion of compound **11** to compound **14.** Compound **81** is converted to compound **83** in a manner analogous to the conversion of compound **11** to compound **20.**

### Example A(7)

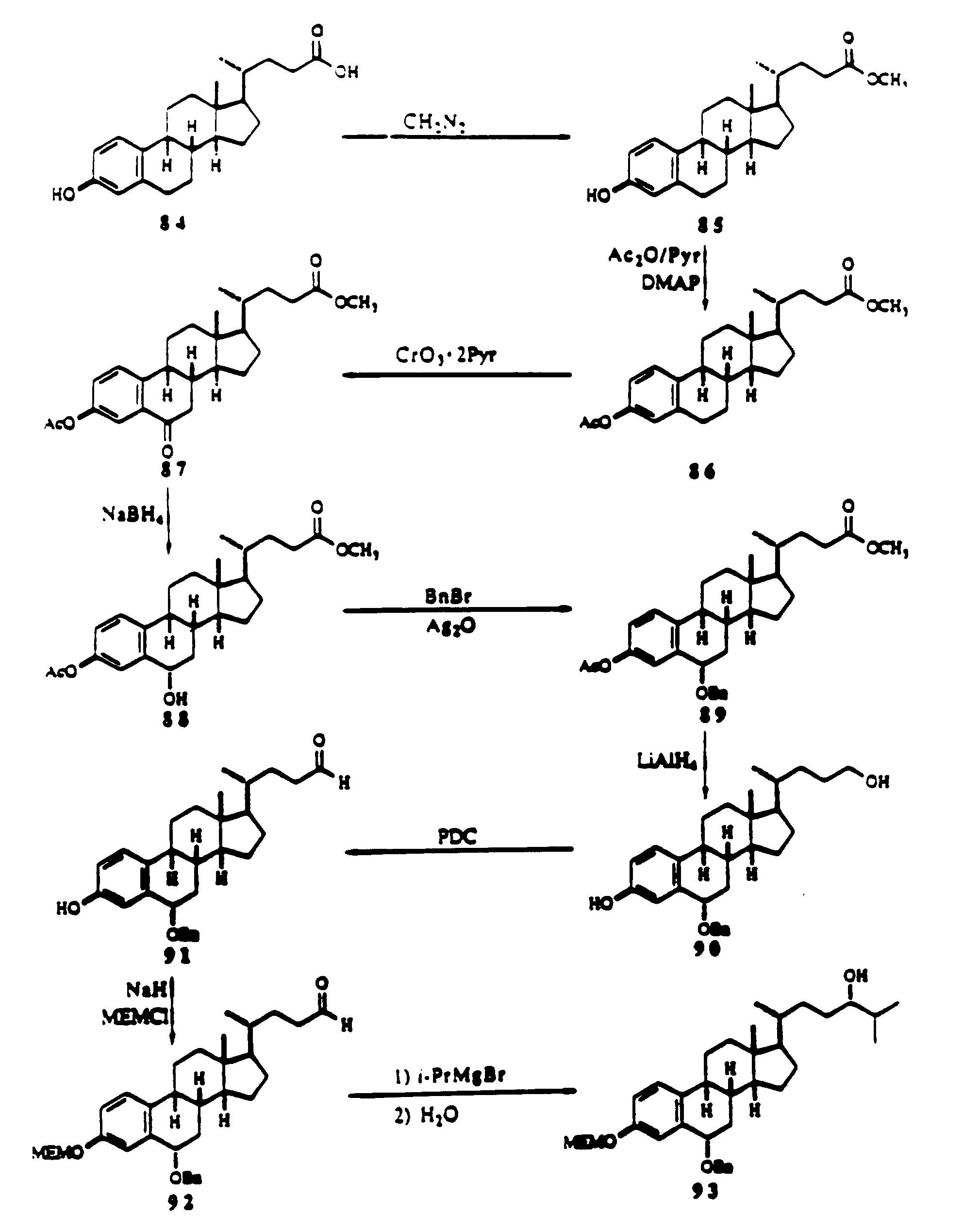

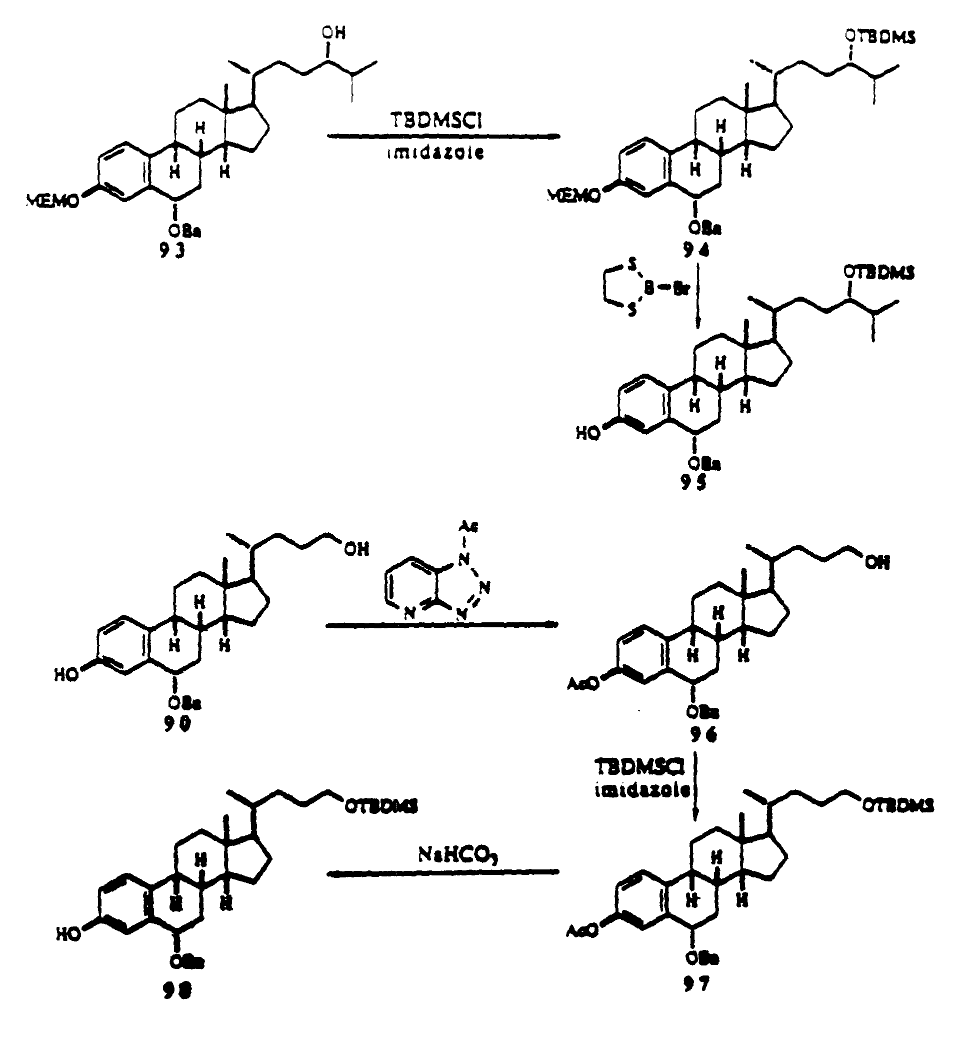

Known estrogen analog **84** (T. Namba, T. Hirota, S. Hayakawa, *J. Lipid Res.* 29, 1988, 809) is treated with diazomethane to afford methyl ester **85.** The phenolic hydroxyl is then acetylated with acetic anhydride/pyridine in the presence of a catalytic amount of DMAP. Benzylic oxidation of compound **86** with Collin's reagent (G.A. Garza, P.N. Rao, *Steroids* 42, 1983, 469) gives ketone **87.** Reduction of this ketone with sodium borohydride gives 7α-alcohol **88** (O. Wintersteiner, M. Moore, *J. Am. Chem. Soc.* 81, 1959, 442). Protection of the resultant alcohol as the benzyl ether (giving compound **89**) followed by reduction of the esters with lithium aluminum hydride gives compound **90.** Oxidation of the primary alcohol of compound **90** with pyridinium dichromate (PDC) (E.J. Corey, G. Schmidt, *Tetrahedron Lett.,* 1979, 399) gives aldehyde **91.** Protection of the phenolic alcohol as MEM ether (E.J. Corey, J.-L. Gras, P. Ulrich, *Tetrahedron Lett.,* 1976, 809) (giving compound **92**) followed by treatment with isopropylmagnesium bromide and subsequent hydrolysis yields compound **93.** Protection of the resultant 24-alcohol as TBDMS ether (E. J. Corey, A. Venkateswarlu, *J. Am. Chem. Soc.* 94, 1972, 6190) (giving compound **94**) and selective removal of the MEM protecting group (D.R. Williams, S. Sakdarat, *Tetrahedron Lett.* 24, 1983, 3965) gives compound **95.**

Selective protection of the phenolic alcohol of compound **90** by treatment with 1-acetyl-v-triazolo-[4,5-b]pyridine (M.P. Paradist, G.P. Zecchini, I. Torrini, *Tetrahedron Lett.* 27, 1986, 5029) gives compound **96.** The 24-alcohol of compound **96** is protected as the TBDMS ether (giving compound **97**) and the acetate is removed by treatment with sodium bicarbonate to give compound **98.**

### Example A(8)

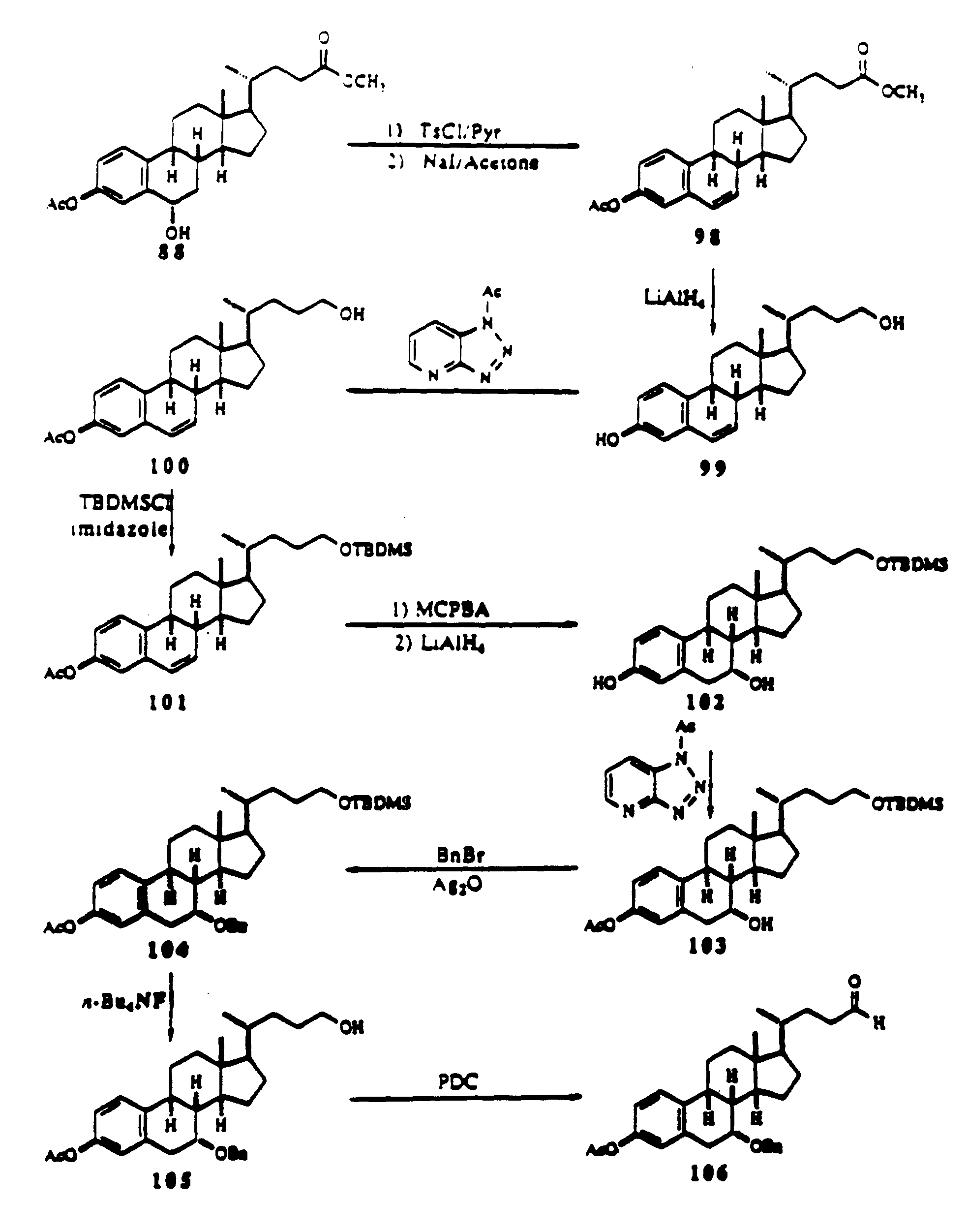

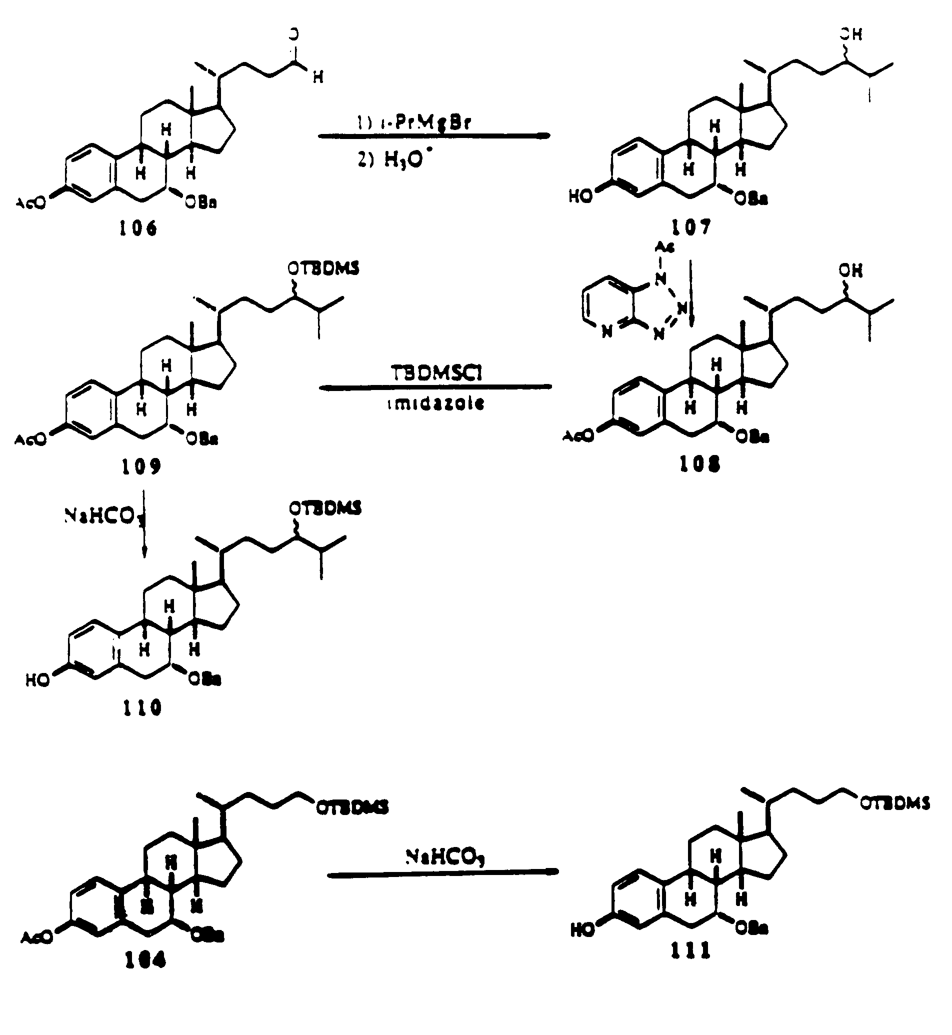

Compound **88** is treated with tosyl chloride and pyridine followed by sodium iodide in acetone to give unsaturated compound **98** (T. Arunachalam, C. Longcope, E. Caspi, *J. Org. Chem.* 254, 1979, 5900). Reduction of the esters with lithium aluminum hydride, giving compound **99,** followed by selective acetylation (M.P. Paradist, G.P. Zecchini, I. Torrini, *Tetrahedron Lett.* 27, 1986, 5029) gives compound **100.** The 24-alcohol is then protected as the TBDMS ether to yield compound **101.** Treatment with MCPBA affords the 6α,7α-oxide, which is reduced with lithium aluminum hydride to yield 7α-alcohol **102** (J. Iriarte, H.J. Ringoid, C. Djerassi, *J. Am. Chem. Soc.* 80, 1958, 6105). The phenolic alcohol is then reprotected as the acetate (giving compound-**103**) and the 7α-alcohol is protected as the benzyl ether (L. Van Hijfte, R.D. Little, *J. Org. Chem.* 50, 1985, 3940) to give compound **104.** Removal of the TBDMS protecting group (giving compound **105**) followed by oxidation with PDC (E.J. Corey, G. Schmidt, *Tetrahedron Lett.,* 1979, 399) gives aldehyde **106.** This aldehyde is treated with isopropylmagnesium bromide to give 24-alcohol **107.** Selective acetylation of the phenolic alcohol (giving compound **108**), protection of the 24-alcohol as the TBDMS ether (compound **109**), and removal of the phenolic acetate yields compound **110.**

Compound **104** is deprotected by treatment with sodium bicarbonate to give compound **111.**

### Example A(9)

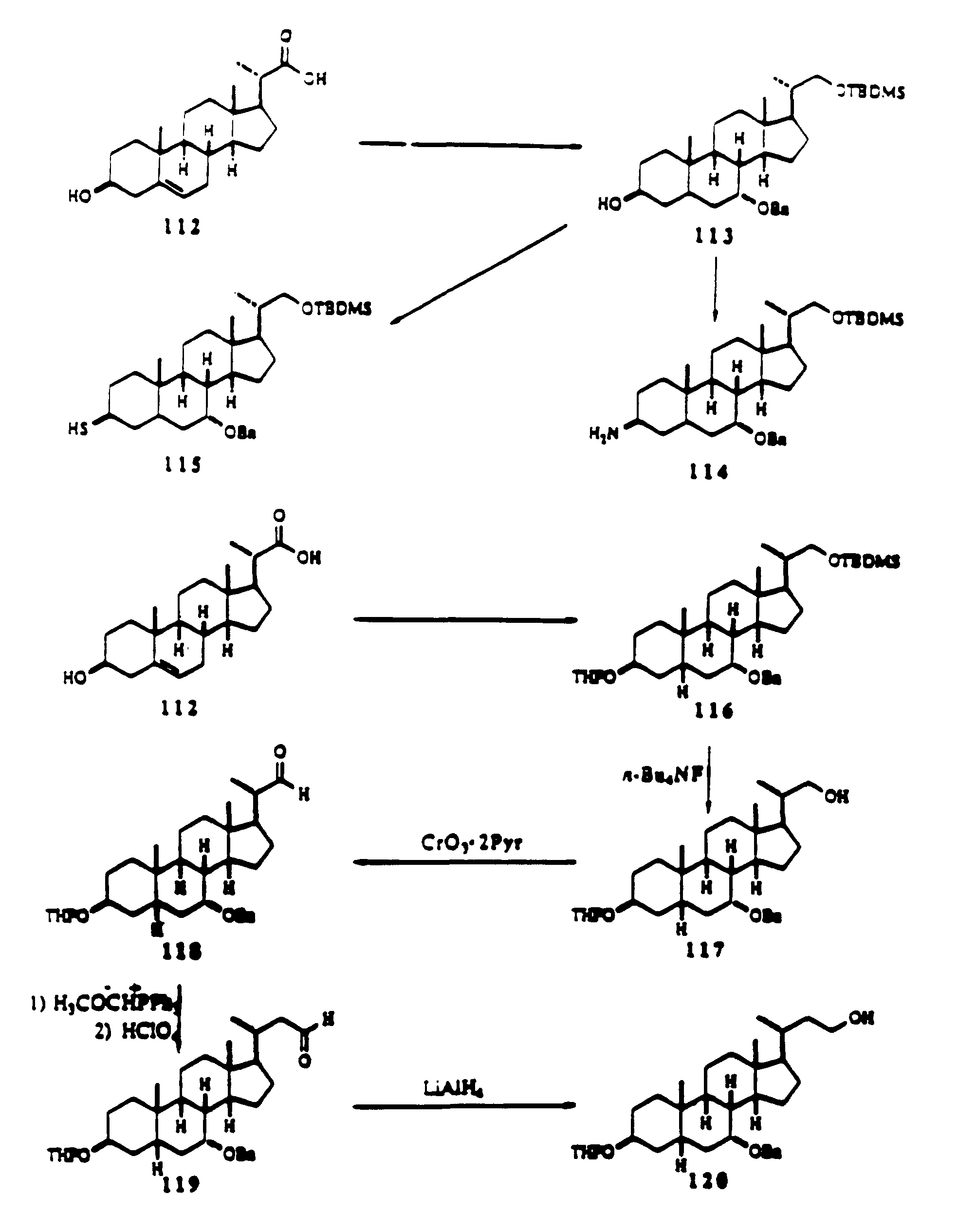

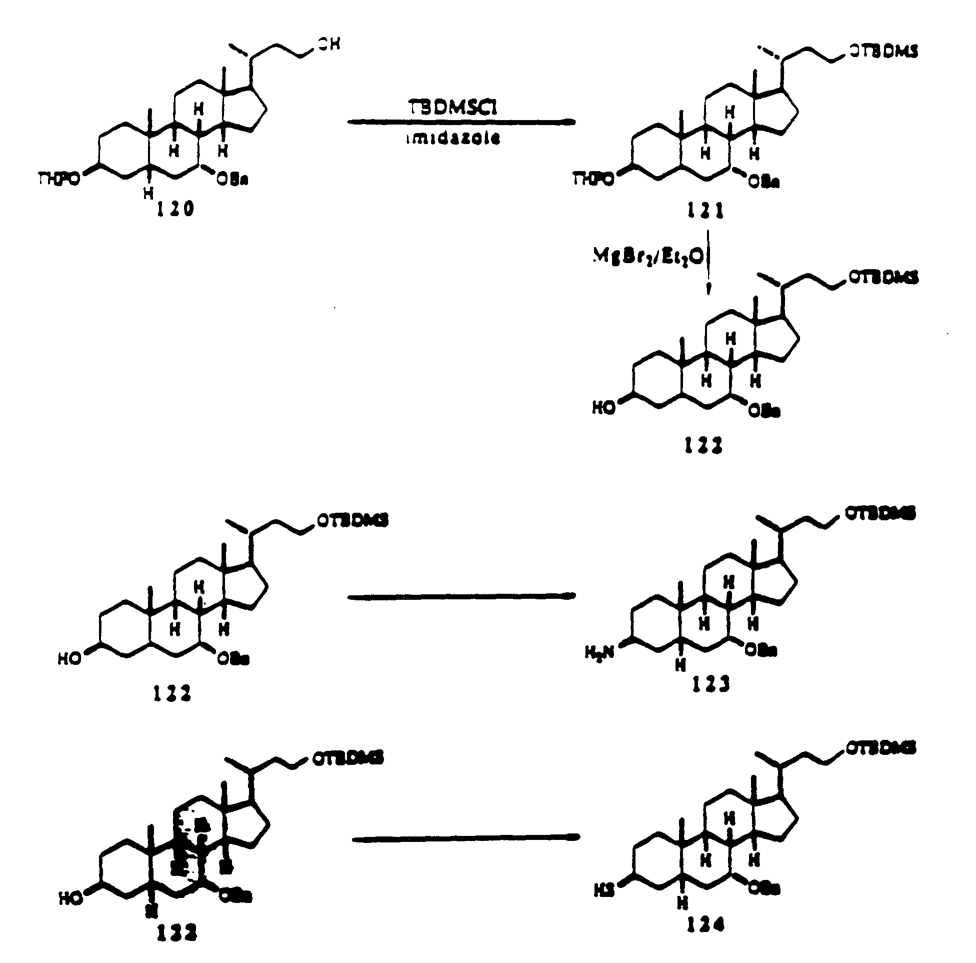

23,24-Bisnorcholenic acid **112** is converted to compound **113** in a manner analogous to the conversion of compound **1** to compound **4** (Example A(1)) and conversion of compound **4** to compound **71** (Example A(5)). The 3β-alcohol of compound **113** is converted to the corresponding 3β-amine, compound **114,** in a manner analogous to the conversion of compound **11** to compound **14.** Compound **113** is converted to compound **115** in a manner analogous to the conversion of compound **11** to compound **20.**

23,24-Bisnorcholenic acid **112** is converted to compound **116** in a manner analogous to the conversion of compound **1** to compound **4** (Example A(1)) and compound **4** to compound **70** (Example A(5)). Removal of the TBDMS protecting group from the 22-alcohol of compound **116** followed by oxidation of the resultant primary alcohol **117** with Collin's reagent gives aldehyde **118.** This aldehyde is then homologated by treatment with the ylide prepared from methoxymethyltriphenylphosphonium bromide followed by hydrolysis of the resultant enol ether to give compound **119** (L.L. Frye, C.H. Robinson, *J. Org. Chem.* 55, 1990, 1579). Aldehyde **119** is then reduced with lithium aluminum hydride, the resultant 23-alcohol **120** is protected as the TBDMS ether **121,** and the THP protecting group at C-3 is selectively removed to give compound **122.**

Compound **122** is converted to 3β-amine **123** in a manner analogous to the conversion of compound **11** to compound **14.** 3β-Alcohol **122** is converted to the corresponding thiol, compound **124,** in a manner analogous to the conversion of compound **11** to compound **20.**

### Example A(10)

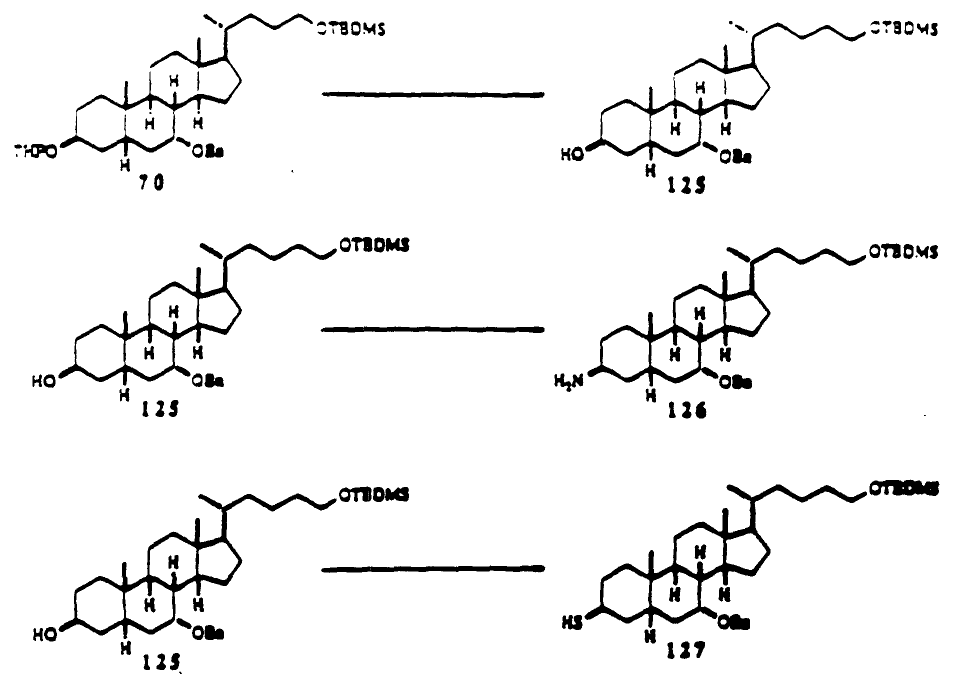

Compound **70** is converted to homologated compound **125** in a manner analogous to the conversion of compound **116** to compound **122.** Compound **125** is converted to the corresponding 3β-amine, compound **126,** in a manner analogous to the conversion of compound **11** to compound **14.** 3β-Alcohol **125** is converted to 3β-thiol **127** in a manner analogous to the conversion of compound **11** to compound **20.**

### Example A(11)

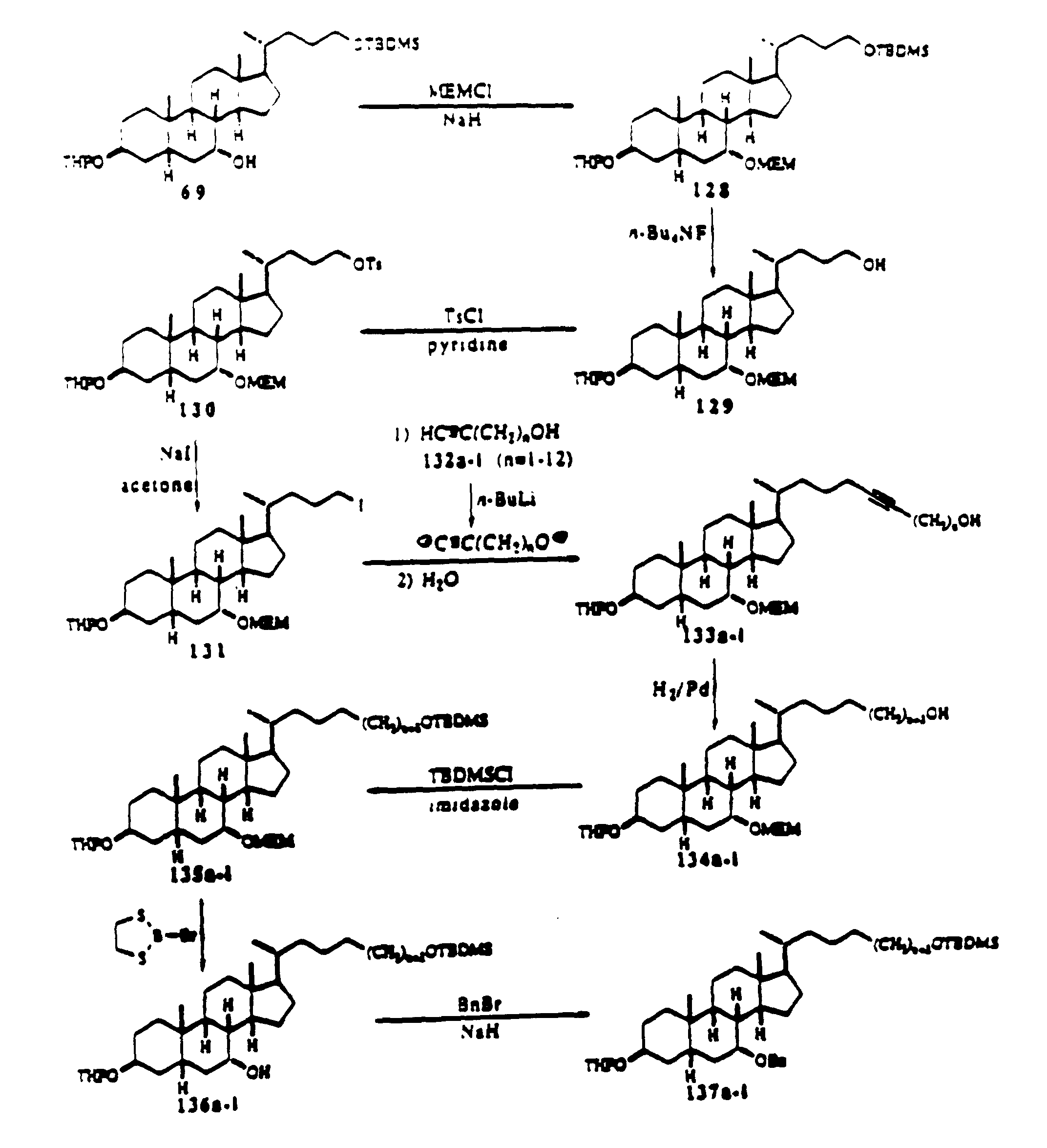

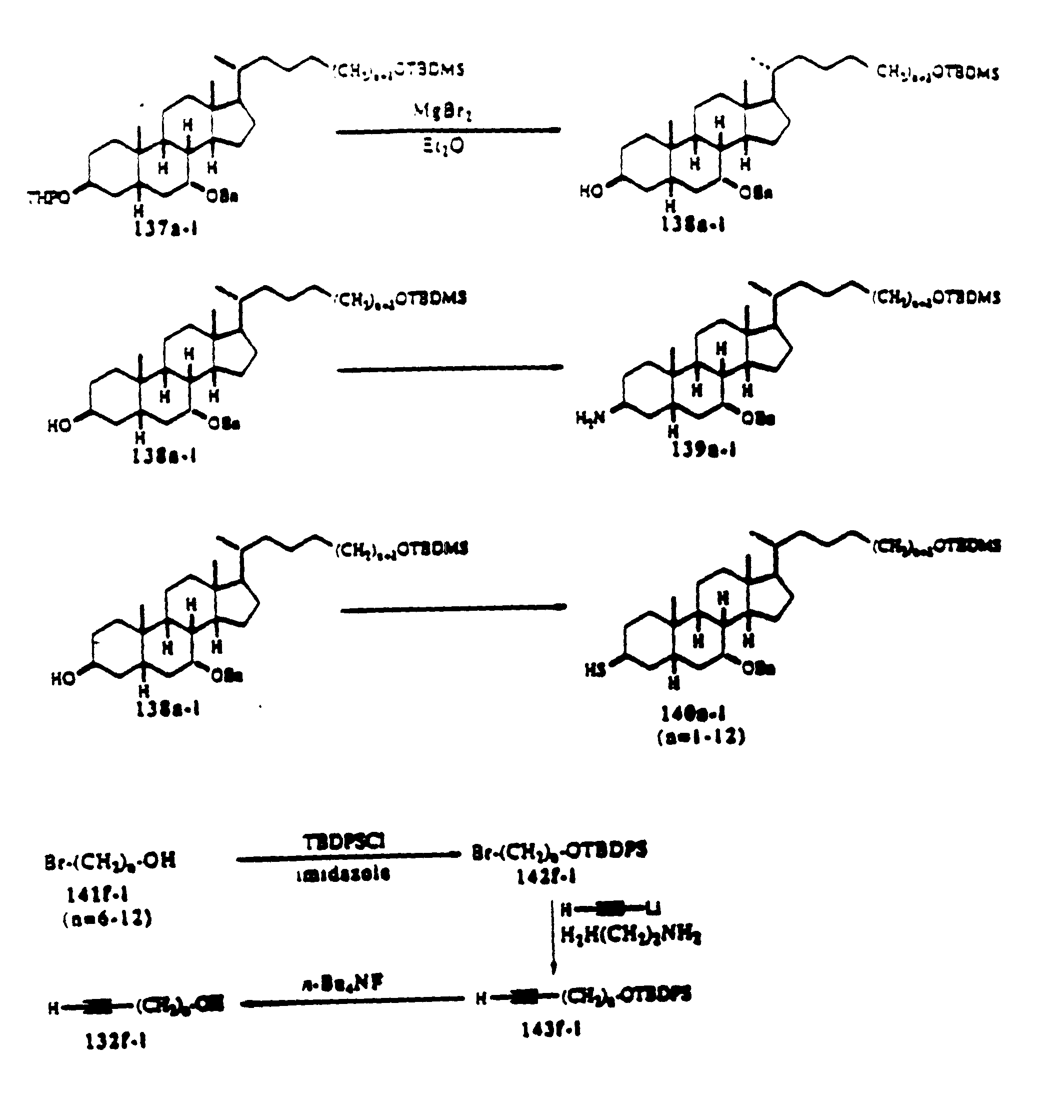

The 7α-alcohol of compound **69** is protected as the MEM ether (E.J. Corey, J.-L. Gras, P. Ulrich, *Tetrahedron Lett.,* 1976, 809) to give compound **128.** The TBDMS protecting group of the 24-alcohol is removed by treatment with tetra-n-butylammonium fluoride (E.J. Corey, A. Venkateswarlu, *J. Am. Chem. Soc.* 94, 1972, 6190) to yield alcohol **129.** Treatment with tosyl chloride (giving compound **130**) followed by sodium iodide in acetone gives compound **131.** Displacement of the primary iodide of compound **131** with the dianion of a variety of acetylenic alcohols (compounds **132a-1**) (S. Hahn, I.L. Stoilov, T.B. Tam Ha, D. Raederstorff, G. A. Doss, H.-T. Li, C. Djerassi, *J. Am. Chem. Soc.* 110, 1988, 8117) affords compounds **133a-1.** Catalytic hydrogenation of the acetylene moieties (giving compounds **134a-1**) followed by protection of the terminal alcohols as the TBDMS ethers yields compounds **135a-1.** Selective removal of the MEM protecting groups (D.R. Williams, S. Sakdarat, *Tetrahedron Lett.* 24, 1983, 3965) followed by protection of the resultant 7α-alcohols **136a-1** with benzyl bromide and sodium hydride affords compounds **137a-1.** The THP protecting groups of the 3α-alcohols are then removed by treatment with magnesium bromide in diethyl ether (S. Kim, J.H. Park, *Tetrahedron Lett.* 28, 1987, 439) to give compounds **138a-1.** Compounds **138a-1** are converted to the corresponding 3β-amines **139a-1** in a manner analogous to the conversion of compound **11** to compound **14.** Compounds **138a-1** are converted to the corresponding 3α-thiols **140a-1** in a manner analogous to the conversion of compound **11** to compound **20.**

Acetylenic alcohols **132a-e** (n = 1-5) are commercially available. Acetylenic alcohols **132f-l** are prepared from the corresponding bromo alcohols **141f-l.** The terminal alcohols of compounds **141f-l** are protected as the t-butyldiphenylsilyl (TBDPS) ethers (S. Hanessian, P. Lavallee, *Can. J. Chem.* 53, 1975, 2975) by treatment with TBDPS chloride and imidazole to give compounds **142f-l.** Displacement of the bromide of these compounds with lithium acetylide/ethylene diamine yields compounds **143f-l,** which are deprotected with tetra-n-butylammonium fluoride to give the acetylenic alcohols **132f-l.**

### Example A(12)

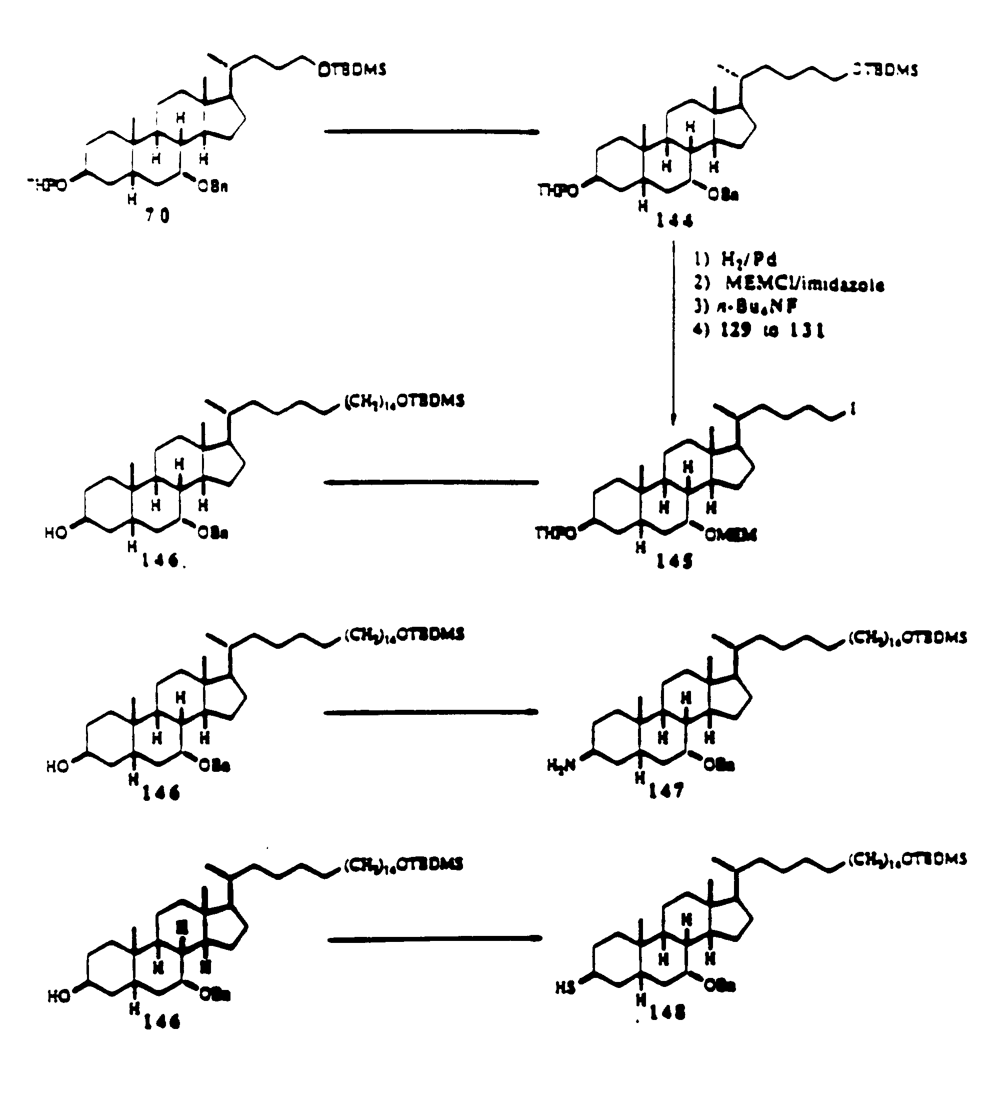

Compound **70** is converted to compound **144** in a manner analogous to the conversion of compound **116** to compound **121.** Compound **144** is converted to iodide **145** by conversion of the benzyl protecting group to an MEM protecting group (hydrogenation followed by treatment with MEMCl and imidazole), removal of the TBDMS protecting group of the 25-alcohol, and conversion of this alcohol to the corresponding iodide, compound **145,** in a manner analogous to the conversion of compound **129** to compound **131.** Compound **145** is converted to compound **146** in a manner analogous to the conversion of compound **131** to compounds **138a-l** utilizing acetylenic alcohol **132l** (n = 12).

Compound **146** is converted to compound **147** in a manner analogous to the conversion of compound **11** to compound **14.** Compound **146** is converted to compound **148** in a manner analogous to the conversion of compound **11** to compound **20.**

### Example A(13)

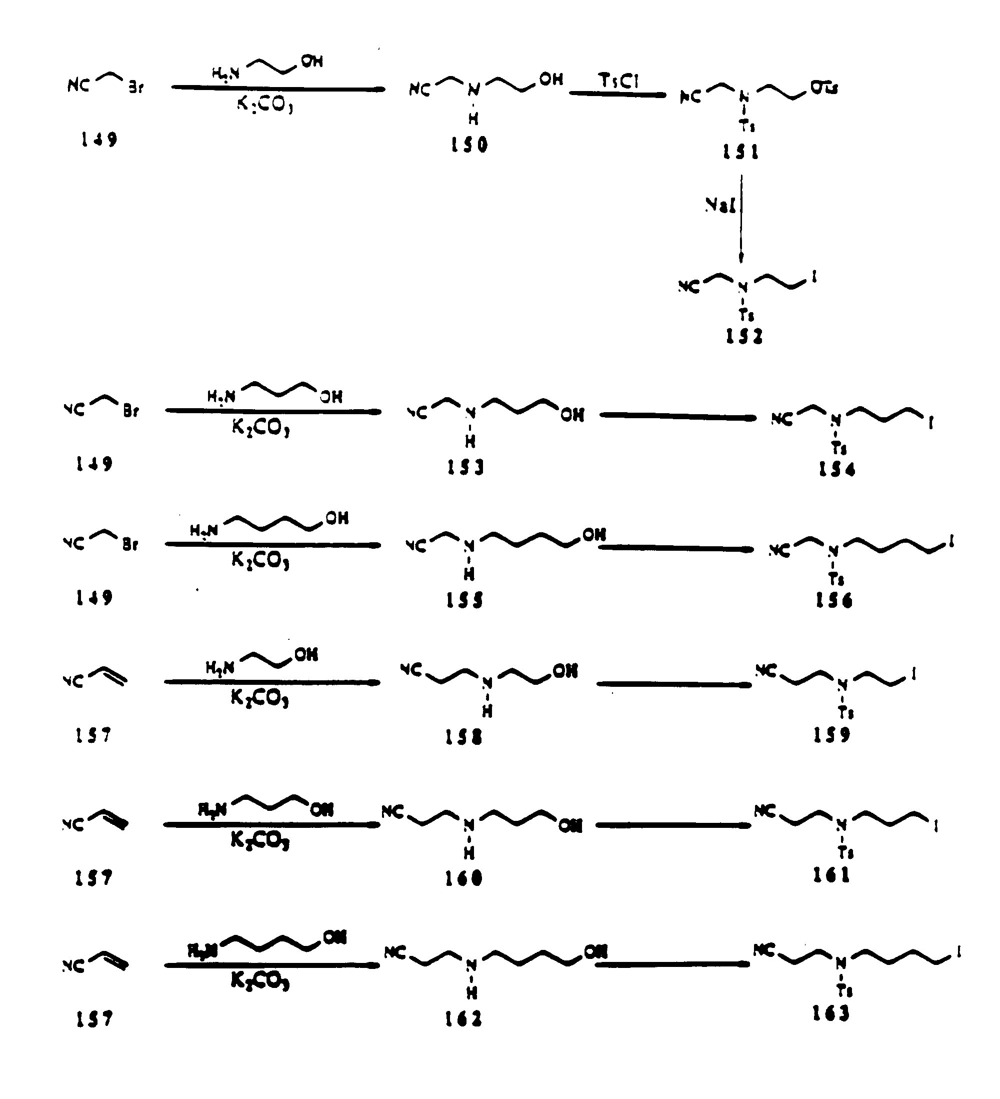

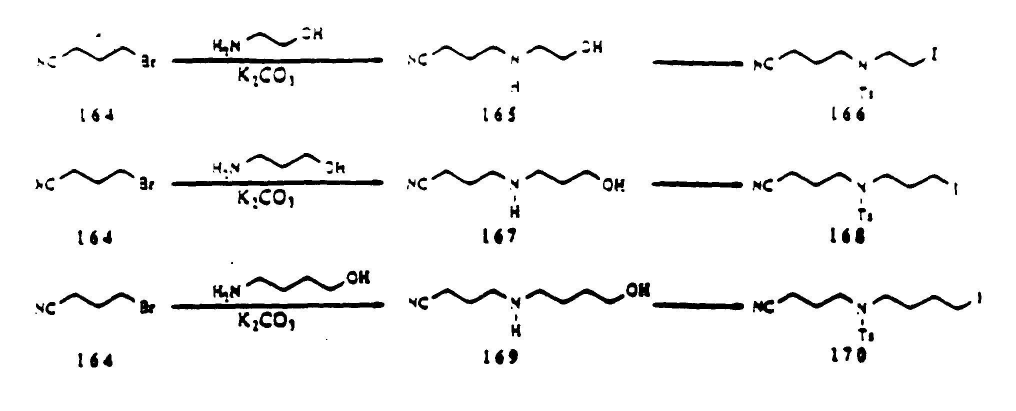

Bromoacetonitrile **149** is treated with 2-aminoethanol in the presence of potassium carbonate to give compound **150.** Treatment with tosyl chloride results in tosylation of both the alcohol and the amine, giving compound **151.** Treatment of compound **151** with sodium iodide gives compound **152.** Compound **154** is prepared in a manner analogous to the conversion of bromoacetonitrile **149** to compound **152** by utilizing 3-aminopropan-1-ol instead of 2-aminoethanol. Compound **156** is prepared in a manner analogous to the conversion of bromoacetonitrile **149** to compound **152** by utilizing 4-aminobutan-1-ol instead of 2-aminoethanol.

Treatment of acrylonitrile **157** with 2-aminoethanol in the presence of potassium carbonate gives conjugate addition product **158** (M. Israel, J.S. Rosenfield, E.J. Modest, *J. Med. Chem.* 7, 1964, 710), which is converted to the corresponding iodide **159** in a manner analogous to the conversion of compound **150** to compound **152.** Similarly, compound **161** is prepared from compound **157** in a manner analogous to the conversion of compound **157** to compound **159** utilizing 3-aminopropan-1-ol instead of 2-aminoethanol. Compound **163** is prepared from compound **157** in a manner analogous to the conversion of compound **157** to compound **159** utilizing 4-aminobutan-1-ol instead of 2-aminoethanol.

Compound **166** is prepared from compound **164** in a manner analogous to the conversion of bromoacetonitrile **149** to compound **152** using compound **164** instead of bromoacetonitrile **149.** Compound **168** is prepared from compound **164** in a manner analogous to the conversion of bromoacetonitrile **149** to compound **154** using compound **164** instead of bromoacetonitrile **149.** Compound **170** is prepared from compound **164** in a manner analogous to the conversion of bromoacetonitrile **149** to compound **156** using compound **164** instead of bromoacetonitrile **149.**

### Example A(14)

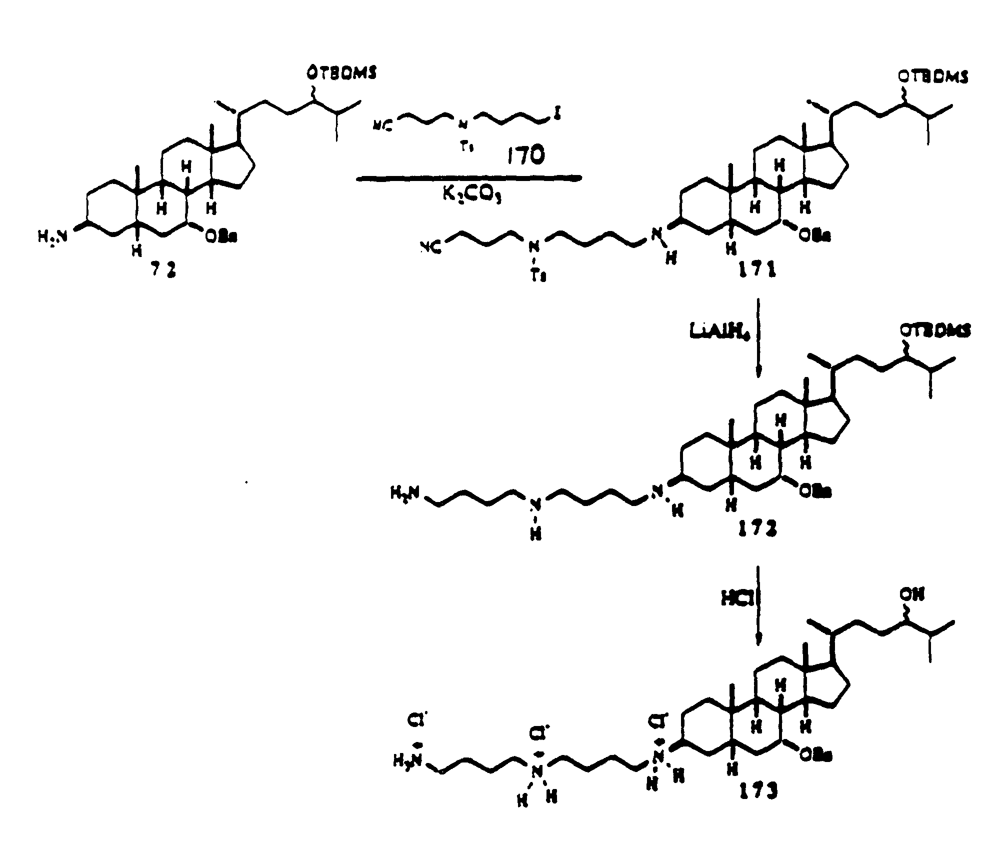

The cationic side chains (compounds **152, 154, 156, 159, 161, 163, 166, 168,** and **170**) are attached to the suitably protected flat ring systems (compounds **11, 14, 20, 21, 22, 23, 33, 34, 35, 36, 37, 38, 51, 52, 53, 64, 65, 66, 71, 72, 73, 74, 75, 76, 78, 79, 80, 81, 82, 83, 95, 98, 110, 111, 113, 114, 115, 122, 123, 124, 125, 126, 127, 138a-l, 139a-l, 140a-1, 146, 147,** and **148**) as illustrated for the conversion of compound **72** to compound **173.** Thus, compound **72** is treated with the iodide **170** in the presence of potassium carbonate to give compound **171.** Reduction of the nitrile to the corresponding amine and removal of the tosyl protecting group from the amine (T.W. Greene, P.G.M. Wuts, "Protective Groups in Organic Synthesis," 2nd ed., John Wiley and Sons, New York, 1991, 380) by treatment with lithium aluminum hydride yields compound **172.** Treatment with HCl results in the formation of the trisammonium salt and removal of the TBDMS protecting group, giving compound **173.**

### Example A(15)

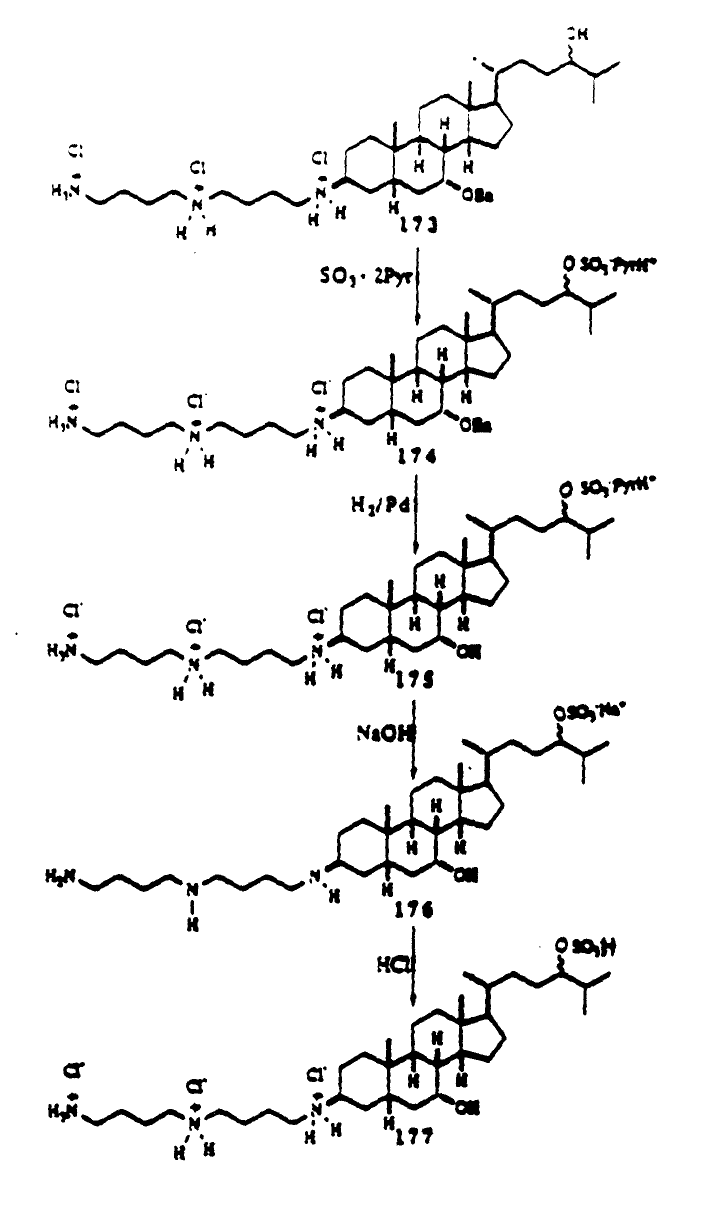

The introduction of a sulfate into the anionic side chain of a flat ring system with a cationic side chain attached is illustrated by the conversion of compound **173** to compound **177.** Compound **173** is treated with sulfur trioxide dipyridine (S. Bernstein, J.P. Dusza, J.P. Joseph, "Chemical and Biological Aspects of Steroid Bioconjugation," S. Bernstein and S. Solomon (eds.), Springer-Verlag, New York, 1970, 25-36) to yield pyridinium sulfate **174.** The benzyl protecting group is typically removed by hydrogenation with palladium as illustrated in the conversion of compound **174** to compound **175.** It should be noted that in the cases where the flat ring system contains a double bond or the link between the flat ring system and the cationic side chain is a sulfur atom, other methods are used to remove the benzyl protecting group such as treatment with Ph₃C⁺BF₄⁻ (T.R. Hoye, A.J. Caruso, J.F. Dellaria, Jr., M. J. Kurth, *J. Am. Chem. Soc.* 104, 1982, 6704). Treatment of compound **175** with sodium hydroxide (giving compound **176**) followed by HCl results in the formation of compound **177.**

### Example A(16)

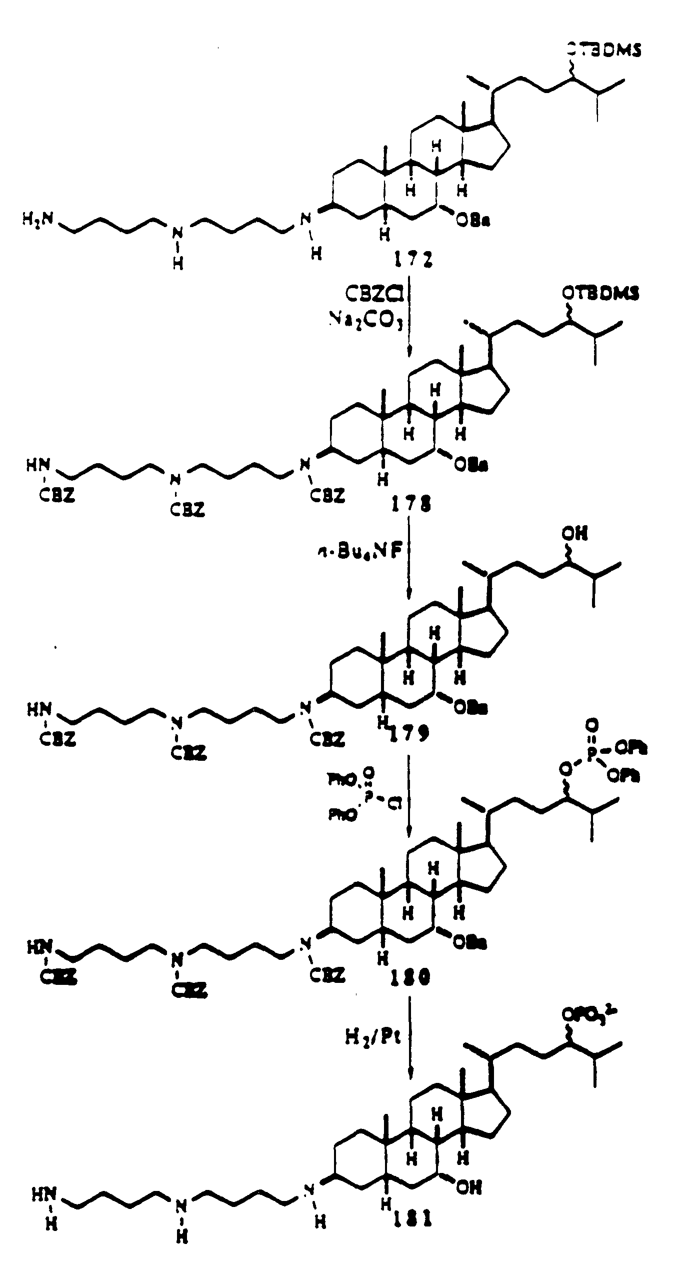

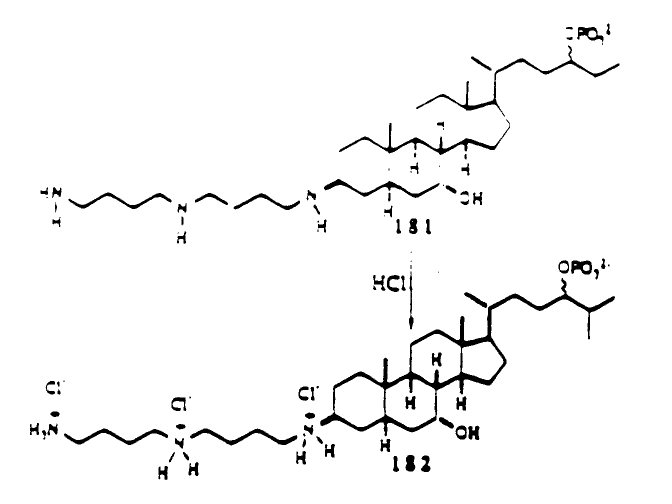

The introduction of a phosphate into the anionic side chain of a flat ring system with a cationic side chain attached is illustrated by the conversion of compound **172** to **181.** The amines of compound **172** are protected as the benzyl carbamates to give compound **178** by treatment with PhCH₂OCOCl (CBZCl) in the presence of sodium carbonate. The TBDMS protecting group is then removed by treatment with tetra-n-butylammonium fluoride to give alcohol **179.** Treatment of compound **179** with diphenyphosphoryl chloride gives compound **180** (H.G. Khorana, "Some Recent Developments in the Chemistry of Phosphate Esters of Biological Interest," John Wiley and Sons, New York, 1961, 16). Reduction of compound **180** with hydrogen/platinum followed by treatment with HCl results in the formation of compound **182.**

### Example A(17)

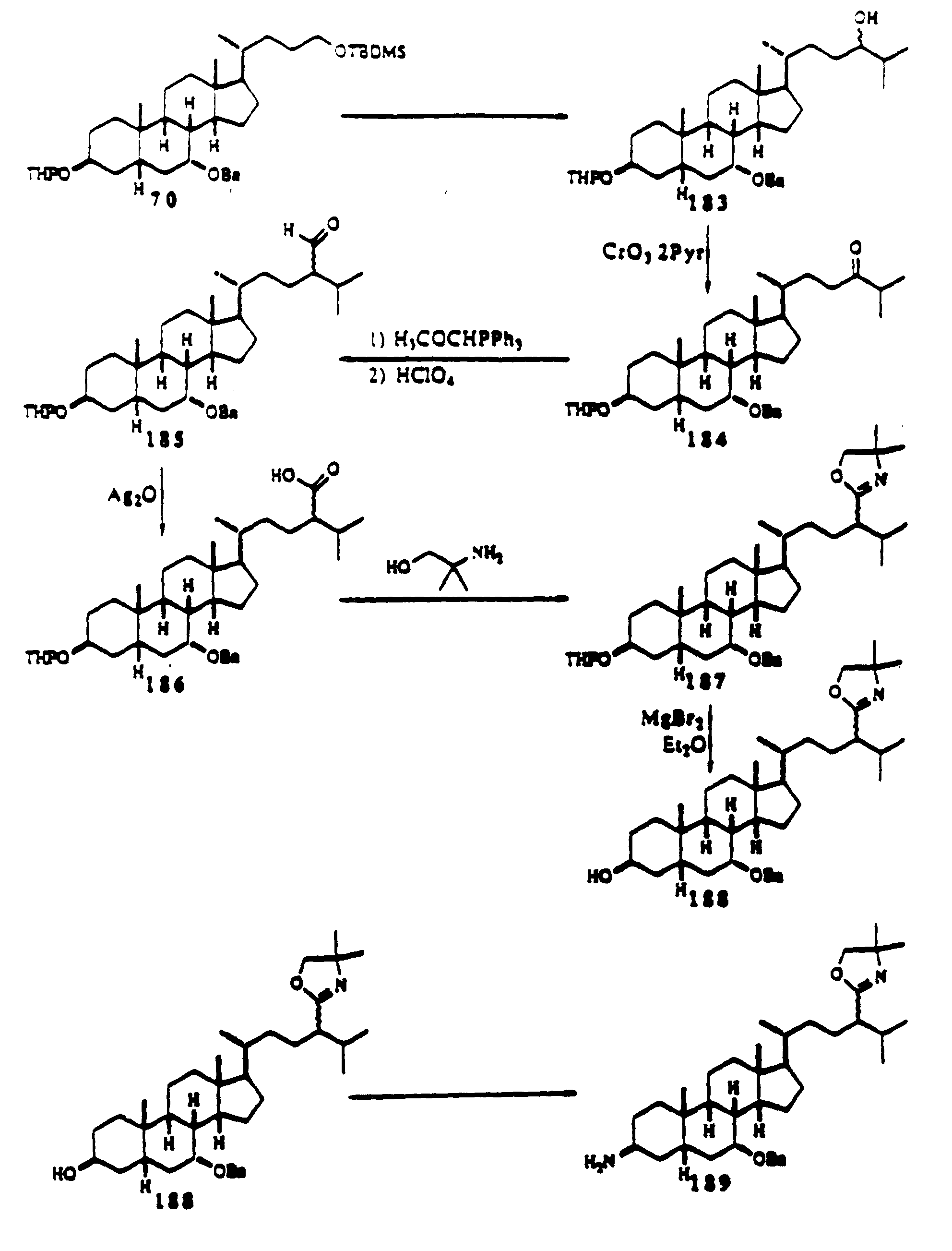

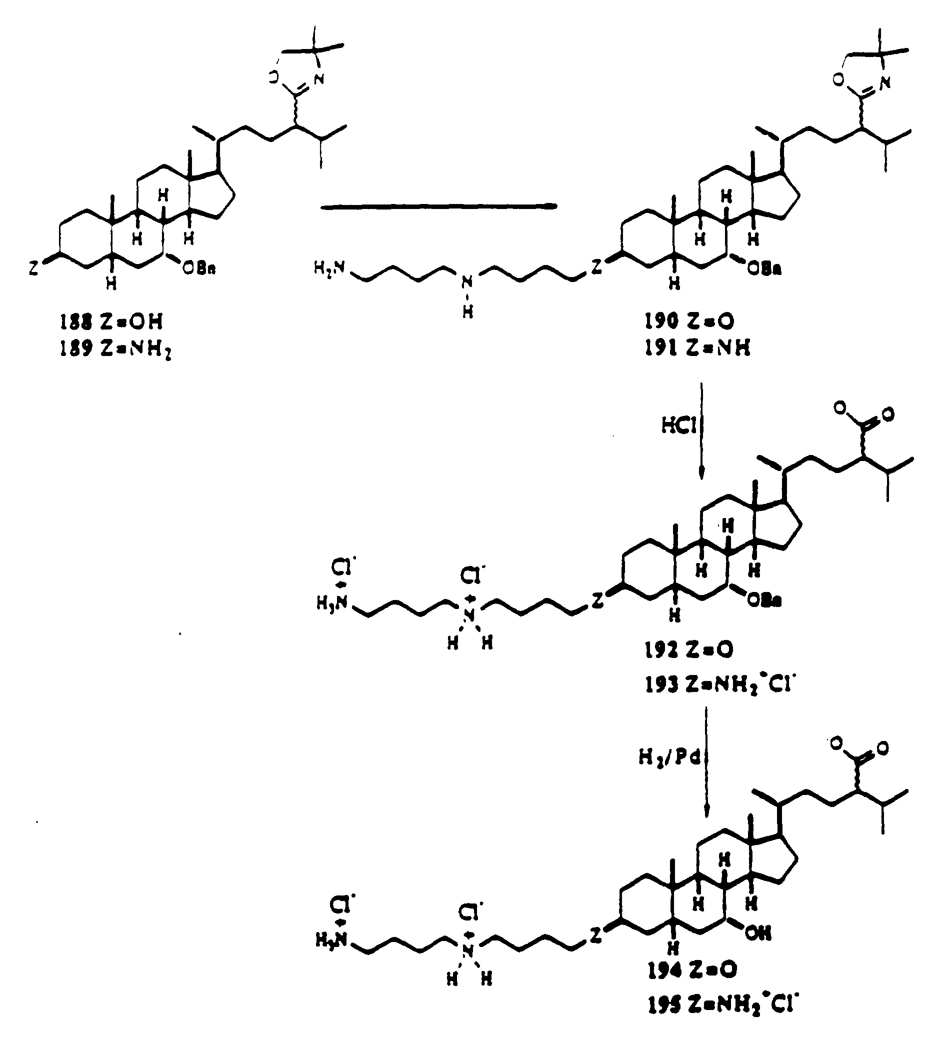

Preparation of compounds with a carboxylate in the side chain requires that a protected carboxylic acid be introduced into the side chain prior to attachment of the cationic side chain. This general procedure is illustrated in the conversion of compound **70** to compounds **194** and **195.** Compound **70** is converted to compound **183** in a manner analogous to the conversion of compound **6** to compound **9.** The 24-alcohol is oxidized to ketone **184** with Collin's reagent. Compound **184** is treated with the ylide from methoxymethyltriphenylphosphonium bromide followed by HClO₄ to give compound **185** (L.L. Frye, C.H. Robinson, *J. Org. Chem.* 55, 1990, 1579). Oxidation of aldehyde **185** to carboxylic acid **186** is accomplished by treatment with silver oxide (E.J. Corey, N.W. Gilman, B.E. Ganem, *J. Am. Chem. Soc.* 90, 1968, 5616). The resultant carboxylic acid is protected as the 1,3-oxazoline by treatment with 2-amino-2-methylpropan-1-ol to give compound **187.** Removal of the THP protecting group with magnesium bromide (S. Kim, J. H. Park, *Tetrahedron Lett.* 28, 1987, 439) yields compound **188.** Compound **188** is converted to the corresponding 3β-amine, compound **189,** in a manner analogous to the conversion of compound **11** to compound **14.** The cationic side chain is introduced in a manner analogous to the conversion of compound **72** to compound **172** to give compound **190** from compound **188** and compound **191** from compound **189.** Treatment of compound **190** with HCl results in the removal of the oxazoline protecting group and protonation of the amines to give compound **192.** Compound **191** is converted to compound **193** in a manner analogous to the conversion of compound **190** to compound **192.** Treatment of compound **192** with hydrogen in the presence of palladium gives compound **194.** Compound **193** is converted to compound **195** in a manner analogous to the conversion of compound **192** to compound **194.**

### Example B

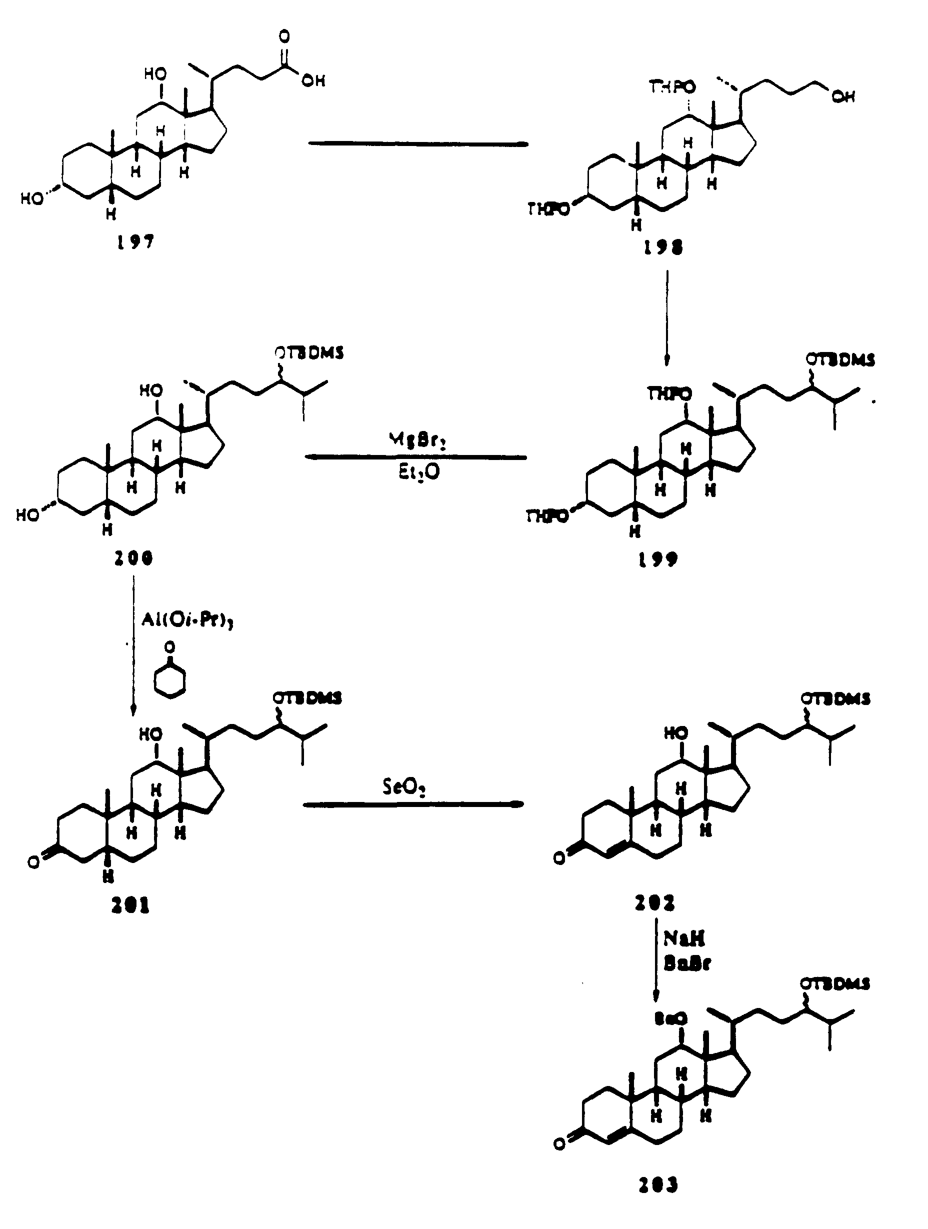

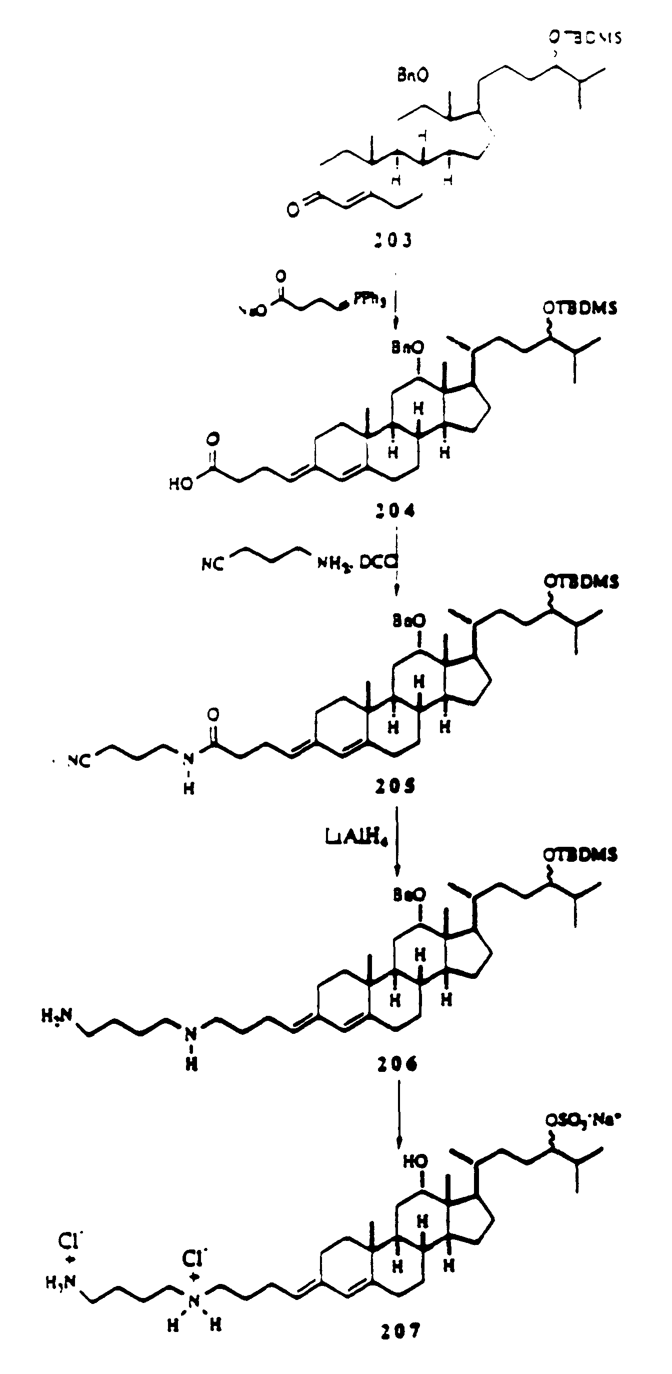

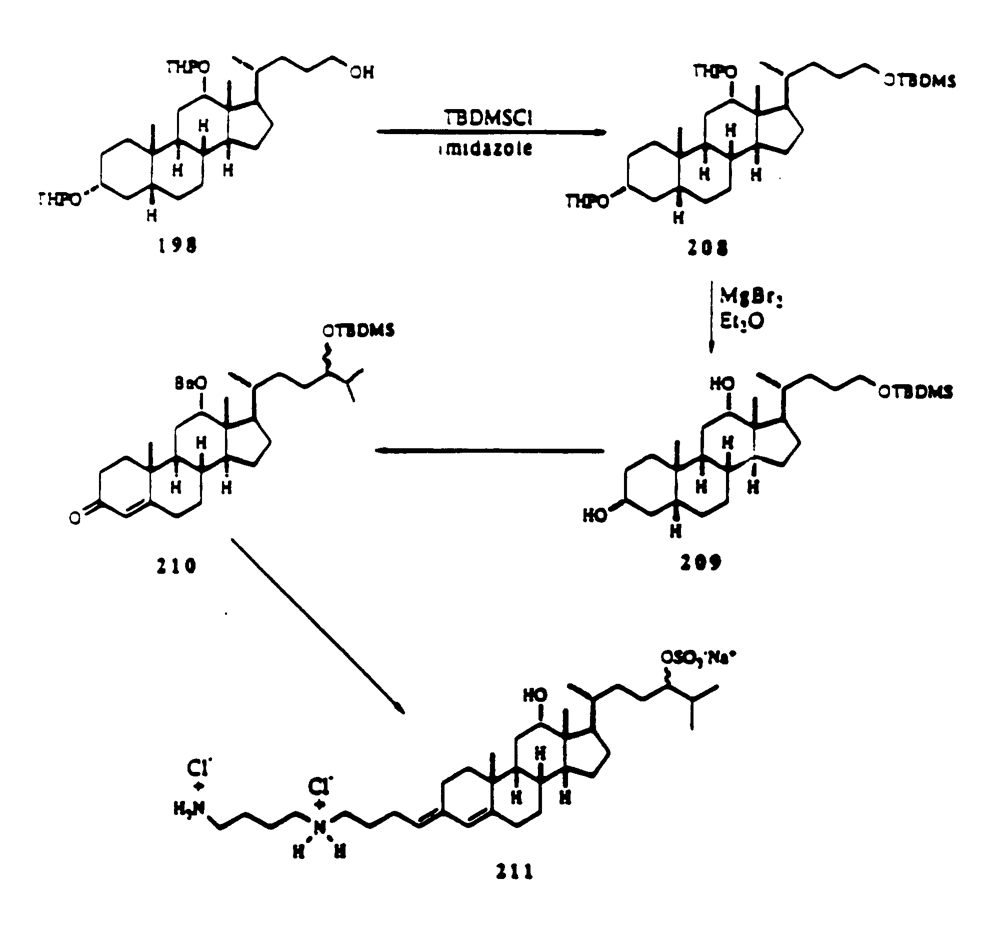

The preparation of compounds with a double bond in the Δ⁴-position of the flat ring system is illustrated by the preparation of compounds **207** and **211.** Deoxycholic acid **197** is converted to compound **198** in a manner analogous to the conversion of cholenic acid **1** to compound **3**. Compound **198** is then converted to compound **199** in a manner analogous to the conversion of compound **7** to compound **10.** The THP protecting groups are then removed by treatment with magnesium bromide in ether to give compound **200** (S. Kim, J.H. Park, *Tetrahedron Lett.* 28, 1987, 439). The equatorial 3-hydroxyl is selectively oxidized with aluminum isopropoxide and cyclohexanone to give compound **201** (M. Ehrenstein, T.O. Stevens, *J. Org. Chem.* 5, 1940, 660). The double bond at C-4 is introduced by treatment of compound **201** with selenium dioxide (I. Bjorkhem, H. Danielsson, C. Issidorides, A. Kallner, *Acta Chem. Scand.* 19, 1965, 2151; S.J. Branca, A.B. Smith, III, *J. Am. Chem. Soc.* 100, 1978, 7767), giving compound **202.** The C-12 alcohol is then protected as the benzyl ether by treatment with benzyl bromide and sodium hydride to give compound **203.** Compound **203** is condensed with the Wittig reagent derived from 5-triphenylphosphoniopentanoic acid and sodio-methylsulfinylcarbamide in dimethyl sulfoxide to give compound **204** (E.J. Corey, N.M. Weinshenker, T.K. Schaff, W. Huber, *J. Am. Chem. Soc.* 91, 1969, 5675). Compound **205** is then prepared by DCC mediated coupling of compound **204** with 4-aminobutanenitrile (F. Mares, J.E. Galle, S.E. Diamond, F.J. Regina, *J. Catal.* 112, 1988, 145). Reduction of the nitrile and the amide is accomplished by treatment with lithium aluminum hydride, giving compound **206.** Compound **206** is converted to compound **207** in a manner analogous to the conversion of compound **172** to compound **177.** The benzyl group is removed by treatment with Ph₃C+BF₄- as in Example A(15).

The 24-hydroxyl of compound **198** is protected as the TBDMS ether by treatment with TBDMS chloride and imidazole to give compound **208** (E.J. Corey, A. Venkateswarlu, *J. Am. Chem. Soc.* 94, 1972, 6190). Selective removal of the THP protecting groups from the C-3 and C-12 alcohols by treatment with magnesium bromide in ether gives compound **209** (S. Kim, J.H. Park, *Tetrahedron Lett.* 28, 1987, 439). Compound **209** is converted to compound **210** is a manner analogous to the conversion of compound **200** to compound **203.** Compound **210** is converted to compound **211** in a manner analogous to the conversion of compound **203** to compound **207.**

### Example D

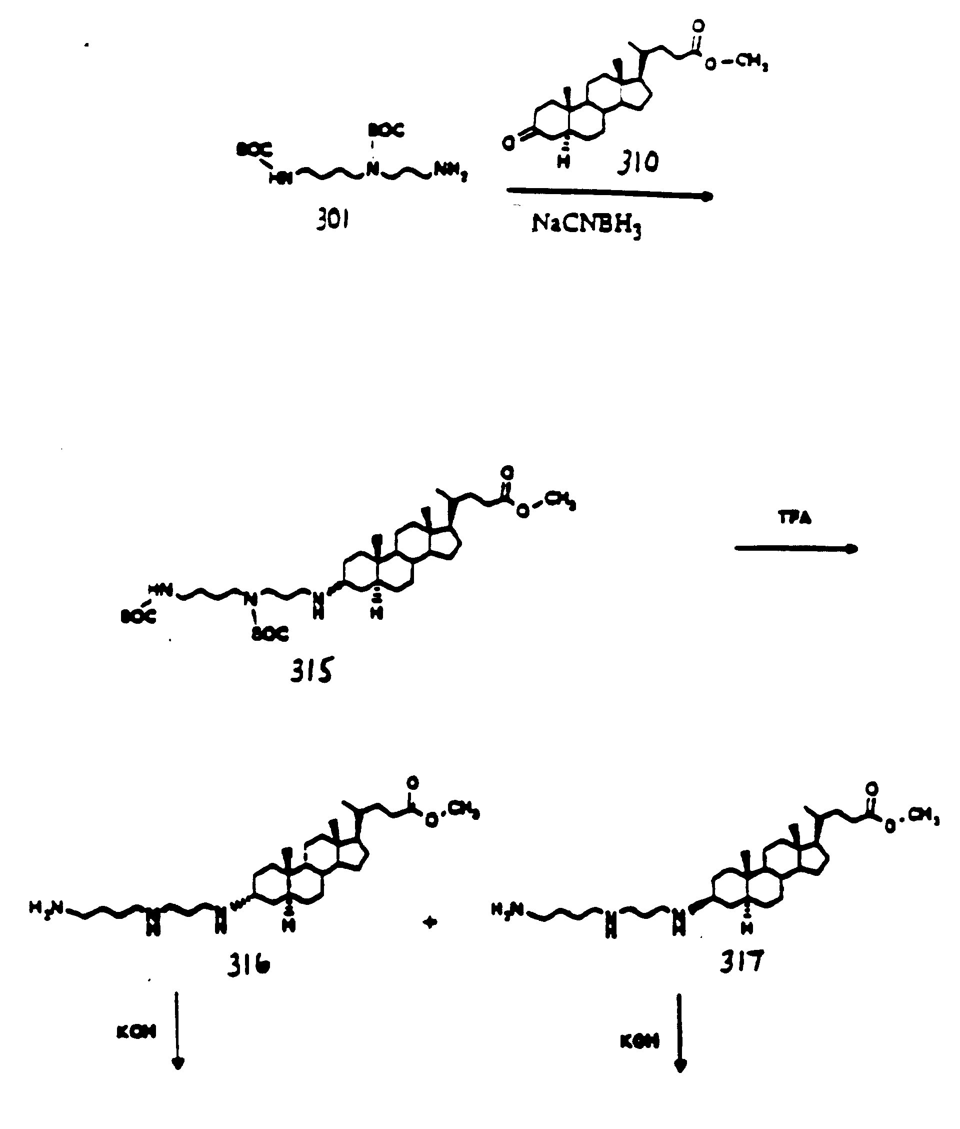

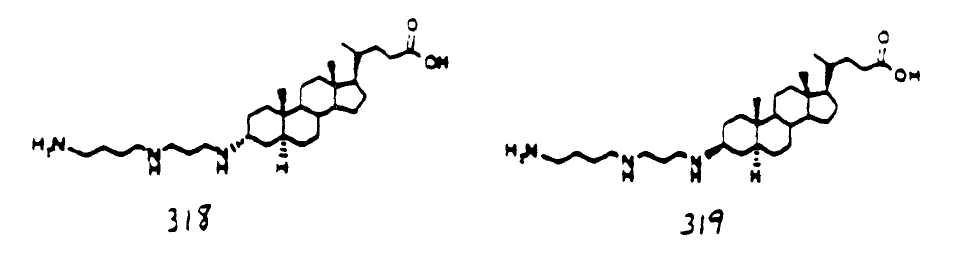

Preparation of compound **315:** To a solution of 5α-cholanic acid-3-one methyl ester **310** (719 mg, 1.85 mmol) in anhydrous tetrahydrofuran (10 ml) was added 3Å sieves (4 g), a solution of triamine **301** (650 mg, 1.88 mmol) in dry methanol (25 ml), and sodium cyanoborohydride (600 mg, 9.55 mmol). After stirring for eighteen hours at room temperature, the reaction mixture was filtered through Celite and washed with methanol (20 ml), dichloromethane (20 ml), 10% sodium hydroxide (15 ml), and brine (25 ml). The layers were separated and the aqueous layer was extracted with more dichloromethane (3 x 10 ml), and the combined organic layers were washed with brine, dried (Na₂SO₄), and evaporated. The crude material was purified by flash chromatography (2 cm, gradient elution with 2-4% 2N methanolic ammonia (Aldrich) in dichloromethane), affording compound **315** (1.09 g, 82% yield) as a mixture of C-3 isomers. ¹H NMR (200 MHz, CDCl₃) δ: 4.57 (br s, NH), 3.65 (s, 3H), 3.4-3.0 (m, 6H), 2.8-2.5 (m, 3H), 2.4-1.0 (m, 34H), 1.45 (s, 9H), 1.44 (s, 9H), 0.91 (d, J = 6 Hz, 3H), 0.78 (s, 3H), 0.64 (s, 3H); MS(+FAB): 719 (M+H, 100).

Preparation of compounds **316** and **317:** A solution of compound **315** (910 mg, 1.27 mmol) in chloroform (39 ml) was treated with trifluoroacetic acid (33 ml) at 0°C. After one hour at room temperature, the reaction mixture was evaporated, dissolved in chloroform, and evaporated again (three times). The crude material was dissolved in methanol, treated with isopropylamine, and preadsorbed onto silica gel. Flash chromatography (2 cm, gradient elution with 1:4:15 to 1:4:6 isopropylamine:methanol:chloroform) yielded the 3α-amino isomer **316** as a crude product and 3β-amino isomer **317** as a pure product (319 mg, 48% yield). ¹H NMR (200 MHz, CDCl₃) δ: 3.66 (s, 3H), 2.8-2.6 (m, 8H), 2.47 (br m, 3α-H), 2.4-1.0 (m, 34H), 0.90 (d, J = 6 Hz, 3H), 0.78 (s, 3H), 0.64 (s, 3H); MS(+FAB): 518 (M+H, 100).

Preparation of compound **318:** Crude compound **316,** obtained as described above, was dissolved in methanol (20 ml) and treated with 0.5N potassium hydroxide solution (15 ml) in methanol and water (5 ml). After refluxing for thirty minutes and leaving at room temperature overnight, the reaction mixture was purified in the manner described below for the isolation of compound **319,** affording 3α-amino isomer **318** (50 mg, 8% yield, two steps). ¹H NMR (200 MHz, CD₃OD) δ: 3.13 (m, 3β-H), 3.0-2.6 (m, 8H), 2.3-1.0 (m, 34H), 0.96 (d, J = 6 Hz, 3H), 0.84 (s, 3H), 0.70 (s, 3H); IR (KBr, cm ¹): 2930, 2850, 1560, 1444, 1396, 1120, 752; MS(+FAB): 504 (M+H, 100).

Preparation of compound **319:** A solution of compound **317** (240 mg, 0.46 mmol) in methanol (15 ml) was treated with 0.5N potassium hydroxide in methanol (10 ml) and water (3.3 ml) under nitrogen at reflux for 3.5 hours. After cooling to room temperature, the reaction mixture was acidified with 1N HCl to a pH of 4-5, extracted with chloroform (3 x 20 ml), and dried over MgSO₄. The solvent was evaporated and the product was purified by flash chromatography (1 cm, elution with 1:3:10 ammonium hydroxide:methanol:chloroform), affording 3β-amino isomer **319** as a beige solid (130 mg, 56% yield). ¹H NMR (200 MHz, CD₃OD) δ: 2.9-2.6 (m, 9H), 2.2-1.0 (m, 34H), 0.95 (d, J = 6 Hz, 3H), 0.84 (s, 3H), 0.70 (s, 3H); IR (KBr, cm⁻¹) : 3268, 2928, 2850, 1560, 1444, 1396, 1118, 750; MS(+FAB) : 504 (M+H, 100).

### Example E

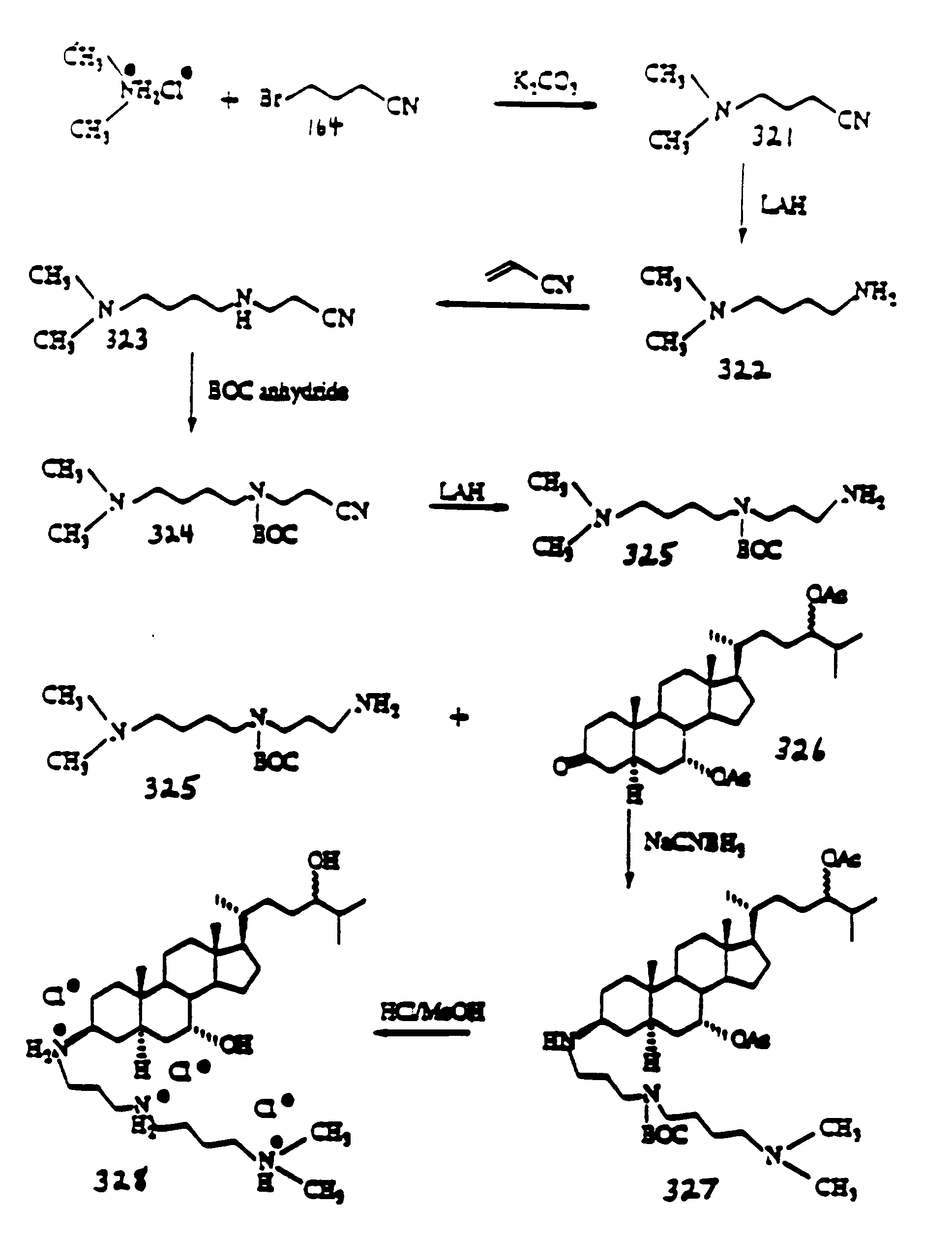

### Preparation of side chain 325:

A solution of 3-cyanopropylbromide (4-bromobutyronitrile) **164** (6.38 g, 43.10 mmol) in dry acetonitrile (50 ml) was added dropwise to a gentle refluxing suspension of dimethylamine hydrochloride (5.27 g, 64.62 mmol) and anhydrous potassium carbonate (20.85 g, 150.86 mmol) in dry acetonitrile (100 ml). After the addition was complete, the reaction mixture was refluxed further for six hours. Acetonitrile was removed *in vacuo,* and the residue was extracted with ether (100 ml). Evaporation of ether *in vacuo* afforded N-(3-cyanopropyl)-N,N-dimethylamine **321** as a colorless oil (4.20 g, 87% yield based on 3-cyano-propylbromide). ¹H NMR (400 MHz, CDCl₃) δ: 1.79 (2H, m, -CH₂-CH₂-CH₂-), 2.20 (6H, s, -N(CH₃)₂), 2.37 (2H, t, -NCH₂CH₂-), 2.41 (2H, t, -CH₂CH₂CN).

To a suspension of lithium aluminum hydride (LAH) (4.74 g, 120.90 mmol) in dry ether (100 ml) was added dropwise a solution of N-(3-cyanopropyl)-N,N-dimethylamine **321** (4.10 g, 36.61 mmol) in dry ether (50 ml) at 0°C. After complete addition, the reaction mixture was stirred for two hours while allowing the temperature to raise from 0°C to room temperature. The reaction mixture was quenched with 2N NaOH at 0°C, and the resulting white suspension was filtered through Celite and washed with ether. The ether filtrate was dried over K₂CO₃, filtered and concentrated *in vacuo,* yielding N,N-dimethyl-1,4-diaminobutane **322** as a colorless oil (2.5 g, 60% yield). ¹H NMR (400 MHz, CDCl₃) δ: 1.43 (4H, m, -CH₂-CH₂-CH₂-CH₂-), 1.93 (2H, br s, -NH₂), 2.16 (6H, s, -N(CH₃)₂), 2.21 (2H, t, -CH₂CH₂N), 2.66 (2H, t, -CH₂CH₂NH₂).

A solution of acrylonitrile (1.17 g, 22.07 mmol) in methanol (1.0 ml) was added dropwise to a solution of N,N-dimethyl-1,4-diaminobutane **322** (2.1 g, 18.42 mmol) in methanol (1.0 ml) at 0°C, and the mixture was stirred at 0°C for sixteen hours. Evaporation of the solvent *in vacuo* afforded almost pure N-(2-cyanoethyl)-N',N'-dimethyl-1,4-diaminobutane **323** as a colorless oil (2.5 g, 80% yield based on **322**). ¹H NMR (400 MHz, CDCl₃) δ: 1.45 (4H, m, -CH₂-CH₂-CH₂-CH₂-), 2.15 (6H, s, -N(CH₃)₂), 2.22 (2H, t, -CH₂N), 2.47 (2H, t, -CH₂CH₂CN), 2.60 (2H, t, -CH₂CH₂NH-), 2.88 (2H, t, -CH₂CH₂NH-), 3.37 (1H, s, -NH).

To a stirred solution of N-(2-cyanoethyl)-N',N'-dimethyl-1,4-diaminobutane **323** (2.0 g, 11.83 mmol) in dry dichloromethane (50 ml) was added dropwise a solution of di-tert-butyldicarbonate (2.84 g, 13.01 mmol) in dry dichloromethane (50 ml) at room temperature, and the mixture was stirred for sixteen hours. The reaction mixture was concentrated *in vacuo,* and the residue was dissolved in ethyl acetate (100 ml), washed with saturated NaHCO₃, washed with brine, dried over K₂CO₃, filtered, and evaporated *in vacuo,* producing N-(tert-butoxycarbonyl)-N-(2-cyanoethyl)-N',N'-dimethyl-1,4-diaminobutane **324** as a viscous oil (2.24 g, 70% yield), which was used in the next step without further purification. ¹H NMR (400 MHz, CDCl₃) δ: 1.45 and 1.46 (9H+2H, merged s and m, -C(CH₃)₃ and -CH₂-CH₂-CH₂-), 1.52 (2H, m, -CH₂CH₂CH₂-), 2.19 (6H, s, -N(CH₃)₂), 2.25 (2H, t, -CH₂CH₂N), 2.59 (2H, m, -CH₂CN), 3.25 (2H, t, -CH₂CH₂NCO-), 3.25 (2H, t, -CH₂CH₂NCO-).

To a solution of LAH (0.62 mg, 16.30 mmol) in anhydrous ether (100 ml) was added N-(tert-butoxycarbonyl)-N-(2-cyanoethyl)-N',N'-dimethyl-1,4-diaminobutane **324** (2.22 g, 8.10 mmol) in anhydrous ether (50 ml) at 0°C. The mixture was stirred at 0°C for thirty minutes. The excess LAH was quenched with 1N NaOH at 0°C, and the resulting suspension was filtered through Celite and washed with ether. The combined ether layers were washed with brine, dried over MgSO₄, and concentrated *in vacuo* to yield a crude product. The crude product was purified on a flash silica gel column and eluted with chloroform:methanol:isopropylamine (15:1:1) to give N-(3-aminopropyl)-N-(tert-butoxycarbonyl)-N',N'-dimethyl-1,4-diaminobutane **325** (1.30 g, 59% yield). ¹H NMR (400 MHz, CDCl₃) δ: 1.40 (9H, s, t-Bu), 2.15 (6H, s, -N(CH₃)₂), 2.22 (2H, m) , 2.65 (2H, t), 3.20 (4H, m).

### Reductive amination to form compounds 327 and 328:

To a solution of 3-oxo-7α,24-diacetoxy-5α-cholestane **326** (490 mg, 1.00 mmol) and N-(3-aminopropyl)-N-(tert-butoxycarbonyl)-N',N'-dimethyl-1,4-diaminobutane **325** (410 mg, 1.5 mmol) in methanol (30 ml) was added 3Å molecular sieves (2.00 g) and NaCNBH₃ (94.2 mg, 1.50 mmol). The reaction mixture was stirred at room temperature for sixteen hours. After filtering through Celite, the methanol was removed *in vacuo.* The residue was purified on a flash silica gel column and eluted with chloroform:methanol:isopropylamine (15:1:1), producing 3β-N-{N-[3-(4-N',N'-dimethylaminobutyl)]-3-tert-butoxycarbonyl-1,3-diaminopropane}-7a,24-diacetoxy-5α-cholestane **327** (501 mg, 66% yield). ¹H NMR (400 MHz, CDCl₃) δ: 0.63 (3H, s, 18-CH₃), 0.84 (3H, s, 19-CH₃), 2.04 (3H, s, CH₃CO₂-), 2.07 (3H, s, CH₃CO₂-), 2.38 (6H, br s, -N(CH₃)₂), 2.49 (1H, m, 3α-H), 4.67 (1H, m 24-H), 4.89 (1H, m, 7β-H).

To a solution of 3β-N-{N-[3-(4-N',N'-dimethylaminobutyl)]-3-tert-butoxycarbonyl-1,3-diaminopropane}-7α,24-diacetoxy-5α-cholestane **327** (400 mg, 0.52 mmol) in methanol (20 ml) was added methanol saturated with HCl gas (5 ml). The reaction mixture was stirred at room temperature for twenty-four hours. After removing the methanol *in vacuo,* the crude product was purified on a flash silica gel column and eluted with dichloromethane:methanol:ammonium hydroxide (7.5:2:0.5), giving 3β-N-1-{N-[3-(4-N',N'-dimethylaminobutyl)]-1,3-diaminopropane}-7α,24-dihydroxy-5α-cholestane **328,** which was dissolved in methanol, treated with methanolic HCl and evaporated to yield **328**-3HCl (174 mg, 58% yield). ¹H NMR (400 MHz, CDCl₃) δ: 0.65 (3H, s, 18-CH₃), 0.76 (3H, s, 19-CH3), 2.18 (6H, s, -N(CH₃)₂), 2.45 (1H, m, 3α-H), 3.27 (1H, m 24-H), 3.79 (1H, m, 7β-H); MS(+FAB) : 577 (M⁺+1, 41.48%), 576 (100%).

### Example F

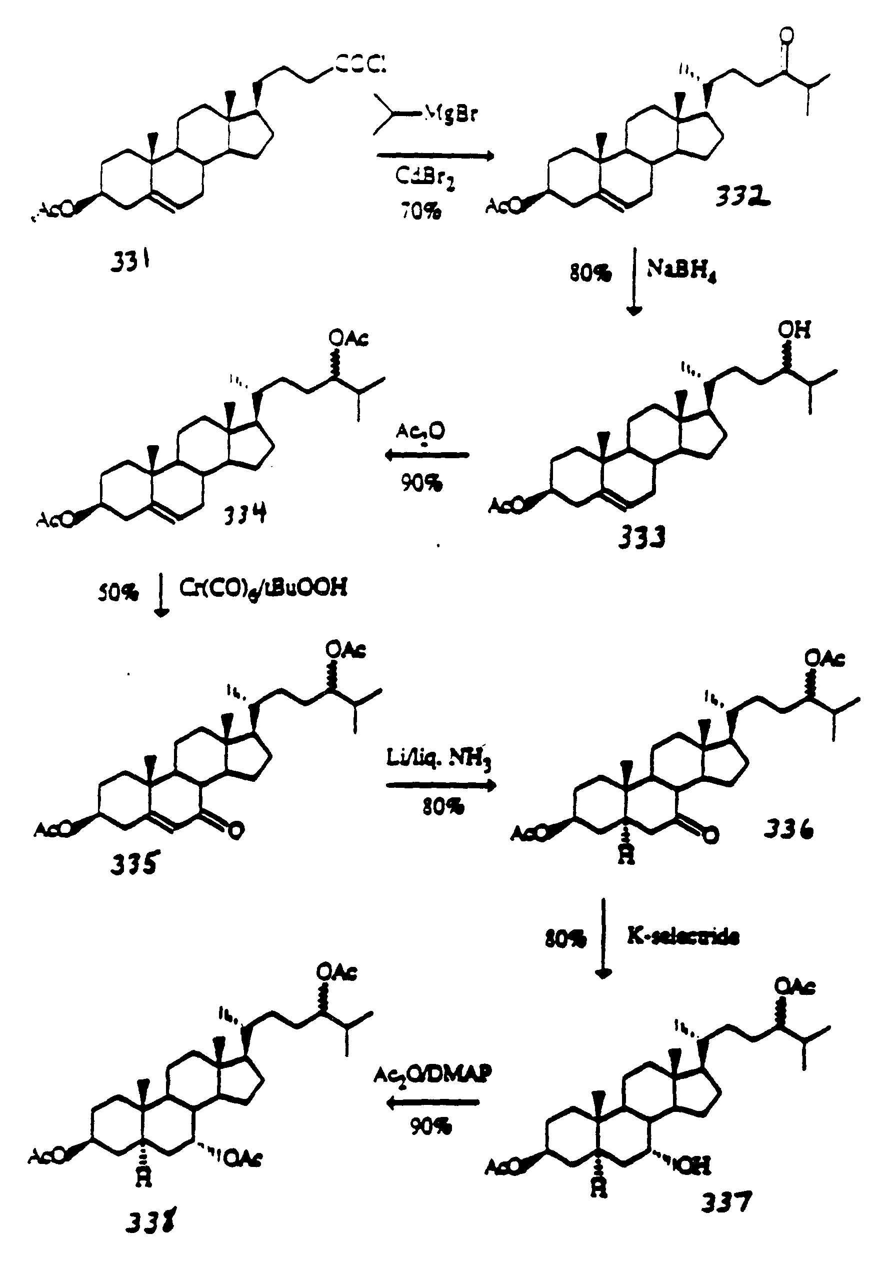

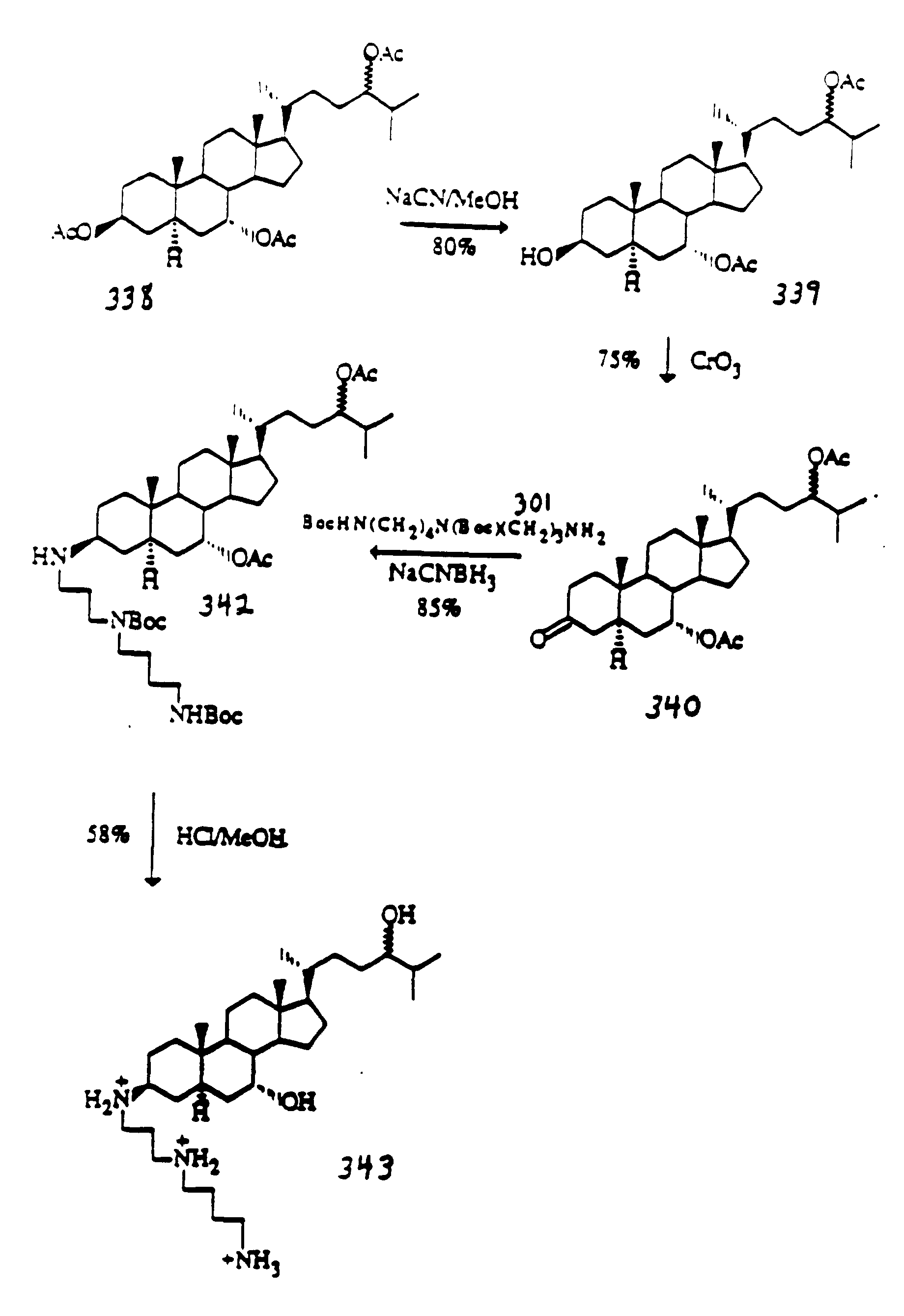

Preparation of compound **332:** To a solution of 3β-acetoxy-5-cholenic acid (50.0 g, 118 mmole) in dry dichloromethane (200 ml) was added dropwise oxalyl chloride (30 ml, 448 mmole). The solution was stirred at room temperature for one hour and then concentrated *in vacuo* to obtain 3β-acetoxy-5-cholenic acid chloride **331.** ¹H NMR (400 MHz, CDCl₃) δ: 0.70 (3H, s, 18-CH₃), 0.95 (3H, d, 21-CH₃), 1.05 (3H, s, 19-CH₃), 2.04 (3H, s, -OCOCH₃), 4.60 (1H, m, 3α-H), 5.38 (1H, m, 6-H). Compound **331** was used in the following step without purification.

To a mixture of magnesium (24 g, 1.00 mole) in dry ether (500 ml) was added dropwise 2-bromopropane (60 ml, 639 mmole) while stirring. After the addition was completed, the reaction mixture was stirred for thirty minutes. The ethereal solution was transferred to another flask. Then to the resulting isopropyl-magnesium bromide solution was added portionwise cadmium bromide (75 g, 276 mmole) at room temperature. The resulting dark solution was refluxed gently for one hour, followed by the addition of dry benzene (200 ml), then most of the ether was removed. To this mixture, 3β-acetoxy-5-cholenic acid chloride **331** (50 g, 115 mmole) in dry benzene (300 ml) was added dropwise. The reaction mixture was stirred at room temperature for one hour and then poured slowly into a crushed ice and 10% hydrochloric acid mixture. The organic layer was separated. The aqueous layer was extracted with ether (3 x 300 ml). The combined ethereal solution was washed with 10% HCl, washed with water, dried (MgSO₄), filtered, and concentrated *in vacuo* to give crude product, 3β-acetoxy-24-oxo-5-cholestene **332** (35.6 g, 70% yield), which was used for the next reaction without further purification. ¹H NMR (400 MHz, CDCl₃) δ: 0.69 (3H, s, 18-CH₃), 0.95 (3H, d, 21-CH₃), 1.04 (3H, s, 19-CH₃), 1.25 (6H, 2d, 26-CH₃, 27-CH₃), 2.04 (3H, s, -OCOCH₃), 4.61 (1H, m, 3α-H), 5.38 (1H, m, 6-H).

Preparation of compound **333:** To a solution of 3β-acetoxy-24-oxo-5-cholestene **332** (35 g, 79.0 mmole) in methanol (300 ml) was added portionwise sodium borohydride (6.0 g, 158 mmole) while stirring. After the addition was completed, the reaction mixture was stirred for an additional hour and then poured slowly into crushed ice and 10% hydrochloric acid mixture. Most of the methanol was removed *in vacuo.* The aqueous solution was extracted with ether (3 x 300 ml). The combined ethereal solution was washed with 10% hydrochloric acid, washed with brine, dried (MgSO₄), filtered, and concentrated *in vacuo.* The crude product was purified by flash chromatography on silica gel (elution with 20% ethyl acetate in hexane) to give 3β-acetoxy-24ζ-hydroxy-5-cholestene **333** (27.7 g, 80% yield). ¹H NMR (400 MHz, CDCl₃) δ: 0.69 (3H, s, 18-CH₃), 0.92 (9H, m, 21-CH₃, 26-CH₃, 27-CH₃), 1.02 (3H, s, 19-CH₃), 2.04 (3H, s, -OCOCH₃), 3.34 (1H, m, 24ζ-H), 4.60 (1H, m, 3α-H), 5.38 (1H, m, 6-H).

Preparation of compound **334:** A solution of 3β-acetoxy-24ζ-hydroxy-5-cholestene **333** (20.0 g, 45 mmole), dry pyridine (200 ml, 2.5 mole) and acetic anhydride (30 ml, 318 mmole) was stirred at room temperature for sixteen hours. Then the reaction mixture was poured into crushed ice and saturated NaHCO₃ solution. The aqueous solution was extracted with ether (3 x 300 ml). The combined ether extracts were washed with saturated NaHCO₃ solution (2 x 100 ml), water (2 x 150 ml), 2N HCl (3 x 75 ml) and brine (1 x 100 ml), and then dried (MgSO₄), filtered and concentrated in *vacuo* to yield a crude product, which was purified by flash chromatography on silica gel (elution with 10% ethyl acetate in hexane) to give pure 3β,24ζ-diacetoxy-5-cholestene **334** (18.4 g, 90% yield). ¹H NMR (400 MHz, CDCl₃) δ: 0.68 (3H, s, 18-CH₃), 0.90 (9H, m, 21-CH₃, 26-CH₃, 27-CH₃), 1.02 (3H, s, 19-CH₃), 2.07 (3H, s, -OCOCH₃), 2.09 (3H, s, -OCOCH₃), 4.58 (1H, m, 3α-H), 4.68 (1H, m, 24ζ-H), 5.38 (1H, m, 6-H).

Preparation of compound **335:** A solution of 3β, 24ζ-diacetoxy-5-cholestene **334** (15 g, 33.0 mmole), chromium hexacarbonyl (11.6 g, 52.7 mmole) and tert-butyl hydroperoxide (100 ml, 94 g, 1.04 mole) in dry acetonitrile (500 ml) was refluxed under argon for twelve hours. The acetonitrile was removed *in vacuo*, and the residue was dissolved in ether (500 ml). The ether extract was washed with saturated NaHCO₃ (3 x 150 ml) and brine (1 x 100 ml), dried (MgSO₄), filtered and concentrated *in vacuo.* The crude product was purified by flash chromatography on silica gel (elution with 20% ethyl acetate in hexane) to give pure 3β,24ζ-diacetoxy-7-oxo-5-cholestene **335** (8.26 g, 50% yield). ¹H NMR (400 MHz, CDCl₃) δ: 0.68 (3H, s, 18-CH₃), 0.90 (9H, m, 21-CH₃, 26-CH₃, 27-CH₃), 1.22 (3H, s, 19-CH₃), 2.05 (6H, s, 2(-OCOCH₃)), 4.65 (2H, m, 3α-H and 24ζ-H), 5.69 (1H, m, 6-H).

Preparation of compound **336:** To a solution of 3β,24ζ-diacetoxy-7-oxo-5-cholestene **335** (8.0 g, 16.0 mmole) in dry ether (50 ml) was added distilled liquid ammonia (approx. 200 ml) at -78°C. Lithium (0.5 g, 72.1 mmole) was added in small portions until a blue coloration persisted for ten minutes, after which the solution was quenched with solid NH₄Cl (approx. 50 g). The ammonia was evaporated, and the resulting residue was partitioned between water (500 ml) and ether (300 ml). The aqueous solution was extracted further with ether (3 x 200 ml). The combined ether extracts were washed with brine (1 x 100 ml), dried (MgSO₄), filtered and concentrated *in vacuo* to produce a crude product, which was purified by flash chromatography on silica gel (elution with 20% ethyl acetate in hexane) to afford pure 3β,24ζ-diacetoxy-7-oxo-5α-cholestane **336** (6.4 g, 80% yield). ¹H NMR (400 MHz, CDCl₃) δ: 0.65 (3H, s, 18-CH₃), 0.90 (9H, m, 21-CH₃, 26-CH₃, 27-CH₃), 1.10 (3H, s, 19-CH₃), 2.02 (3H, s, -OCOCH₃), 2.04 (3H, s, -OCOCH₃), 2.35 (2H, t, 6-CH₂), 4.66 (2H, m, 3α-H and 24ζ-H).

Preparation of compound **337:** To a solution of 3β,24ζ-diacetoxy-7-oxo-5α-cholestane **336** (6.0 g, 11.9 mmole) in dry tetrahydrofuran (200 ml) was added dropwise a solution of K-Selectride® (potassium tri-sec-butyl-borohydride) (1.0M in THF, 60 ml, 60 mmole) at -50°C. The reaction mixture was stirred at that temperature for five hours, and then quenched with 30% hydrogen peroxide solution (20 ml) and saturated NH₄Cl. The aqueous solution was extracted with ether (3 x 100 ml). The combined ether extracts were washed with saturated NaHCO₃ (2 x 70 ml), water (2 x 100 ml) and brine (1 x 70 ml), and then dried (MgSO₄), filtered and concentrated *in vacuo* to give a crude product. The crude product was purified by flash chromatography on silica gel (elution with 30% ethyl acetate in hexane) to give pure 3β,24ζ-diacetoxy-7α-hydroxy-5α-cholestane **337** (4.8 g, 80% yield). ¹H NMR (400 MHz, CDCl₃) δ: 0.68 (3H, s, 18-CH₃), 0.82 (3H, s, 19-CH₃), 0.91 (9H, m, 21-CH₃, 26-CH₃, 27-CH₃), 2.05 (3H, s, -OCOCH₃), 2.08 (3H, s, -OCOCH₃), 3.82 (1H, m, 7β-H), 4.65 (2H, m, 3α-H and 24ζ-H) ; CIMS(m/e): 505 (M⁺+1, 5%), 487 (11.0%), 443 (9.8%), 427 (100%), 367 (39.3%).

Preparation of compound **338:** To a solution of 3β,24ζ-diacetoxy-7α-hydroxy-5α-cholestane **337** (4.0 g, 7.92 mmole) and 4-dimethylaminopyridine (9.66 g, 79.2 mmole) in dry CH₂Cl₂ (40 ml) was added acetic anhydride (6.5 g, 73.4 mmole) at room temperature. After eighteen hours, methanol was added to the reaction mixture, then the organic solvents were evaporated *in vacuo* to get oily residue. The residue was dissolved in EtOAc (100 ml), washed with 2N HCl (3 x 25 ml), water (1 x 50 ml), saturated NaHCO₃ (3 x 25 ml) and brine (1 x 25 ml), and then dried (MgSO₄), filtered and evaporated *in vacuo.* The resulting crude product was purified by flash chromatography on silica gel (elution with 20% ethyl acetate in hexane) to give 3β,7α,24ζ-triacetoxy-5α-cholestane **338** (3.9 g, 90% yield) as a white solid. ¹H NMR (400 MHz, CDCl₃) δ: 0.66 (3H, s, 18-CH₃), 0.84 (3H, s, 19-CH₃), 0.90 (9H, m, 21-CH3, 26-CH₃, 27-CH₃), 2.05 (3H, s, -OCOCH₃), 2.07 (3H, s, -OCOCH₃), 2.10 (3H, s, -OCOCH₃), 4.68 (2H, m, 3α-H and 24ζ-H), 4.88 (1H, m, 7β-H); CIMS(m/e): 548 (M⁺+1, 1.0%), 487 (11.0%), 443 (9.8%), 427 (100%), 367 (39.3%).

Preparation of compound **339:** A solution of 3β,7α,24ζ-triacetoxy-5α-cholestane **338** (2.20 g, 4.02 mmole) and sodium cyanide (0.20 g, 4.08 mmole) in methanol (70 ml) was stirred at room temperature for forty hours. After completion of the reaction, methanol was evaporated in vacuo, and the residue was extracted with CH₂Cl₂ (3 x 30 ml). The combined CH₂Cl₂ extracts were concentrated *in vacuo* to give 7α,24ζ-diacetoxy-3β-hydroxy-5α-cholestane **339** as a white solid (1.62 g, 80% yield). ¹H NMR (400 MHz, CDCl₃) δ: 0.66 (3H, s, 18-CH₃), 0.82 (3H, s, 19-CH₃), 0.91 (9H, m, 21-CH₃, 26-CH₃, 27-CH₃), 2.05 (3H, s, -OCOCH₃), 2.08 (3H, s, -OCOCH₃), 3.60 (1H, m, 3α-H), 4.67 (1H, m, 24ζ-H), 4.88 (1H, m, 7β-H) ; CIMS(m/e): 505 (M⁺+1, 3.7%), 487 (4.4%), 444 (19.1%), 401 (11.0%), 385 (100%), 367 (31.9%).

Preparation of compound **340:** To a solution of 7α,24ζ-diacetoxy-3β-hydroxy-5α-cholestane **339** (1.5 g, 2.97 mmole) in acetone (100 ml) was added Jones reagent (aqueous chromic acid solution; CrO₃ in sulfuric acid and water) dropwise at 0°C until an orange color persisted. The reaction mixture was stirred at 0°C for ten minutes, then isopropanol was added until a green color was observed. Water (50 ml) and sodium acetate (5 g) were added to the mixture, and then the organic solvents were removed in vacuo. The residue was extracted with CHCl₃ (3 x 50 ml). The combined organic extracts were washed with saturated NaHCO₃ (2 x 50 ml), water (2 x 50 ml) and brine (1 x 50 ml), and then dried (MgSO₄), filtered and evaporated *in vacuo* to provide a crude product, which was purified by flash chromatography on silica gel (elution with 20% ethyl acetate in hexane), affording pure 3-oxo-7α,24ζ-diacetoxy-5α-cholestane **340** as a white solid (1.12 g, 75% yield). ¹H NMR (400 MHz, CDCl₃) δ: 0.66 (3H, s, 18-CH₃), 0.82 (9H, m, 21-CH₃, 26-CH₃, 27-CH₃), 0.99 (3H, s, 19-CH₃), 1.98 (3H, s, -OCOCH₃), 2.01 (3H, s, -OCOCH₃), 4.63 (1H, m, 24ζ-H), 4.88 (1H, m, 7β-H) ; CIMS(m/e): 442 (8.3%), 383 (100%), 312 (6.4%).

Preparation of compound **301:** To a solution of 1,4-diaminobutane (4.3 g, 48.8 mmole) in methanol (1.5 ml) was added a solution of acrylonitrile (3.1 g, 58.4 mmole) in methanol (1.5 ml) at 0°C, and the mixture was stirred for twelve hours. Evaporation of the solvent *in vacuo* afforded N-(2'-cyanoethyl)-1,4-diaminobutane as a colorless oil (5.5 g, 80% yield). ¹H NMR (400 MHz, CDCl₃) δ: 1.45 (4H, br, -CH₂CH₂-), 2.46 (2H, t), 2.58 (2H, t), 2.62 (2H, t), 2.84 (2H, t).

To a solution of the thus-obtained N-(2'-cyanoethyl)-1,4-diaminobutane (2.8 g, 20 mmole) in dichloromethane (70 ml) was added dropwise a solution of di-tert-butyldicarbonate (9.6 g, 44 mmole) in dichloromethane (10 ml) at room temperature, and the mixture was stirred for twelve hours. The organic solvent was removed *in vacuo* and the residual oil was dissolved in ethyl acetate (100 ml), followed by washing with saturated NaHCO₃ (2 x 50 ml), water (2 x 50 ml) and brine (50 ml), drying (MgSO₄), filtering and evaporation. The resulting crude viscous oil was purified by flash chromatography on silica gel, yielding pure N-(2'-cyanoethyl)-N,N'-(di-tert-butoxycarbonyl)-1,4-diaminobutane as a colorless, viscous oil (4.2 g, 75% yield). ¹H NMR (400 MHz, CDCl₃) δ: 1.44 (9H, t-Boc), 1.46 (9H, merged s, t-Boc), 2.60 (2H, m), 3.15 (2H, m), 3.28 (2H, t), 3.45 (2H, t); CIMS(m/e) : 342 (M⁺+1, 62.7%), 239 (100%), 186 (83.1%).

To a suspension of LAH (0.6 g, 16.3 mmole) in dry ether (100 ml) was added a solution of the N-(2'-cyanoethyl)-N,N'-(di-tert-butoxycarbonyl)-1,4-diaminobutane (1.6 g, 4.6 mmole) in dry ether (50 ml) dropwise at 0°C, and the mixture was stirred for thirty minutes. The excess LAH was quenched with 1N NaOH at 0°C, and the resulting white suspension was filtered through Celite and washed with ether, and the ether extract was washed with brine, dried (MgSO₄), filtered and evaporated *in vacuo.* The resulting crude oil was purified by flash chromatography on silica gel, yielding pure N-(3'-aminopropyl)-N,N'-(di-tert-butoxycarbonyl)-1,4-diaminobutane **301** (1.1 g, 68% yield) as a colorless oil. ¹H NMR (400 MHz, CDCl₃) δ: 1.44 (18H, s, 2(t-Boc)), 2.68 (2H, t), 3.05-3.25 (6H, br), 4.65 (1H, br) ; CIMS(m/e): 346 (M⁺+1, 100%), 290 (3.1%), 246 (32.2%).

Preparation of compound **342:** To a solution of **340** (1.0 g, 1.99 mmole) and **301** (1.03 g, 2.98 mmole) in methanol (60 ml) was added 3Å molecular sieves (4.00 g) and NaCNBH₃ (187.1 mg, 2.98 mmole). The reaction mixture was stirred at room temperature for sixteen hours. After filtering through Celite, methanol was removed *in vacuo.* The resulting residue was purified by flash chromatography on silica gel (first 10:1 chloroform:methanol, and then 15:1:1 chloroform: methanol:isopropylamine), yielding 3β-N-1{N-[3-(4-tert-butoxycarbonylaminobutyl)-3-tert-butoxycarbonyl]-1,3-diaminopropane}-7α,24ζ-diacetoxy-5α-cholestane **342** (1.4 g, 85% yield). ¹H NMR (400 MHz, CDCl₃) δ: 0.65 (3H, s, 18-CH₃), 0.80 (3H, s, 19-CH₃), 0.88 (9H, m, 21-CH₃, 26-CH₃, 27-CH₃), 1.45 (18H, s, 2(t-Boc)), 2.05 (3H, s, -OCOCH₃), 2.08 (3H, s, -OCOCH₃), 2.42 (1H, m, 3α-H), 4.67 (1H, m, 24ζ-H), 4.85 (1H, m, 7β-H) ; CIMS (m/e) : 832 (M⁺+1, 22.5%), 758 (100%), 698 (33.4%), 658 (44.7%), 548 (68.0%).

Preparation of compound **343:** To a solution of **342** (1.0 g, 1.2 mmole) in methanol (40 ml) was added methanol saturated with HCl gas (10 ml). The reaction mixture was stirred at room temperature for twenty-four hours. After removing the methanol in vacuo, the crude product was purified by flash chromatography on silica gel, eluting with dichloromethane:methanol:ammonium hydroxide (7.5:2:0.5), producing 3β-N-1-{N-[3-(4-aminobutyl)]-1,3-diaminopropane}-7α,24ζ-dihydroxy-5α-cholestane **343,** which was dissolved in methanol, treated with methanolic HCl and evaporated to give **343-3HCl** (382 mg, 58%). ¹H NMR (400 MHz, CD₃OD) δ: 0.73 (3H, s, 18-CH₃), 0.89 (3H, s, 19-CH₃), 3.01 (2H, t, -CH₂N-), 3.12 (2H, t, -CH₂N-), 3.18 (6H, m, 3α,24ζ-H and 2(-CH₂-)), 3.80 (1H, m, 7β-H); MS(+FAB): 548 (M⁺+1, 100%), 531 (50.8%), 477 (21.8%).

In general, the compounds of the invention may be prepared in neutral or salt form. Pharmaceutically acceptable salts include those formed with free amino groups such as those derived from hydrochloric, phosphoric, sulfuric, acetic, trifluoroacetic, oxalic, tartaric acids, etc., and those formed with free carboxyl groups such as those derived from sodium, potassium, ammonium, calcium, ferric hydroxides, isopropylamine, triethylamine, 2-ethylamino, ethanol, histidine, procaine, etc.

### Uses Based on Antiangiogenic Activity:

Squalamine and the compounds of Formula I, and pharmaceutically acceptable salts thereof, may also be used as fibroblast growth inhibitors, antiangiogenic agents and cytotoxic agents. Squalamine has an especially potent activity.

Fibroblast growth factors stimulate the proliferation of endothelial cells. Fibroblast growth factors are able to elicit the formation of new bloods vessels (a process called angiogenesis) by stimulating endothelial cells to migrate through the vessel wall and proliferate as capillary sprouts. Endothelial cells secrete a group of growth factors that are mutagenic for endothelium and can induce formation of new blood vessels. Since the growth of tumors depends on an adequate blood supply, which in turn depends on the growth of new blood vessels in tumors, a compound that inhibits fibroblast growth or angiogenesis can cause tumor regression in animals or man or inhibit the growth of tumors beyond a certain size. Moreover, a compound that is antiangiogenic or that inhibits fibroblast growth can also be used to treat other pathological conditions that are deleteriously affected by angiogenesis, such as diabetic retinopathy.

Squalamine and the compounds of Formula I are effective fibroblast growth inhibitors, antiangiogenic agents or cytotoxic agents and are therefore useful for treating cancerous tumors. Squalamine has potent antiangiogenic activity and is a preferred compound for cancer therapy.

Preferred compositions for treating cancer include one or more compounds selected from squalamine and the compounds of Formula I in combination with a suitable lipid. An especially preferred and advantageous composition contains squalamine and an anionic lipid.

### Uses Based on Na⁺/K⁺-ATPase-Inhibiting Activity:

Squalamine and the compounds of Formula I, and pharmaceutically acceptable salts thereof, are also useful inhibitors of Na⁺, K⁺ adenosine triphosphatase (ATPase). Na⁺/K⁺-ATPase inhibition causes a decrease in tubular salt transport. Consequently, these compounds are useful as diuretics, hypertensives, gastric-acid antisecretory agents and muscle relaxants. A preferred compound for such utilities is squalamine.

### Therapeutic Administration and Compositions

The mode of administration is selected to suit the particular therapeutic use. Modes of administration generally include, but are not limited to, transdermal, intramuscular, intraperitoneal, intravenous, subcutaneous, intranasal, inhalation, intralesional, endothelial and oral routes. The compounds may be administered by any convenient route, for example by infusion or bolus injection by absorption through epithelial or mucocutaneous linings (e.g., oral mucosa, rectal and intestinal mucosa, etc.) and may be administered together with other biologically active agents. Administration may be systemic.

The present invention also provides pharmaceutical compositions. Such compositions comprise a therapeutically effective amount of at least one compound of the invention, and a pharmaceutically acceptable carrier or excipient. Examples of such a carrier include but are not limited to saline, buffered saline, dextrose, water, glycerol, ethanol, and combinations thereof. The formulation should suit the mode of administration.

The composition, if desired, may also contain minor amounts of wetting or emulsifying agents, or pH buffering agents. The composition may be in the form of a liquid solution, suspension, emulsion, tablet, pill, capsule, sustained release formulation, or powder. The composition may be formulated as a suppository, with traditional binders and carriers such as triglycerides. Oral formulations may include standard carriers such as pharmaceutical grades of mannitol, lactose, starch, magnesium stearate, sodium saccharine, cellulose, magnesium carbonate, etc.

Various delivery systems are known and may be used to administer a therapeutic compound of the invention, e.g., encapsulation in liposomes, microparticles, microcapsules and the like.

In a preferred embodiment, the composition is formulated in accordance with routine procedures as a pharmaceutical composition adapted for intravenous administration to humans. Typically, compositions for intravenous administration are solutions in sterile isotonic aqueous buffer. Where necessary, the composition may also include a solubilizing agent and a local anesthetic to ameliorate any pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it may be dispensed with an infusion bottle containing sterile pharmaceutical-grade water or saline. Where the composition is administered by injection, an ampoule of sterile water for injection or saline may be provided so that the ingredients may be mixed prior to administration.

The amount of the therapeutic compound of the invention which will be effective in the treatment of a particular disorder or condition will depend on the nature of the disorder or condition, and can be determined by standard clinical techniques. The precise dose to be employed in the formulation will also depend on the route of administration and the seriousness of the disease or disorder, and should be decided according to the judgment of the practitioner and each patient's circumstances. Effective therapeutic doses may be determined from extrapolations of dose-response curves derived from *in vitro* or animal-model test systems.

Suitable dosage ranges for intravenous administration are generally about 20 micrograms to 40 milligrams of active compound per kilogram body weight. Suitable dosage ranges for intranasal administration are generally about 0.01 mg/kg body weight to 1 mg/kg body weight. Suitable dosage ranges for topical administration are generally at least about 0.01% by weight. Suitable dosages for oral administration are generally about 500 micrograms to 800 milligrams per kilogram body weight, more specifically about 50-200 mg/kg body weight. In many cases it is not necessary to employ the steroid compound in an amount greater than 2.0% by weight. Suppositories generally contain, as the active ingredient, a compound of the invention in the range of 0.5% to 10% by weight; oral formulations preferably contain 10% to 95% active ingredient.

For use of the compounds as antiangiogenic or cytotoxic agents, or in cancer therapies, exemplary dosages are from about 0.01 mg/kg body weight to about 100 mg/kg body. Preferred dosages are from 0.1 to 25 mg/kg body weight.

For use of the compounds as Na⁺/K⁺-ATPase inhibitors, exemplary dosages are from about 0.01 mg/kg body weight to about 100 mg/kg body, and are preferably from 0.1 to 25 mg/kg body weight. Thus, such dosages apply to the use of the compounds as diuretics, antihypertensives, antisecretory agents and muscle relaxants.

The invention also provides a pharmaceutical pack or kit comprising one or more containers filled with one or more of the active ingredients of the pharmaceutical compositions of the invention. Associated with such containers may be a notice in the form prescribed by a governmental agency regulating the manufacture, use or sale of pharmaceuticals or biological products, which notice reflects approval by the agency of manufacture, use or sale for human administration.

## Claims

1. The use of an agent selected from the group consisting of squalamine and salts thereof, and compounds and salts of formula I: 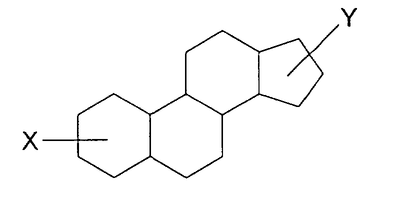 wherein X is a cationic hydrophilic side chain having at least two positively charged amino groups; Y is an anionic hydrophilic side chain; and the steroid ring nucleus includes saturated, unsaturated or partially saturated rings and at least one substituent selected from the group consisting of hydroxy, thio, fluorine, alkyl, alkoxy, and amino, for the production of a pharmaceutical for inhibiting angiogenesis in a patient.

2. The use of an agent selected from the group consisting of squalamine and salts thereof, and compounds and salts of formula I: 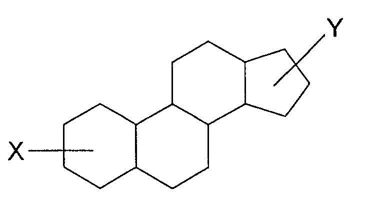 wherein X is a cationic hydrophilic side chain having at least two positively charged amino groups; Y is an anionic hydrophilic side chain; and the steroid ring nucleus includes saturated, unsaturated or partially saturated rings and at least one substituent selected from the group consisting of hydroxy, thio, fluorine, alkyl, alkoxy, and amino, for the production of a pharmaceutical for inhibiting fibroblast growth in a patient.

3. The use of an agent selected from the group consisting of squalamine and salts thereof, and compounds and salts of formula I: 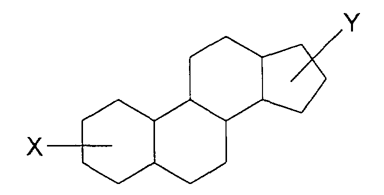 wherein X is a cationic hydrophilic side chain having at least two positively charged amino groups; Y is an anionic hydrophilic side chain; and the steroid ring nucleus includes saturated, unsaturated or partially saturated rings and at least one substituent selected from the group consisting of hydroxy, thio, fluorine, alkyl, alkoxy, and amino, for the production of a pharmaceutical for inhibiting Na⁺/K⁺-ATPase in a patient.

4. A use according to claim 3, wherein said pharmaceutical is a diuretic agent.

5. A use according to claim 3, wherein said pharmaceutical is a hypertensive agent.

6. A use according to claim 3, wherein said pharmaceutical is a gastric-acid antisecretory agent.

7. A use according to any one of claims 1 to 6, wherein said agent is squalamine.

8. A use according to claim 3, wherein said pharmaceutical is a muscle relaxant.

## Patentansprüche

1. Verwendung eines Mittels, ausgewählt aus der Gruppe bestehend aus Squalamin und Salzen davon, und Verbindungen und Salze der Formel (I): 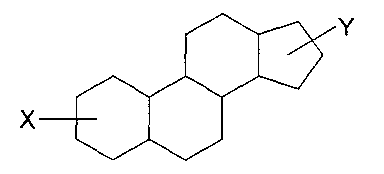 worin X eine kationische hydrophile Seitenkette mit mindestens zwei positiv geladenen Aminogruppen ist; Y ist eine anionische hydrophile Seitenkette und der Steroidringkern schliesst gesättigte, ungesättigte und teilweise ungesättigte Ringe ein und mindestens einen Substituent, ausgewählt aus der Gruppe bestehend aus Hydroxy, Thio, Fluor, Alkyl, Alkoxy und Amino, zur Herstellung eines Pharmazeutikums zur Hemmung der Angiogenese in einem Patienten.

2. Verwendung eines Mittels, ausgewählt aus der Gruppe bestehend aus Squalamin und Salzen davon, und Verbindungen und Salzen der Formel (I) 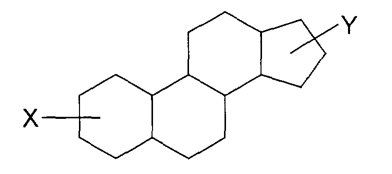 worin X eine kationische hydrophile Seitenkette mit mindestens zwei positiv geladenen Aminogruppen ist; Y ist eine anionische hydrophile Seitenkette und der Steroidringkern schliesst gesättigte, ungesättigte und teilweise ungesättigte Ringe ein und mindestens einen Substituent, ausgewählt aus der Gruppe bestehend aus Hydroxy, Thio, Fluor, Alkyl, Alkoxy und Amino, zur Herstellung eines Pharmazeutikums zur Hemmung von Fibroblastenwachstum in einem Patienten.

3. Verwendung eines Mittels, ausgewählt aus der Gruppe bestehend aus Squalamin und Salzen davon, und Verbindungen und Salze der Formel (I): 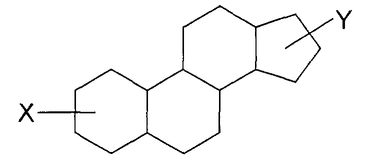 worin X eine kationische hydrophile Seitenkette mit mindestens zwei positiv geladenen Aminogruppen ist; Y ist eine anionische hydrophile Seitenkette und der Steroidringkern schliesst gesättigte, ungesättigte und teilweise ungesättigte Ringe ein und mindestens einen Substituent, ausgewählt aus der Gruppe bestehend aus Hydroxy, Thio, Fluor, Alkyl, Alkoxy und Amino, zur Herstellung eines Pharmazeutikums zur Hemmung der Na⁺/K⁺-ATPase in einem Patienten.

4. Verwendung gemäss Anspruch 3, wobei das Pharmazeutikum ein diuretisches Mittel ist.

5. Verwendung gemäss Anspruch 3, wobei das Pharmazeutikum ein Bluthochdruckmittel ist.

6. Verwendung gemäss Anspruch 3, wobei das Pharmazeutikum ein Mittel gegen die Sekretion von Gallensäure ist.

7. Verwendung gemäss einem der Ansprüche 1 bis 6, wobei das Mittel Squalamin ist.

8. Verwendung gemäss Anspruch 3, wobei das Pharmazeutikum ein Muskelrelaxanz ist.

## Revendications

1. Utilisation d'un agent choisi dans le groupe constitué par la squalamine et les sels de celle-ci, et des composés et sels de formule I : 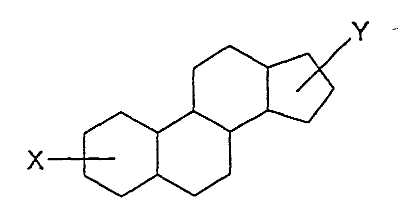 dans laquelle X représente une chaîne latérale hydrophile cationique ayant au moins deux groupes amino chargés positivement; Y représente une chaîne latérale hydrophile anionique; et le noyau cyclique stéroïde comprend des cycles saturés, insaturés ou partiellement saturés et au moins un substituant choisi dans le groupe constitué par les groupes hydroxy, thio, l'atome de fluor, les groupes alkyle, alcoxy et amino, pour la production d'un produit pharmaceutique pour inhiber l'angiogénèse chez un patient.

2. Utilisation d'un agent choisi dans le groupe constitué par la squalamine et les sels de celle-ci, et des composés et sels de formule I : 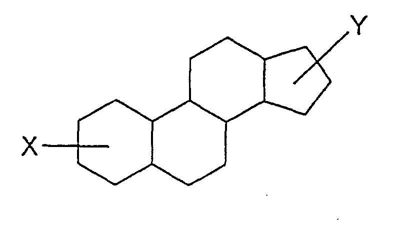 dans laquelle X représente une chaîne latérale hydrophile cationique ayant au moins deux groupes amino chargés positivement; Y représente une chaîne latérale hydrophile anionique; et le noyau cyclique stéroïde comprend des cycles saturés, insaturés ou partiellement saturés et au moins un substituant choisi dans le groupe constitué par les groupes hydroxy, thio, l'atome de fluor, les groupes alkyle, alcoxy et amino, pour la production d'un produit pharmaceutique pour inhiber la croissance de fibroblastes chez un patient.

3. Utilisation d'un agent choisi dans le groupe constitué par la squalamine et les sels de celle-ci, et des composés et sels de formule I : 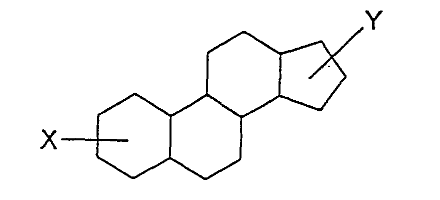 dans laquelle X représente une chaîne latérale hydrophile cationique ayant au moins deux groupes amino chargés positivement; Y représente une chaîne latérale hydrophile anionique; et le noyau cyclique stéroïde comprend des cycles saturés, insaturés ou partiellement saturés et au moins un substituant choisi dans le groupe constitué par les groupes hydroxy, thio, l'atome de fluor, les groupes alkyle, alcoxy et amino, pour la production d'un produit pharmaceutique pour inhiber la Na⁺/K⁺-ATPase chez un patient.

4. Utilisation selon la revendication 3, dans laquelle ledit produit pharmaceutique est un agent diurétique.

5. Utilisation selon la revendication 3, dans laquelle ledit produit pharmaceutique est un agent hypertenseur.

6. Utilisation selon la revendication 3, dans laquelle ledit produit pharmaceutique est un agent anti-sécrétoire d'acide gastrique.

7. Utilisation selon l'une quelconque des revendications 1 à 6, dans laquelle ledit agent est la squalamine.

8. Utilisation selon la revendication 3, dans laquelle ledit produit pharmaceutique est un relaxant musculaire.
